# EUROPÄISCHE PATENTSCHRIFT

(11) **EP 4 146 337 B1**
(45) Veröffentlichungstag und Bekanntmachung des Hinweises auf die Patenterteilung: **17.04.2024**
(21) Anmeldenummer: 20765022.7
(22) Anmeldetag: 07.09.2020
(51) Int. Cl.: A61P 3/00, C07C 67/08, C12P 7/62, C07C 69/716, C07C 69/675, A23L 33/115, A61K 31/225

(54) **VERFAHREN ZUR HERSTELLUNG VON POLYGLYCERINESTERN VON MIT OXOBUTANOL VERESTERTEN POLYCARBONSÄUREN**
METHOD FOR PRODUCING POLYGLYCEROL ESTERS OF POLYCARBOXYLIC ACIDS ESTERIFIED WITH OXOBUTANOL
PROCÉDÉ DE PRODUCTION D'ESTERS DE POLYGLYCÉROL D'ACIDES POLYCARBOXYLIQUES ESTÉRIFIÉS AVEC DE L'OXOBUTANOL

(30) Priorität: 13.07.2020 WO PCT/EP2020/069712
(43) Veröffentlichungstag der Anmeldung: 15.03.2023
(73) Patentinhaber: KetoLipix Therapeutics GmbH, 20459 Hamburg (DE)
(72) Erfinder: LOCHMANN, Dirk, 58453 Witten (DE); REYER, Sebastian, 58453 Witten (DE); STEHR, Michael, 58453 Witten (DE)
(74) Vertreter: Von Rohr Patentanwälte Partnerschaft mbB
(86) Internationale Anmeldenummer: PCT/EP2020/074892
(87) Internationale Veröffentlichungsnummer: WO 2022/012767

(56) Entgegenhaltungen:
- WO-A1-2010/021766
- US-B1- 9 925 164

## Beschreibung

Die vorliegende Erfindung betrifft das Gebiet der Ketokörper und des damit zusammenhängenden Stoffwechsels sowie die Therapie von damit im Zusammenhang stehenden Erkrankungen.

Insbesondere betrifft die vorliegende Erfindung ein Verfahren zur Herstellung von Polyglycerinestern von mit Oxobutanol veresterten Polycarbonsäuren sowie die auf diese Weise erhältlichen bzw. hergestellten Reaktionsprodukte (d. h. Polyglycerinester von mit Oxobutanol veresterten Polycarbonsäuren) und deren Verwendung, insbesondere in pharmazeutischen Zusammensetzungen, wie Arzneimitteln oder Medikamenten, oder in Nahrungsmittel- und/oder Lebensmittelerzeugnissen, sowie deren weitere Anwendungen bzw. Verwendungen.

Des Weiteren betrifft die vorliegende Erfindung pharmazeutische Zusammensetzungen, insbesondere Arzneimittel oder Medikamente, welche die nach dem erfindungsgemäßen Herstellungsverfahren erhältlichen bzw. hergestellten Reaktionsprodukte (d. h. Polyglycerinester von mit Oxobutanol veresterten Polycarbonsäuren) umfassen, sowie deren Anwendungen bzw. Verwendungen.

Schließlich betrifft die vorliegende Erfindung Nahrungsmittel- und/oder Lebensmittelerzeugnisse, insbesondere Nahrungsergänzungsmittel, funktionelle Lebensmittel (*Functional Food*)*, Novel Food,* Lebensmittelzusatzstoffe, Nahrungszusätze, diätetische Lebensmittel, Power-Snacks, Appetitzügler und Kraft- und/oder Ausdauersport-Supplements, welche die nach dem erfindungsgemäßen Herstellungsverfahren erhältlichen bzw. hergestellten Reaktionsprodukte (d. h. Polyglycerinester von mit Oxobutanol veresterten Polycarbonsäuren) umfassen, sowie deren Anwendungen bzw. Verwendungen.

Im menschlichen Energiestoffwechsel ist Glucose der kurzfristig zur Verfügung stehende Energieträger, welcher in den Mitochondrien unter Freisetzung von Wasser und Kohlendioxid zu Energie verstoffwechselt wird. Bereits durch die Schlafperiode während der Nacht sind aber die Glycogenspeicher der Leber geleert. Jedoch benötigen vor allem das menschliche zentrale Nervensystem (ZNS) und das Herz eine permanente Energieversorgung.

Die physiologische Alternative zu Glucose, welche vor allem dem zentralen Nervensystem zur Verfügung steht, sind die sogenannten Ketokörper (synonym auch als Ketonkörper bezeichnet oder Englisch auch als *"Keton Bodies"* bezeichnet).

Der Begriff der Ketokörper ist insbesondere eine Sammelbezeichnung für drei Verbindungen, welche vor allem in katabolen Stoffwechsellagen (wie z. B. bei Hunger, Reduktionsdiät oder kohlenhydratarmer Ernährung) gebildet werden und unter Umständen zu einer Ketose führen. Unter den Begriff der Ketokörper fasst man insbesondere die drei Verbindungen Acetoacetat (synonym auch Acetacetat genannt) und Aceton sowie 3-Hydroxybuttersäure (nachfolgend synonym auch als beta-Hydroxybuttersäure oder BHB oder 3-BHB bezeichnet) bzw. deren Salz (d. h. 3-Hydroxybutyrat oder beta-Hydroxybutyrat) zusammen, wobei letztere Verbindung die bedeutendste der drei vorgenannten Verbindungen ist. 3-Hydroxybuttersäure bzw. deren Salz kommt physiologisch als (R)-Enantiomer vor, d. h. als (R)-3-Hydroxybuttersäure (synonym auch (3R)-3-Hydroxybuttersäure genannt, um das Chiralitätszentrum in 3-Position hervorzuheben) bzw. deren Salz.

Diese Ketokörper werden auch in großer Zahl beim Fasten oder Hungern physiologisch aus im Körper eingelagerten Lipiden durch Lipolyse bereitgestellt und ersetzen den Energieträger Glucose fast vollständig.

Die Ketokörper werden in der Leber aus Acetyl-Coenzym A (= Acetyl-CoA) gebildet, welches aus der beta-Oxidation stammt; sie stellen eine transportable Form des Acetyl-Coenzyms A im menschlichen Körper dar. Zur Verwertung der Ketokörper müssen sich Gehirn und Muskeln aber zunächst umstellen, indem sie Enzyme exprimieren, welche zur Rückwandlung von Ketokörpern in Acetyl-Coenzym A benötigt werden. Insbesondere in Hungerzeiten tragen die Ketokörper einen beträchtlichen Anteil zur Energiegewinnung bei. So ist es beispielsweise dem Gehirn nach einiger Zeit möglich, mit nur einem Drittel der Tagesmenge an Glucose auszukommen.

Physiologisch erfolgt die Synthese der Ketokörper aus zwei Molekülen aktivierter Essigsäure in Form von Acetyl-Coenzym A, dem normalen Zwischenprodukt des Fettsäureabbaus, wobei zunächst mit Hilfe der Acetyl-Coenzym A-Acetyltransferase das Acetoacetyl-Coenzym A gebildet wird, welches unter Verwendung einer weiteren Acetyl-Coenzym A-Einheit und des Enzyms HMG-CoA-Synthase zum Zwischenprodukt 3-Hydroxy-3-methyl-glutaryl-CoA (HMG-CoA) verlängert wird, wobei schließlich die HMG-CoA-Lyase das Acetoacetat abspaltet. Diese drei Schritte finden ausschließlich in den Mitochondrien der Leber statt (Lynenzyklus), wobei 3-Hydroxybutyrat schließlich im Zytosol durch die D-beta-Hydroxybutyrat-Dehydrogenase entsteht. HMG-CoA ist außerdem ein Endprodukt beim Abbau der Aminosäure Leucin, während Acetoacetat beim Abbau der Aminosäuren Phenylalanin und Tyrosin entsteht.

Durch spontane Decarboxylierung entsteht aus Acetoacetat Aceton; es ist gelegentlich im Atem von Diabetikern und Diäthaltenden wahrzunehmen. Es kann vom Körper nicht weiterverwendet werden. Der Anteil von Aceton an den Ketokörpern ist allerdings gering.

Acetoacetat wird also reduktiv in die physiologisch relevante Form der 3-Hydroxybuttersäure bzw. des 3-Hydroxybutyrats überführt, kann aber auch unter Kohlenstoffdioxidfreisetzung in das physiologisch unbrauchbare Aceton zerfallen, was bei einer schweren Ketose, einer Ketoacidose (z. B. bei Diabetes mellitus Typ 1-Patienten ohne Insulinsubstitution), im Urin und in der Ausatemluft nachweisbar und olfaktorisch wahrnehmbar ist.

3-Hydroxybuttersäure wird derzeit im Bereich des Kraftsports als Natrium-, Magnesium- oder Calcium-Salz eingesetzt und in den Handel gebracht.

Jedoch ist 3-Hydroxybuttersäure für den Menschen evolutionär nicht oder in nur sehr geringer Menge bekannt, da Pflanzen keine 3-Hydroxybuttersäure produzieren und 3-Hydroxybuttersäure im tierischen Organismus nur bei toten ausgezehrten Tieren in der Ketose vorkommt, so dass 3-Hydroxybuttersäure bei peroraler Verabreichung Brechreiz auslöst. 3-Hydroxybuttersäure in Form der freien Säure sowie deren Salzen schmeckt zudem stark bitter und kann schweres Erbrechen und Übelkeit hervorrufen.

Zudem können Patienten, vor allem Neugeborene, aber auch Erwachsene größere Mengen an Salzen der 3-Hydroxybuttersäure nicht permanent verkraften, da diese Verbindungen nierenschädigend wirken können.

Außerdem ist die Plasmahalbwertszeit von 3-Hydroxybuttersäure und deren Salzen derart gering, dass selbst bei Einnahme von mehreren Gramm die Ketose nur für ca. drei bis vier Stunden vorhält, d. h. Patienten können insbesondere während der Nacht daher nicht von einer Therapie mit 3-Hydroxybuttersäure oder deren Salzen kontinuierlich profitieren. Bei Stoffwechselerkrankungen kann dies zu lebensbedrohlichen Situationen führen.

Daher werden im Fall der Therapie derartiger Stoffwechselerkrankungen heute sogenannte mittelkettige Triglyceride, sogenannte MCTs, für die ketogene Therapie eingesetzt, d. h. es wird die metabolische Umwandlung von Capron-, Capryl- und Caprinsäure (d. h. von gesättigten linearen C₆-, C₈- und C₁₀-Fettsäuren) aus den korrespondierenden Triglyceriden beabsichtigt.

Grundsätzlich stellt aber aus pharmazeutischer und klinischer Hinsicht 3-Hydroxybuttersäure demgegenüber ein wirksameres pharmazeutisch-pharmakologisches Zielmolekül, welches nach den Erkenntnissen des Standes der Technik prinzipiell für die Therapie einer Vielzahl von Erkrankungen zum Einsatz kommen könnte, aber aufgrund seiner mangelnden physiologischen Kompatibilität dort nicht zum Einsatz kommen kann (z. B. bei Erkrankungen im Zusammenhang mit einer Störung des Energiestoffwechsels, insbesondere Ketokörperstoffwechsels, oder neurodegenerativen Erkrankungen wie Demenz, Morbus Alzheimer, Morbus Parkinson etc., Fettstoffwechselerkrankungen usw.).

Die nachfolgende Tabelle veranschaulicht rein beispielhaft, aber keinesfalls beschränkend potentielle Therapiemöglichkeiten bzw. mögliche Indikationen für den Wirkstoff 3-Hydroxybuttersäure.

| **Indikation** | **Therapeutischer Effekt** |
|---|---|
| Schädel-Hirn-Trauma | Unter BHB sinkt die Apoptose und Nekrose-Rate von Nervenzellen. |
| Schlaganfall | Unter BHB sinkt die Apoptose und Nekrose-Rate von Nervenzellen. |
| Refeeding-Syndrom | Bei Anorexie, Absetzen enteraler oder parenteraler Ernährung und nach langen Hungerperioden kann der Konsum von Stärke oder Glucose zum Tod führen (siehe auch WHO-Schema Erdnusspaste). BHB kann hier als Therapeutikum zum schnelleren Erlangen einer normalen Nahrungsaufnahme eingesetzt werden. |
| Appetitzügler | BHB unterdrückt im Zentralnervensystem (ZNS) das Hungergefühl. |
| Epilepsie | Herkömmliche ketogene Diät zur signifikanten Reduzierung der Häufigkeit von Krampfanfällen hat extrem schlechte Patienten-Verträglichkeit. BHB bietet hier eine unmittelbar wirksame Alternative. |
| Morbus Alzheimer, Demenz | Unter BHB zeigen Patienten eine bessere kognitive Leistung. BHB ist auch für die Prävention von neurodegenerativen Erkrankungen wirksam. |
| Störungen der Fettsäureoxidation (z. B. Electron-Transfer-Protein Defekt) | Ausgleich eines Nährstoffmangels bei Defekt im Energiestoffwechsel. |

Daher ist es aus pharmazeutischer und klinischer Hinsicht wünschenswert, wirksame Präkursoren oder Metabolite auffinden zu können, welche physiologisch einen direkten oder indirekten Zugang zu 3-Hydroxybuttersäure oder deren Salzen ermöglichen, insbesondere im physiologischen Stoffwechsel des menschlichen oder tierischen Körpers.

Folglich hat es im Stand der Technik nicht an Versuchen gefehlt, physiologisch geeignete Präkursoren oder Metaboliten für 3-Hydroxybuttersäure bzw. deren Salze aufzufinden. Bislang wurden im Stand der Technik jedoch keine effizienten diesbezüglichen Verbindungen aufgefunden. Auch ist ein diesbezüglicher Zugang zu solchen Verbindungen nach dem Stand der Technik bislang nicht bzw. nicht ohne Weiteres möglich.

Die US 9,925,164 B1 betrifft ein Verfahren zur Behandlung von leichter bis mittelschwerer nicht-penetrierender geschlossener traumatischer Hirnverletzung und leichter bis mittelschwerer SHT aufgrund eines chirurgischen Eingriffs unter Verwendung von 3-Hydroxybutyatglyceriden.

Des Weiteren betrifft die WO 2010/021766 eine Verbindung, welche in Bezug auf (3R)-Hydroxybutyl-(3R)-Hydroxybutyrat enantiomer angereichertes 3-Hydroxybutyl-3-Hydroxybutyrat der Formel (I) ist, wobei die Verbindung der Formel (I) ein wirksamer und schmackhafter Vorläufer des Ketokörpers (3R)-Hydroxybutyrat sind und zur Behandlung eines Zustands verwendet werden können, welcher durch einen erhöhten Plasmaspiegel an freien Fettsäuren bei einem Menschen oder Tier verursacht, verschlimmert oder damit verbunden ist (beispielsweise ein Zustand, bei welchem Gewichtsverlust oder Gewichtszunahme eine Rolle spielt) oder aber zur Förderung der Wachsamkeit oder zur Verbesserung der kognitiven Funktion oder zur Behandlung, Verhinderung oder Verringerung der Auswirkungen von Neurodegeneration, Toxizität durch freie Radikale, hypoxische Zustände oder Hyperglykämie eingesetzt werden können.

Das der vorliegenden Erfindung zugrundeliegende Problem liegt also in der Bereitstellung eines effizienten Herstellungsverfahrens von physiologisch geeigneten bzw. physiologisch kompatiblen Präkursoren und/oder Metaboliten von 3-Hydroxybuttersäure (d. h. beta-Hydroxybuttersäure bzw. BHB bzw. 3-BHB) oder deren Salzen.

Ein solches Verfahren soll insbesondere die betreffenden BHB-Präkursoren und/oder BHB-Metaboliten in effizienter Weise zugänglich machen, insbesondere auch in größeren Mengen und ohne nennenswerte Mengen an toxischen Nebenprodukten.

In vollkommen überraschender Weise hat die Anmelderin nunmehr herausgefunden, dass Polyglycerinester von mit Oxobutanol veresterten Polycarbonsäuren einen effizienten und physiologisch wirksamen bzw. physiologisch kompatiblen Präkursor und/oder Metaboliten für den Ketokörper 3-Hydroxybuttersäure bzw. deren Salze darstellen, und hat in diesem Zusammenhang ein effizientes Herstellungsverfahren für diese Verbindungen auffinden bzw. entwickeln können, welches einen direkten und wirksamen, insbesondere ökonomischen wie auch großtechnisch umsetzbaren Zugang zu diesen Verbindungen ermöglicht.

Zur Lösung des zuvor geschilderten Problems schlägt die vorliegende Erfindung daher - gemäß einem **ersten** Aspekt der vorliegenden Erfindung - ein Verfahren zur Herstellung von Polyglycerinestern von mit Oxobutanol veresterten Polycarbonsäuren gemäß Anspruch 1 vor; weitere, insbesondere besondere und/oder vorteilhafte Ausgestaltungen des erfindungsgemäßen Verfahrens sind Gegenstand der diesbezüglichen Verfahrensunteransprüche.

Weiterhin betrifft die vorliegende Erfindung - gemäß einem **zweiten** Aspekt der vorliegenden Erfindung - ein nach dem erfindungsgemäßen Verfahren erhältliches Reaktionszwischenprodukt bzw. einen Polycarbonsäureester von Oxobutanol gemäß dem diesbezüglichen Anspruch (Anspruch 6) sowie darüber hinaus ein nach dem erfindungsgemäßen Verfahren erhältliches Reaktionsprodukt bzw. einen Polyglycerinester von mit Oxobutanol veresterten Polycarbonsäuren gemäß den diesbezüglichen Ansprüchen (Ansprüche 7 bis 14) bzw. ein diesbezügliches Gemisch von mindestens zwei Polyglycerinestern von mit Oxobutanol veresterten Polycarbonsäuren gemäß dem diesbezüglichen Anspruch (Anspruch 15); weitere, insbesondere besondere und/oder vorteilhafte Ausgestaltungen dieses Erfindungsaspekts sind Gegenstand der diesbezüglichen Unteransprüche.

Gleichermaßen betrifft die vorliegende Erfindung - gemäß einem **dritten** Aspekt der vorliegenden Erfindung - eine pharmazeutische Zusammensetzung, insbesondere Arzneimittel oder Medikament, gemäß dem diesbezüglichen unabhängigen Anspruch (Anspruch 16); weitere, insbesondere besondere und/oder vorteilhafte Ausgestaltungen dieses Erfindungsaspekts sind Gegenstand des diesbezüglichen Unteranspruchs.

Darüber hinaus betrifft die vorliegende Erfindung - gemäß einem **vierten** Aspekt der vorliegenden Erfindung - ein erfindungsgemäßes Reaktionsprodukt bzw. einen erfindungsgemäßen Polyglycerinester von mit Oxobutanol veresterten Polycarbonsäuren bzw. ein erfindungsgemäßes Gemisch von mindestens zwei Polyglycerinestern von mit Oxobutanol veresterten Polycarbonsäuren zur prophylaktischen und/oder therapeutischen Behandlung bzw. zur Verwendung bei der prophylaktischen und/oder therapeutischen Behandlung von Erkrankungen des menschlichen oder tierischen Körpers gemäß dem diesbezüglichen unabhängigen Anspruch (Anspruch 18).

Des Weiteren betrifft die vorliegende Erfindung - gemäß einem **fünften** Aspekt der vorliegenden Erfindung - ein Nahrungsmittel und/oder Lebensmittelerzeugnis gemäß dem diesbezüglichen unabhängigen Anspruch (Anspruch 19).

Es versteht sich bei den nachfolgenden Ausführungen von selbst, dass Ausgestaltungen, Ausführungsformen, Vorteile und dergleichen, welche nachfolgend zu Zwecken der Vermeidung von Wiederholungen nur zu einem Erfindungsaspekt ausgeführt sind, selbstverständlich auch in Bezug auf die übrigen Erfindungsaspekte entsprechend gelten, ohne dass dies einer gesonderten Erwähnung bedarf.

Des Weiteren versteht es sich von selbst, dass einzelne Aspekte und Ausführungsformen der vorliegenden Erfindung auch in beliebiger Kombination mit anderen Aspekten und Ausführungsformen der vorliegenden Erfindung als offenbart gelten und insbesondere auch eine beliebige Kombination von Merkmalen und Ausführungsformen, wie sie sich aus den Rückbezügen aller Patentansprüche ergibt, umfangreich als offenbart gilt, und zwar im Hinblick auf alle sich ergebenden Kombinationsmöglichkeiten.

Bei allen nachstehend genannten relativen bzw. prozentualen gewichtsbezogenen Angaben, insbesondere relativen Mengen- oder Gewichtsangaben, ist weiterhin zu beachten, dass diese im Rahmen der vorliegenden Erfindung vom Fachmann derart auszuwählen sind, dass sie sich in der Summe unter Einbeziehung aller Komponenten bzw. Inhaltsstoffe, insbesondere wie nachfolgend definiert, stets zu 100 % bzw. 100 Gew.-% ergänzen bzw. addieren; dies versteht sich aber für den Fachmann von selbst.

Im Übrigen gilt, dass der Fachmann - anwendungsbezogen oder aber einzelfallbedingt - von den nachfolgend angeführten Bereichsangaben erforderlichenfalls abweichen kann, ohne dass er den Rahmen der vorliegenden Erfindung verlässt.

Zudem gilt, dass alle im Folgenden genannten Werte- bzw. Parameterangaben oder dergleichen grundsätzlich mit genormten bzw. standardisierten oder explizit angegebenen Bestimmungsverfahren oder andernfalls mit dem Fachmann auf diesem Gebiet an sich geläufigen Bestimmungs- bzw. Messmethoden ermittelt bzw. bestimmt werden können.

Dies vorausgeschickt, wird die vorliegende Erfindung nunmehr nachfolgend im Detail erläutert.

Gegenstand der vorliegenden Erfindung - gemäß einem **ersten** Aspekt der vorliegenden Erfindung - ist somit ein
Verfahren zur Herstellung von Polyglycerinestern von mit Oxobutanol veresterten Polycarbonsäuren, insbesondere ein- oder mehrwertigen Polyglycerinestern von mit 4-Oxo-4-(C₁-C₅-alkoxy)-2-butanol oder mit 4-Oxo-4-(hydroxy-C₃-C₅-alkoxy)-2-butanol veresterten Polycarbonsäuren,
wobei
   (i) mindestens ein Oxobutanol, insbesondere ein 3-Hydroxybutanoat, bevorzugt ein 4-Oxo-2-butanol, vorzugsweise ein 4-Oxo-4-(C₁-C₅-alkoxy)-2-butanol oder ein 4-Oxo-4-(hydroxy-C₃-C₅-alkoxy)-2-butanol, der allgemeinen Formel (I)

      CH₃-CH(OH)-CH₂-C(O)OR¹ (I)

      wobei in der allgemeinen Formel (I) der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt,
   (ii) mindestens eine Polycarbonsäure (II), insbesondere mindestens eine mindestens zwei Carboxylgruppen enthaltende Polycarbonsäure, wobei die Polycarbonsäure (II) in Form der freien Polycarbonsäure, in Form eines Salzes der Polycarbonsäure, in Form eines Polycarbonsäureesters oder in Form des Polycarbonsäureanhydrids eingesetzt wird, und
   (iii) mindestens ein Polyglycerin (III)
miteinander umgesetzt und/oder zur Reaktion gebracht werden,
wobei das Verfahren einstufig oder aber mehrstufig durchgeführt wird, und
wobei das Verfahren in Abwesenheit von Lösemitteln und/oder ohne jedwedes Lösemittel durchgeführt wird, und
wobei bei Verwendung der Polycarbonsäure (II) in Form der freien Säure bei der Umsetzung gleichzeitig Wasser gebildet wird, wobei das Wasser der Umsetzung kontinuierlich entzogen wird; und/oder
wobei bei Verwendung der Polycarbonsäure (II) in Form des Anhydrids die entsprechende freie Polycarbonsäure (II) und Wasser gebildet werden, wobei die entstehende freie Polycarbonsäure (II) weiter umgesetzt wird oder nach erfolgter Reaktion entfernt und gegebenenfalls rezykliert wird und wobei das Wasser der Umsetzung kontinuierlich entzogen wird; und/oder
wobei bei Verwendung der Polycarbonsäure (II) in Form des Esters der entsprechende Esteralkohol und Wasser gebildet werden, wobei der entstehende Esteralkohol und Wasser der Umsetzung kontinuierlich entzogen werden,
so dass als Reaktionsprodukt (IV) ein oder mehrere Polyglycerinester von mit Oxobutanol veresterten Polycarbonsäuren, insbesondere ein oder mehrere Polyglycerinester von mit 4-Oxo-2-butanol veresterten Polycarbonsäuren, vorzugsweise ein oder mehrere ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, erhalten werden.

Gemäß der vorliegenden Erfindung wird somit insbesondere ein Herstellungsverfahren für Polyglycerinester von mit 3-Hydroxybutanot veresterten Polycarbonsäuren bereitgestellt. 3-Hydroxybutanoat kann synonym auch als 3-Hydroxybuttersäureester oder aber als ein 4-Oxo-2-butanol bezeichnet werden.

Genau genommen wird als Oxobutanol bzw. 3-Hydroxybutanoat der allgemeinen Formel (I) entweder ein (C₁-C₅-Alkyl)-3-hydroxybutanoat (= 3-Hydroxybuttersäure-(C₁-C₅-alkyl)-ester), d. h. ein C₁-C₅-Alkyester der 3-Hydroxybuttersäure, welcher synonym auch als ein 4-Oxo-4-(C₁-C₅-alkoxy)-2-butanol bezeichnet werden kann, oder aber ein (Hydroxy-C₃-C₅-alkyl)-3-hydroxybutanoat (= 3-Hydroxybuttersäure-(hydroxy-C₃-C₅-alkyl)-ester), d. h. ein Hydroxy-C₃-C₅-alkyester der 3-Hydroxybuttersäure, welcher synonym auch als ein 4-Oxo-4-(hydroxy-C₃-C₅-alkoxy)-2-butanol bezeichnet werden kann, eingesetzt.

In diesem Zusammenhang können die 3-Hydroxybutanoate durch Veresterung der freien 3-Hydroxybuttersäure mit den entsprechenden Alkoholen (z. B. Monoalkohole wie Ethanol etc. oder Diole) hergestellt werden. Im Folgenden ist die Synthese von Hydroxybutyl-3-hydroxybutanoat (d. h. 3-Hydroxybutanoat der allgemeinen Formel (I) mit R¹ = Hydroxybutyl) schematisch dargestellt:

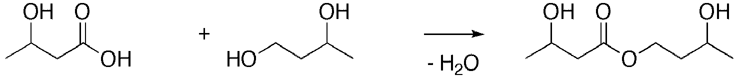

Weiterhin ist im Folgenden die Synthese von Hydroxypentyl-3-hydroxybutanoat (d. h. 3-Hydroxybutanoat der allgemeinen Formel (I) mit R¹ = Hydroxypentyl) schematisch dargestellt:

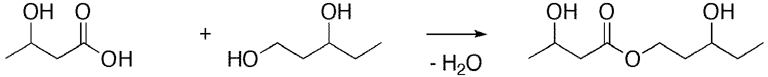

Die Herstellung bzw. Synthese weiterer 3-Hydroxybutanoate verläuft analog.

Im Rahmen des erfindungsgemäßen Verfahrens fungiert also das Edukt bzw. die Ausgangsverbindung 3-Hydroxybutanoat der allgemeinen Formel (I) über die Hydroxylgruppe in 3-Position als Veresterungs-Alkohol und reagiert hierüber mit der Carboxylgruppe der Polycarbonsäure (II), sodass als Reaktionszwischenprodukt (IV') ein entsprechender Polycarbonsäureester von 3-Hydroxybutanoat, d. h. ein entsprechender Polycarbonsäureester von 4-Oxo-4-(C₁-C₅-alkoxy)-2-butanol bzw. von 4-Oxo-4-(hydroxy-C₃-C₅-alkoxy)-2-butanol, gebildet wird.

Im Rahmen der vorliegenden Erfindung ist die eingesetzte Polycarbonsäure (II) eine organische Polycarbonsäure; d. h. die Polycarbonsäure (II) ist eine organische Verbindung mit mehreren Carboxylgruppen (-COOH), welche folglich einen aciden Charakter aufweist.

Bei den erfindungsgemäß erhaltenen Polyglycerinestern handelt es sich Ester von Polyglycerin (wie beispielsweise von Diglycerin) und gerade nicht um mehrfach verestertes Glycerin (d. h. es handelt sich insbesondere nicht um Mono-, Di- und Triglyceride, also die ein-, zwei- und dreiwertigen Ester des Glycerins bzw. 1,2,3-Propantriols).

Die Ein- und Mehrwertigkeit bezieht sich im Rahmen der vorliegenden Erfindung also auf die Anzahl der veresterten Gruppen des Polyglycerins (d. h. mit Polycarbonsäureestern von Oxobutanol veresterte Gruppen).

Überraschenderweise hat die Anmelderin eine effiziente wie wirksame Möglichkeit gefunden 3-Hydroxybuttersäure bzw. deren Derivat in einer physiologisch wie organoleptisch kompatiblen Form bereitzustellen, wobei die 3-Hydroxybuttersäure dennoch gut freigesetzt werden kann, insbesondere vom tierischen oder menschlichen Körper.

Darüber hinaus ist es der Anmelderin gelungen, die organoleptisch und physiologisch kompatible Form der 3-Hydroxybuttersäure derart bereitzustellen, dass ein Retardierungseffekt vorliegt; d. h. die 3-Hydroxybuttersäure wird über einen längeren Zeitraum kontinuierlich freigesetzt, insbesondere vom menschlichen oder tierischen Körper.

Außerdem sind auch die weiteren Abbau- bzw. Abspaltprodukte (d. h. die Abspaltprodukte, welche neben bzw. zusätzlich zu der 3-Hydroxybuttersäure freigesetzt werden) vom Körper verwertbar, zumindest aber vom Körper verarbeitbar. Insbesondere werden u. a. Abspaltprodukte freigesetzt, welche Edukte, Produkte oder Zwischenprodukte des Citratzyklus sind oder aber Derivate bzw. Salze sind, welche durch Oxidation eines Edukts, Produkts oder Zwischenprodukts des Citratzyklus gebildet werden. Somit können auch die weiteren Abbau- bzw. Abspaltprodukte, welche bei der Freisetzung von 3-Hydroxybuttersäure gebildet werden, als Energiequelle vom tierischen oder menschlichen Körper genutzt werden. Bei diesen Abspaltprodukten handelt es sich typischerweise um die Polycarbonsäure (II). Darüber hinaus ist das Polyglycerin ein nicht-toxischer und physiologisch kompatibler Träger, welcher vom Körper ohne Weiteres ausgeschieden wird.

Durch die Verwendung von Polyglycerin, welches eine Vielzahl von Hydroxylgruppen aufweist, ist es möglich, ein Molekül mit hoher Wirkstoffdichte, insbesondere hoher Dichte an 3-Hydroxybutanoaten bzw. 3-BHB oder entsprechenden Derivaten, bereitzustellen. Weiterhin kann in diesem Zusammenhang eine hohe Dichte auch an Polycarbonsäuren bereitgestellt werden, welche - wie zuvor ausgeführt - als weitere Energiequelle vom tierischen und/oder menschlichen Körper genutzt werden können.

Wie zuvor ausgeführt, hat die Anmelderin nämlich vollkommen überraschend herausgefunden, dass die auf diese Weise hergestellten Polyglycerinester von mit Oxobutanol veresterten Polycarbonsäuren, insbesondere Polyglycerinester von mit 4-Oxo-2-butanol veresterten Polycarbonsäuren, vorzugsweise ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, effiziente, da physiologisch verträgliche und auch organoleptisch kompatible Präkursoren und/oder Metabolite der 3-Hydroxybuttersäure bzw. deren Salzen darstellen, welche daher auch pharmazeutisch bzw. klinisch auch in größeren Mengen zum Einsatz kommen können.

Die vorgenannten Polyglycerinester von mit Oxobutanol veresterten Polycarbonsäuren, insbesondere Polyglycerinester von mit 4-Oxo-2-butanol veresterten Polycarbonsäuren, vorzugsweise ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, welche durch das erfindungsgemäße Herstellungsverfahren erstmals in effizienter Weise zugänglich sind, stellen somit eine physiologisch und pharmakologisch relevante Alternative zu der freien 3-Hydroxybuttersäure bzw. deren Salzen dar und sind zudem organoleptisch kompatibel.

Die Herstellung von Polyglycerinestern von mit Oxobutanol veresterten Polycarbonsäuren, insbesondere Polyglycerinestern von mit 4-Oxo-2-butanol veresterten Polycarbonsäuren, vorzugsweise ein- oder mehrwertigen Polyglycerinestern von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, mittels herkömmlicher organischer Synthese ist komplex und aufwendig, da 3-Hydroxybuttersäure sowie deren Salze und Ester verstärkt zur Polymerisation und anderen unerwünschten Nebenreaktionen (z. B. Wasserabspaltung, Zersetzung etc.) neigen. Im Rahmen der vorliegenden Erfindung konnte erstmals ein effizient arbeitendes Herstellungsverfahren bereitgestellt werden, mit welchem sich Polyglycerinester von mit Oxobutanol veresterten Polycarbonsäuren, insbesondere Polyglycerinester von mit 4-Oxo-2-butanol veresterten Polycarbonsäuren, vorzugsweise ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, ohne signifikante Nebenproduktbildung herstellen lassen.

Das erfindungsgemäße Verfahren ermöglicht somit erstmalig die Bereitstellung untoxischer Polyglycerinester von mit Oxobutanol veresterten Polycarbonsäuren, insbesondere Polyglycerinester von mit 4-Oxo-2-butanol veresterten Polycarbonsäuren, vorzugsweise ein- oder mehrwertiger Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, aus an sich bekannten, kommerziell verfügbaren und vor allem physiologisch unbedenklichen Komponenten bzw. Edukten (Ausgangsverbindungen). Die resultierenden Polyglycerinester von mit Oxobutanol veresterten Polycarbonsäuren, insbesondere Polyglycerinester von mit 4-Oxo-2-butanol veresterten Polycarbonsäuren, vorzugsweise ein- oder mehrwertigen Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, können physiologisch, insbesondere im Magen und/oder im Darm, aufgespalten werden und das Zielmolekül "3-Hydroxybuttersäure" bzw. deren Salze oder Ester als Wirkstoff bzw. Wirkkomponente freisetzen bzw. generieren.

Darüber hinaus weisen die vorgenannten Polyglycerinester von mit Oxobutanol veresterten Polycarbonsäuren, insbesondere Polyglycerinester von mit 4-Oxo-2-butanol veresterten Polycarbonsäuren, vorzugsweise ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, auch einen akzeptablen Geschmack auf, um eine Kompatibilität auch bei oraler Verabreichung größerer Mengen über einen längeren Zeitraum zu gewährleisten (z. B. Verabreichung von 50 g Tagesdosis oder mehr).

Gleichermaßen ermöglicht es das erfindungsgemäße Herstellungsverfahren, die Polyglycerinester von mit Oxobutanol veresterten Polycarbonsäuren, insbesondere Polyglycerinester von mit 4-Oxo-2-butanol veresterten Polycarbonsäuren, vorzugsweise ein- oder mehrwertigen Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, frei von toxischen Verunreinigungen bereitzustellen.

Darüber hinaus kann bei entsprechenden Ausgangsmaterialien die Herstellung auch enantioselektiv durchgeführt werden. So ermöglicht es beispielsweise das erfindungsgemäße Herstellungsverfahren, die biologisch relevante Form, d. h. das (R)-Enantiomer von BHB anzureichern, beispielsweise durch Enzymkatalyse oder die gezielte Auswahl der eingesetzten Ausgangsverbindungen (Edukte), um bei oraler Verabreichung das renale System von Patienten nicht zu belasten (d. h. Elimination über die Nieren). Grundsätzlich ist es aber auch möglich und kann es unter bestimmten Voraussetzungen zweckdienlich sein, das (S)-Enantiomer von BHB anzureichern.

Darüber hinaus ist das erfindungsgemäße Herstellungsverfahren, einschließlich optionaler Weiterverarbeitungs- bzw. Aufreinigungsverfahrensschritte, wirtschaftlich bzw. ökonomisch betreibbar und auch großtechnisch umsetzbar.

Insbesondere verwendet das erfindungsgemäße Herstellungsverfahren kommerziell verfügbare Edukte oder Edukte, welche durch einfache und großtechnisch durchführbare Verfahren synthetisiert werden können und ermöglicht darüber hinaus insgesamt eine relativ einfache Verfahrensführung auch bei großtechnischer Umsetzung.

Im Gegensatz zu herkömmlichen Herstellungsverfahren des Standes der Technik kommt das erfindungsgemäße Herstellungsverfahren ohne komplexe Edukte und ohne die Verwendung von Schutzgruppen aus. Dennoch werden im Rahmen des erfindungsgemäßen Herstellungsverfahrens exzellente Ausbeuten erzielt, wobei in gleicher Weise die Bildung von Nebenprodukten minimiert bzw. vermieden wird.

Darüber hinaus ist das erfindungsgemäße Verfahren einfach und wirtschaftlich. Insbesondere wird das erfindungsgemäße Verfahren in Abwesenheit von Lösemitteln und/oder ohne jedwedes Lösemittel durchgeführt (d. h. also als Reaktion in Masse bzw. als Reaktion in Substanz bzw. als sogenannte *Bulk Reaction*); folglich sind die erhaltenen Reaktionsprodukte nicht mit Lösemittel verunreinigt und es muss kein Lösemittel nach Durchführung des Verfahrens bzw. der Reaktion aufwendig und energieträchtig entfernt und entsorgt bzw. rezykliert werden. Es werden zudem auch keine toxischen Nebenprodukte gebildet.

Das erfindungsgemäße Herstellungsverfahren führt üblicherweise zu einem Gemisch verschiedener Polyglycerinester von mit Oxobutanol veresterten Polycarbonsäuren, insbesondere Polyglycerinester von mit 4-Oxo-2-butanol veresterten Polycarbonsäuren, vorzugsweise ein- oder mehrwertiger Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, d. h. zu einem Gemisch von mindestens zwei voneinander verschiedenen Polyglycerinestern von mit Oxobutanol veresterten Polycarbonsäuren, insbesondere Polyglycerinestern von mit 4-Oxo-2-butanol veresterten Polycarbonsäuren, vorzugsweise ein- oder mehrwertigen Polyglycerinestern von mit 4-Oxo-4-(C₁-C₅-alkoxy)-2-butanol oder mit 4-Oxo-4-(hydroxy-C₃-C₅-alkoxy)-2-butanol veresterten Polycarbonsäuren. Das resultierende Rohreaktionsprodukt bzw. Rohgemisch kann mit an sich bekannten Methoden ohne Weiteres aufgereinigt werden, insbesondere von gegebenenfalls noch vorhandenen Edukten und/oder gegebenenfalls vorhandenen Nebenprodukten befreit werden, und darüber hinaus - sofern gewünscht - mit ebenfalls an sich bekannten Methoden aufgespalten werden, insbesondere destillativ und/oder chromatographisch (z. B. Fraktionierung in die einzelnen Polyglycerinester von mit Oxobutanol veresterten Polycarbonsäuren, d. h. z. B. Trennung des entsprechenden Monoesters, Diesters etc., oder aber Fraktionierung in Fraktionen mit angereicherten und abgereicherten Anteilen einzelner etc.).

Wie zuvor ausgeführt, betrifft die vorliegende Erfindung also gemäß dem ersten Aspekt ein Verfahren zur Herstellung von Polyglycerinestern von mit Oxobutanol veresterten Polycarbonsäuren, insbesondere ein- oder mehrwertigen Polyglycerinestern von mit 4-Oxo-4-(C₁-C₅-alkoxy)-2-butanol oder mit 4-Oxo-4-(hydroxy-C₃-C₅-alkoxy)-2-butanol veresterten Polycarbonsäuren,
wobei
(i) mindestens ein Oxobutanol, insbesondere ein 3-Hydroxybutanoat, bevorzugt ein 4-Oxo-2-butanol, vorzugsweise ein 4-Oxo-4-(C₁-C₅-alkoxy)-2-butanol oder ein 4-Oxo-4-(hydroxy-C₃-C₅-alkoxy)-2-butanol, der allgemeinen Formel (I)

   CH₃-CH(OH)-CH₂-C(O)OR¹ (I)

   wobei in der allgemeinen Formel (I) der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt,
(ii) mindestens eine Polycarbonsäure (II), insbesondere mindestens eine mindestens zwei Carboxylgruppen enthaltende Polycarbonsäure, wobei die Polycarbonsäure (II) in Form der freien Polycarbonsäure, in Form eines Salzes der Polycarbonsäure, in Form eines Polycarbonsäureesters oder in Form des Polycarbonsäureanhydrids eingesetzt wird, und
(iii) mindestens ein Polyglycerin (III)

miteinander umgesetzt und/oder zur Reaktion gebracht werden,
wobei das Verfahren einstufig oder aber mehrstufig durchgeführt wird, und
wobei das Verfahren in Abwesenheit von Lösemitteln und/oder ohne jedwedes Lösemittel durchgeführt wird, und
wobei bei Verwendung der Polycarbonsäure (II) in Form der freien Säure bei der Umsetzung gleichzeitig Wasser gebildet wird, wobei das Wasser der Umsetzung kontinuierlich entzogen wird; und/oder
wobei bei Verwendung der Polycarbonsäure (II) in Form des Anhydrids die entsprechende freie Polycarbonsäure (II) und Wasser gebildet werden, wobei die entstehende freie Polycarbonsäure (II) weiter umgesetzt wird oder nach erfolgter Reaktion entfernt und gegebenenfalls rezykliert wird und wobei das Wasser der Umsetzung kontinuierlich entzogen wird; und/oder
wobei bei Verwendung der Polycarbonsäure (II) in Form des Esters der entsprechende Esteralkohol und Wasser gebildet werden, wobei der entstehende Esteralkohol und Wasser der Umsetzung kontinuierlich entzogen werden,
so dass als Reaktionsprodukt (IV) ein oder mehrere Polyglycerinester von mit Oxobutanol veresterten Polycarbonsäuren, insbesondere ein oder mehrere Polyglycerinester von mit 4-Oxo-2-butanol veresterten Polycarbonsäuren, vorzugsweise ein oder mehrere ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, erhalten werden.

Das erfindungsgemäße Verfahren wird einstufig, insbesondere als Eintopfsynthese, oder aber mehrstufig, insbesondere zweistufig, bevorzugt mehrstufig, insbesondere zweistufig, besonders bevorzugt zweistufig, durchgeführt.

Gemäß einer Ausführungsform der vorliegenden Erfindung kann das Verfahren mehrstufig, insbesondere zweistufig, durchgeführt werden,
wobei
(a) in einem ersten Verfahrensschritt (a) mindestens ein Oxobutanol, insbesondere ein 3-Hydroxybutanoat, bevorzugt ein 4-Oxo-2-butanol, vorzugsweise ein 4-Oxo-4-(C₁-C₅-alkoxy)-2-butanol oder ein 4-Oxo-4-(hydroxy-C₃-C₅-alkoxy)-2-butanol, der allgemeinen Formel (I)

   CH₃-CH(OH)-CH₂-C(O)OR¹ (I)

   wobei in der allgemeinen Formel (I) der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt,
   mit mindestens einer Polycarbonsäure (II), insbesondere mit mindestens einer mindestens zwei Carboxylgruppen enthaltenden Polycarbonsäure, wobei die Polycarbonsäure (II) in Form der freien Polycarbonsäure, in Form eines Salzes der Polycarbonsäure, in Form eines Polycarbonsäureesters oder in Form des Polycarbonsäureanhydrids eingesetzt wird, umgesetzt und/oder zur Reaktion gebracht wird, insbesondere in einer Veresterungsreaktion und/oder unter Veresterungsbedingungen umgesetzt und/oder zur Reaktion gebracht wird,
   insbesondere sodass als Reaktionszwischenprodukt (IV') des Verfahrensschritts (a) ein oder mehrere Polycarbonsäureester von Oxobutanol, insbesondere ein oder mehrere Polycarbonsäureester von 3-Hydroxybutanoat, bevorzugt ein oder mehrere Polycarbonsäureester von 4-Oxo-2-butanol, vorzugsweise ein oder mehrere Polycarbonsäureester von 4-Oxo-4-(C₁-C₅-alkoxy)-2-butanol oder von 4-Oxo-4-(hydroxy-C₃-C₅-alkoxy)-2-butanol, erhalten werden; und
(b) nachfolgend in einem zweiten Verfahrensschritt (b) das in Verfahrensschritt (a) erhaltene Reaktionszwischenprodukt (IV') mit mindestens einem Polyglycerin (III) umgesetzt und/oder zur Reaktion gebracht wird, insbesondere in einer Veresterungsreaktion und/oder unter Veresterungsbedingungen umgesetzt und/oder zur Reaktion gebracht wird,

wobei das Verfahren in Abwesenheit von Lösemitteln und/oder ohne jedwedes Lösemittel durchgeführt wird, und
wobei bei Verwendung der Polycarbonsäure (II) in Form der freien Säure bei der Umsetzung gleichzeitig Wasser gebildet wird, wobei das Wasser der Umsetzung kontinuierlich entzogen wird; und/oder
wobei bei Verwendung der Polycarbonsäure (II) in Form des Anhydrids die entsprechende freie Polycarbonsäure (II) und Wasser gebildet werden, wobei die entstehende freie Polycarbonsäure (II) weiter umgesetzt wird oder nach erfolgter Reaktion entfernt und gegebenenfalls rezykliert wird und wobei das Wasser der Umsetzung kontinuierlich entzogen wird; und/oder
wobei bei Verwendung der Polycarbonsäure (II) in Form des Esters der entsprechende Esteralkohol und Wasser gebildet werden, wobei der entstehende Esteralkohol und Wasser der Umsetzung kontinuierlich entzogen werden.

Gemäß einer besonderen Ausführungsform betrifft die vorliegende Erfindung gemäß diesem Erfindungsaspekt auch ein Verfahren zur Herstellung von Polyglycerinestern von mit Oxobutanol veresterten Polycarbonsäuren, insbesondere ein- oder mehrwertigen Polyglycerinestern von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, insbesondere ein wie zuvor beschriebenes Verfahren,
wobei:
(a) in einem ersten Verfahrensschritt (a) mindestens ein Oxobutanol, insbesondere ein 3-Hydroxybutanoat, bevorzugt ein 4-Oxo-2-butanol, vorzugsweise ein 4-Oxo-4-(C₁-C₅-alkoxy)-2-butanol oder ein 4-Oxo-4-(hydroxy-C₃-C₅-alkoxy)-2-butanol, der allgemeinen Formel (I)

   CH₃-CH(OH)-CH₂-C(O)OR¹ (I)

   wobei in der allgemeinen Formel (I) der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt,
   mit mindestens einer Polycarbonsäure (II), insbesondere mit mindestens einer mindestens zwei Carboxylgruppen enthaltenden Polycarbonsäure, wobei die Polycarbonsäure (II) in Form der freien Polycarbonsäure, in Form eines Salzes der Polycarbonsäure, in Form eines Polycarbonsäureesters oder in Form des Polycarbonsäureanhydrids eingesetzt wird, umgesetzt und/oder zur Reaktion gebracht wird, insbesondere in einer Veresterungsreaktion und/oder unter Veresterungsbedingungen umgesetzt und/oder zur Reaktion gebracht wird,
   insbesondere sodass als Reaktionszwischenprodukt (IV') des Verfahrensschritts (a) ein oder mehrere Polycarbonsäureester von Oxobutanol, insbesondere ein oder mehrere Polycarbonsäureester von 3-Hydroxybutanoat, bevorzugt ein oder mehrere Polycarbonsäureester von 4-Oxo-2-butanol, vorzugsweise ein oder mehrere Polycarbonsäureester von 4-Oxo-4-(C₁-C₅-alkoxy)-2-butanol oder von 4-Oxo-4-(hydroxy-C₃-C₅-alkoxy)-2-butanol, erhalten werden; und
(b) nachfolgend in einem zweiten Verfahrensschritt (b) das in Verfahrensschritt (a) erhaltene Reaktionszwischenprodukt (IV') mit mindestens einem Polyglycerin (III) umgesetzt und/oder zur Reaktion gebracht wird, insbesondere in einer Veresterungsreaktion und/oder unter Veresterungsbedingungen umgesetzt und/oder zur Reaktion gebracht wird,
   so dass als Reaktionsprodukt (IV) ein oder mehrere Polyglycerinester von mit Oxobutanol veresterten Polycarbonsäuren, insbesondere ein oder mehrere Polyglycerinester von mit 4-Oxo-2-butanol veresterten Polycarbonsäuren, vorzugsweise ein oder mehrere ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, erhalten werden,

wobei das Verfahren in Abwesenheit von Lösemitteln und/oder ohne jedwedes Lösemittel durchgeführt wird, und
wobei bei Verwendung der Polycarbonsäure (II) in Form der freien Säure bei der Umsetzung gleichzeitig Wasser gebildet wird, wobei das Wasser der Umsetzung kontinuierlich entzogen wird; und/oder
wobei bei Verwendung der Polycarbonsäure (II) in Form des Anhydrids die entsprechende freie Polycarbonsäure (II) und Wasser gebildet werden, wobei die entstehende freie Polycarbonsäure (II) weiter umgesetzt wird oder nach erfolgter Reaktion entfernt und gegebenenfalls rezykliert wird und wobei das Wasser der Umsetzung kontinuierlich entzogen wird; und/oder
wobei bei Verwendung der Polycarbonsäure (II) in Form des Esters der entsprechende Esteralkohol und Wasser gebildet werden, wobei der entstehende Esteralkohol und Wasser der Umsetzung kontinuierlich entzogen werden.

Gemäß einer besonderen Ausführungsform der vorliegenden Erfindung kann die Verbindung der allgemeinen Formel (I) in racemischer Form oder in Form des (R)-Enantiomers eingesetzt werden. Die (R)-Konfiguration bezieht sich auf das chirale Kohlenstoffatom in 3-Position der Verbindung der allgemeinen Formel (I).

Erfindungsgemäß bevorzugt ist es, wenn in der allgemeinen Formel (I) der Rest R¹ Ethyl darstellt.

Mit anderen Worten ist es erfindungsgemäß bevorzugt, dass als Verbindung der allgemeinen Formel (I) Ethyl-3-hydroxybutyrat (synonym auch als 3-Hydroxybuttersäureethylester bzw. 4-Ethoxy-4-oxo-2-butanol bezeichnet) der Formel CH₃-CH(OH)-CH₂-C(O)OC₂H₅ eingesetzt wird.

Dies ermöglicht eine besonders effiziente Verfahrensführung und hohe Ausbeuten mit minimierter bzw. unterdrückter Nebenproduktbildung. Zudem ist der 3-Hydroxybuttersäureethylester bzw. 4-Ethoxy-4-oxo-2-butanol auch in großen Mengen kommerziell verfügbar und kann insbesondere großtechnisch (z. B. durch Claisen-Kondensation von Ethylacetat) ohne Weiteres gewonnen werden.

Gemäß der vorliegenden Erfindung wird die Polycarbonsäure (II) in Form der freien Polycarbonsäure, in Form eines Salzes der Polycarbonsäure, in Form eines Polycarbonsäureesters oder in Form des Polycarbonsäureanhydrids, insbesondere in Form der freien Polycarbonsäure oder in Form des Polycarbonsäureanhydrids, bevorzugt in Form des Polycarbonsäureanhydrids, besonders bevorzugt in Form eines cyclischen Polycarbonsäureanhydrids, eingesetzt.

Die Anhydride der Polycarbonsäure sind besonders reaktiv und eignen sich insbesondere für Veresterungsreaktionen. Bei der Verwendung von cyclischen Anhydriden werden im Rahmen einer Veresterungsreaktion keine Abspaltprodukte gebildet, welche gegebenenfalls energieträchtig entfernt werden müssten.

Gemäß einer weiteren besonderen Ausführungsform der vorliegenden Erfindung kann die Polycarbonsäure (II) der allgemeinen Formel (IIa)

HOOC-X-COOH (IIa)

entsprechen, wobei in der allgemeinen Formel (IIa) X einen 1 bis 10, vorzugsweise 2 bis 6 Kohlenstoffatome enthaltenden und gegebenenfalls ein- oder mehrfach substituierten, insbesondere mit einem oder mehreren Hydroxyl-Resten und/oder Carboxyl-Resten substituierten, gesättigten oder ungesättigten organischen Rest darstellt;
insbesondere wobei mindestens eine Carboxylgruppe (COOH-Gruppe), vorzugsweise beide Carboxylgruppen (COOH-Gruppen), endständig ist/sind und/oder eine primäre Carboxylgruppe (COOH-Gruppe) ist/sind.

In diesem Zusammenhang ist es insbesondere bevorzugt, wenn in der allgemeinen Formel (IIa) X einen 2 bis 6 Kohlenstoffatome enthaltenden und gegebenenfalls ein- oder mehrfach substituierten, insbesondere mit einem oder mehreren Hydroxyl-Resten und/oder Carboxyl-Resten substituierten, gesättigten oder ungesättigten organischen Rest darstellt;
insbesondere wobei mindestens eine Carboxylgruppe (COOH-Gruppe), vorzugsweise beide Carboxylgruppen (COOH-Gruppen), endständig ist/sind und/oder eine primäre Carboxylgruppe (COOOH-Gruppe) ist/sind.

Insbesondere durch die Verwendung einer zuvor definierten Carbonsäure mit mindestens einer, vorzugsweise zwei endständigen bzw. primären Carboxylgruppen kann die Veresterungsreaktion zwischen der Polycarbonsäure (II) und dem Oxobutanol der allgemeinen Formel (I) besonders effektiv und mit minimierter Nebenproduktbildung ablaufen, ohne dass extreme Reaktionsbedingungen (z. B. sehr hohe Temperatur, sehr niedriger Druck etc.) notwendig sind. Darüber hinaus kann durch die Anzahl der vorhandenen Carboxylgruppen die Wirkstoffdichte (d. h. insbesondere die 3-Hydroxybutanoat-Dichte) beeinflusst werden.

Im Rahmen des erfindungsgemäßen Verfahrens kann es bevorzugt sein, wenn die Polycarbonsäure (II) ausgewählt ist aus der Gruppe von Bernsteinsäure, Weinsäure, Citronensäure, Äpfelsäure, Adipinsäure, Fumarsäure und Maleinsäure und deren Anhydriden sowie deren Kombinationen oder Mischungen, insbesondere ausgewählt ist aus der Gruppe von Bernsteinsäure, Weinsäure, Citronensäure, Äpfelsäure, Adipinsäure und Fumarsäure und deren Anhydriden sowie deren Kombinationen oder Mischungen, bevorzugt ausgewählt ist aus der Gruppe von Bernsteinsäure und Adipinsäure und deren Anhydriden sowie deren Kombinationen oder Mischungen.

Die zuvor genannten Carbonsäuren eignen sich besonders für die Umsetzung mit 3-Hydroxybutanoaten und sind zudem kommerziell erhältlich.

Insbesondere ist es im Rahmen des erfindungsgemäßen Verfahrens bevorzugt, wenn die Polycarbonsäure (II) eine natürlich vorkommende Carbonsäure oder deren Anhydrid oder Derivat, insbesondere Umsetzungsprodukt, ist, insbesondere eine im menschlichen und/oder tierischen Stoffwechsel vorkommende Carbonsäure oder deren Anhydrid oder Derivat, insbesondere Umsetzungsprodukt. Insbesondere ist es in diesem Zusammenhang vorteilhaft, wenn Carbonsäuren oder deren Anhydride oder Derivate verwendet werden, welche im Citratzyklus vorkommen, aus dem Citratzyklus entstehen oder mit dem Citratzyklus im Zusammenhang stehen. Dabei können Derivate beispielsweise Salze oder Ester darstellen, welche durch Oxidation eines Stoffwechselprodukts (beispielsweise aus dem Citratzyklus) erhältlich werden. Durch die Verwendung von Carbonsäuren oder deren Anhydriden oder Derivaten, welche Teil des menschlichen und/oder tierischen Stoffwechsels bzw. Edukts bzw. Produkts bzw. Zwischenprodukts eines menschlichen und/oder tierischen Stoffwechsels darstellen, kann bei der Verwendung des erfindungsgemäßen Reaktionsprodukts dem menschlichen und/oder tierischen Körper eine weitere Energiequelle (zusätzlich zu dem Ketokörper 3-Hydroxybuttersäure bzw. 3-Hydroxybutanoat) bereitgestellt werden. Die erfindungsgemäßen Reaktionsprodukte sind besonders geeignet für die Verwendung in oder als Arzneimittel, Medikament oder Nahrungsmittel- und Lebensmittelerzeugnis.

Weiterhin kann es im Rahmen des erfindungsgemäßen Verfahrens auch bevorzugt sein, wenn die Polycarbonsäure (II) ein lebensmittelrechtlich zugelassener Inhaltsstoff, insbesondere Zusatzstoff, ist.

Lebensmittelrechtlich zugelassene Inhaltsstoffe bzw. Zusatzstoffe sind zur Verwendung in Lebensmitteln in bestimmten Mengen zugelassen und weisen keine Gesundheitsrisiken auf. EU-weit wird eine Liste für Lebensmittelzusatzstoffe geführt, worin jeder Lebensmittelzusatzstoff eine eigene Kennzeichnung (sogenannte E-Nummer) erhält. Beispielsweise werden die folgenden Carbonsäuren in der Lebensmittelzusatzstoffliste geführt: Bernsteinsäure (E363), Weinsäure (E334), Citronensäure (E330), Äpfelsäure (E296), Adipinsäure (E355) und Fumarsäure (E297). Diese Säuren sind alle Teil des Citratzyklus oder durch Oxidation eines Stoffwechselprodukts des Citratzyklus erhältlich. Der Citratzyklus ist ein Kreislauf biochemischer Reaktionen, der eine wichtige Rolle im Stoffwechsel (Metabolismus) aerober Zellen von Lebewesen spielt und hauptsächlich dem oxidativen Abbau organischer Stoffe zum Zweck der Energiegewinnung und der Bereitstellung von Zwischenprodukten für Biosynthesen dient. Somit können die Säuren, welche bei Verwendung des aus dem erfindungsgemäßen Verfahren erhältlichen Reaktionsprodukts (IV) durch Abbau gebildet werden, als eine weitere alternative Energiequelle vom Körper verwertet werden.

Gemäß einer besonderen Ausführungsform des erfindungsgemäßen Verfahrens kann das Polyglycerin der allgemeinen Formel (Illa)

HO-CH₂-CH(OH)-CH₂-[O-CH₂-CH(OH)-CH₂]ₚ-OH (IIIa)

entsprechen, wobei in der allgemeinen Formel (Illa) die Variable p eine ganze Zahl von 1 bis 6, insbesondere von 1 bis 4, vorzugsweise 1 oder 2, besonders bevorzugt 1, darstellt.

Gemäß einer weiteren besonderen Ausführungsform des erfindungsgemäßen Verfahrens kann das Polyglycerin (III) ein Diglycerin der Formel (Illb)

HO-CH₂-CH(OH)-CH₂-O-CH₂-CH(OH)-CH₂-OH (IIIb)

sein.

Insbesondere ist das Polyglycerin (III) kein Propan-1,2,3-triol (Glycerin).

Gemäß einer besonderen Ausführungsform betrifft die vorliegende Erfindung gemäß diesem Erfindungsaspekt ein Verfahren zur Herstellung von Polyglycerinestern von mit Oxobutanol veresterten Polycarbonsäuren, insbesondere ein- oder mehrwertigen Polyglycerinestern von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, insbesondere ein wie zuvor beschriebenes Verfahren,
wobei:
(a) in einem ersten Verfahrensschritt (a) mindestens ein Oxobutanol, insbesondere ein 3-Hydroxybutanoat, bevorzugt ein 4-Oxo-2-butanol, vorzugsweise ein 4-Oxo-4-(C₁-C₅-alkoxy)-2-butanol oder ein 4-Oxo-4-(hydroxy-C₃-C₅-alkoxy)-2-butanol, der allgemeinen Formel (I)

   CH₃-CH(OH)-CH₂-C(O)OR¹ (I)

   wobei in der allgemeinen Formel (I) der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt,
   mit mindestens einer Polycarbonsäure (II), insbesondere mit mindestens einer mindestens zwei Carboxylgruppen enthaltenden Polycarbonsäure, wobei die Polycarbonsäure (II) in Form der freien Polycarbonsäure, in Form eines Salzes der Polycarbonsäure, in Form eines Polycarbonsäureesters oder in Form des Polycarbonsäureanhydrids eingesetzt wird, umgesetzt und/oder zur Reaktion gebracht wird, insbesondere in einer Veresterungsreaktion und/oder unter Veresterungsbedingungen umgesetzt und/oder zur Reaktion gebracht wird,
   insbesondere sodass als Reaktionszwischenprodukt (IV') des Verfahrensschritts (a) ein oder mehrere Polycarbonsäureester von Oxobutanol, insbesondere ein oder mehrere Polycarbonsäureester von 3-Hydroxybutanoat, bevorzugt ein oder mehrere Polycarbonsäureester von 4-Oxo-2-butanol, vorzugsweise ein oder mehrere Polycarbonsäureester von 4-Oxo-4-(C₁-C₅-alkoxy)-2-butanol oder von 4-Oxo-4-(hydroxy-C₃-C₅-alkoxy)-2-butanol, erhalten werden; und
(b) nachfolgend in einem zweiten Verfahrensschritt (b) das in Verfahrensschritt (a) erhaltene Reaktionszwischenprodukt (IV') mit mindestens einem Polyglycerin der allgemeinen Formel (Illa)

   HO-CH₂-CH(OH)-CH₂-[O-CH₂-CH(OH)-CH₂]ₚ-OH (IIIa)

   wobei in der allgemeinen Formel (Illa) die Variable p eine ganze Zahl von 1 bis 6, insbesondere von 1 bis 4, vorzugsweise 1 oder 2, besonders bevorzugt 1, darstellt,
   umgesetzt und/oder zur Reaktion gebracht wird, insbesondere in einer Veresterungsreaktion und/oder unter Veresterungsbedingungen umgesetzt und/oder zur Reaktion gebracht wird,
   so dass als Reaktionsprodukt (IV) ein oder mehrere Polyglycerinester von mit Oxobutanol veresterten Polycarbonsäuren, insbesondere ein oder mehrere Polyglycerinester von mit 4-Oxo-2-butanol veresterten Polycarbonsäuren, vorzugsweise ein oder mehrere ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, erhalten werden,

wobei das Verfahren in Abwesenheit von Lösemitteln und/oder ohne jedwedes Lösemittel durchgeführt wird, und
wobei bei Verwendung der Polycarbonsäure (II) in Form der freien Säure bei der Umsetzung gleichzeitig Wasser gebildet wird, wobei das Wasser der Umsetzung kontinuierlich entzogen wird; und/oder
wobei bei Verwendung der Polycarbonsäure (II) in Form des Anhydrids die entsprechende freie Polycarbonsäure (II) und Wasser gebildet werden, wobei die entstehende freie Polycarbonsäure (II) weiter umgesetzt wird oder nach erfolgter Reaktion entfernt und gegebenenfalls rezykliert wird und wobei das Wasser der Umsetzung kontinuierlich entzogen wird; und/oder
wobei bei Verwendung der Polycarbonsäure (II) in Form des Esters der entsprechende Esteralkohol und Wasser gebildet werden, wobei der entstehende Esteralkohol und Wasser der Umsetzung kontinuierlich entzogen werden.

Gemäß einer weiteren bevorzugten Ausführungsform betrifft die vorliegende Erfindung gemäß diesem Erfindungsaspekt auch ein Verfahren zur Herstellung von Polyglycerinestern von mit Oxobutanol veresterten Polycarbonsäuren, insbesondere ein- oder mehrwertigen Polyglycerinestern von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, insbesondere ein wie zuvor beschriebenes Verfahren,
wobei:
(a) in einem ersten Verfahrensschritt (a) mindestens ein Oxobutanol, insbesondere ein 3-Hydroxybutanoat, bevorzugt ein 4-Oxo-2-butanol, vorzugsweise ein 4-Oxo-4-(C₁-C₅-alkoxy)-2-butanol oder ein 4-Oxo-4-(hydroxy-C₃-C₅-alkoxy)-2-butanol, der allgemeinen Formel (I)

   CH₃-CH(OH)-CH₂-C(O)OR¹ (I)

   wobei in der allgemeinen Formel (I) der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt,
   mit mindestens einer Polycarbonsäure (II), ausgewählt aus der Gruppe von Bernsteinsäure, Weinsäure, Citronensäure, Äpfelsäure, Adipinsäure, Fumarsäure und Maleinsäure und deren Anhydriden sowie deren Kombinationen oder Mischungen, insbesondere ausgewählt aus der Gruppe von Bernsteinsäure, Weinsäure, Citronensäure, Äpfelsäure, Adipinsäure und Fumarsäure und deren Anhydriden sowie deren Kombinationen oder Mischungen, bevorzugt ausgewählt aus der Gruppe von Bernsteinsäure und Adipinsäure und deren Anhydriden sowie deren Kombinationen oder Mischungen, umgesetzt und/oder zur Reaktion gebracht wird, insbesondere in einer Veresterungsreaktion und/oder unter Veresterungsbedingungen umgesetzt und/oder zur Reaktion gebracht wird,
   insbesondere sodass als Reaktionszwischenprodukt (IV') des Verfahrensschritts (a) ein oder mehrere Polycarbonsäureester von Oxobutanol, insbesondere ein oder mehrere Polycarbonsäureester von 3-Hydroxybutanoat, bevorzugt ein oder mehrere Polycarbonsäureester von 4-Oxo-2-butanol, vorzugsweise ein oder mehrere Polycarbonsäureester von 4-Oxo-4-(C₁-C₅-alkoxy)-2-butanol oder von 4-Oxo-4-(hydroxy-C₃-C₅-alkoxy)-2-butanol, erhalten werden; und
(b) nachfolgend in einem zweiten Verfahrensschritt (b) das in Verfahrensschritt (a) erhaltene Reaktionszwischenprodukt (IV') mit mindestens einem Polyglycerin der allgemeinen Formel (Illa)

   HO-CH₂-CH(OH)-CH₂-[O-CH₂-CH(OH)-CH₂]ₚ-OH (IIIa)

   wobei in der allgemeinen Formel (Illa) die Variable p eine ganze Zahl von 1 bis 6, insbesondere von 1 bis 4, vorzugsweise 1 oder 2, besonders bevorzugt 1, darstellt,
   umgesetzt und/oder zur Reaktion gebracht wird, insbesondere in einer Veresterungsreaktion und/oder unter Veresterungsbedingungen umgesetzt und/oder zur Reaktion gebracht wird,
   so dass als Reaktionsprodukt (IV) ein oder mehrere Polyglycerinester von mit Oxobutanol veresterten Polycarbonsäuren, insbesondere ein oder mehrere Polyglycerinester von mit 4-Oxo-2-butanol veresterten Polycarbonsäuren, vorzugsweise ein oder mehrere ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, erhalten werden,

wobei das Verfahren in Abwesenheit von Lösemitteln und/oder ohne jedwedes Lösemittel durchgeführt wird, und
wobei bei Verwendung der Polycarbonsäure (II) in Form der freien Säure bei der Umsetzung gleichzeitig Wasser gebildet wird, wobei das Wasser der Umsetzung kontinuierlich entzogen wird; und/oder
wobei bei Verwendung der Polycarbonsäure (II) in Form des Anhydrids die entsprechende freie Polycarbonsäure (II) und Wasser gebildet werden, wobei die entstehende freie Polycarbonsäure (II) weiter umgesetzt wird oder nach erfolgter Reaktion entfernt und gegebenenfalls rezykliert wird und wobei das Wasser der Umsetzung kontinuierlich entzogen wird; und/oder
wobei bei Verwendung der Polycarbonsäure (II) in Form des Esters der entsprechende Esteralkohol und Wasser gebildet werden, wobei der entstehende Esteralkohol und Wasser der Umsetzung kontinuierlich entzogen werden.

Gemäß der vorliegenden Erfindung wird das Verfahren in Abwesenheit von Lösemitteln und/oder ohne jedwedes Lösemittel durchgeführt.

Gemäß der vorliegenden Erfindung wird insbesondere der erste Verfahrensschritt (a) und/oder der zweite Verfahrensschritt (b), insbesondere der erste Verfahrensschritt (a) und der zweite Verfahrensschritt (b), in Abwesenheit von Lösemitteln und/oder ohne jedwedes Lösemittel durchgeführt.

D. h. das Verfahren bzw. der erste Verfahrensschritt (a) und der zweite Verfahrensschritt (b) wird/werden also als Reaktion in Masse bzw. als Reaktion in Substanz bzw. als sogenannte *Bulk Reaction* durchgeführt. Dies hat den Vorteil, dass die erhaltenen Reaktionsprodukte nicht mit Lösemittel verunreinigt sind und kein Lösemittel nach Durchführung des Verfahrens bzw. der Reaktion aufwendig und energieträchtig entfernt und entsorgt bzw. rezykliert werden muss. Überraschenderweise verläuft das Verfahren bzw. die Reaktion dennoch mit hohen Umsätzen und Ausbeuten und zumindest im Wesentlichen ohne signifikante Nebenproduktbildung.

Gemäß einer Ausführungsform der vorliegenden Erfindung wird das Verfahren einstufig durchgeführt.

Gemäß einer besonderen Ausführungsform ist es möglich, dass das erfindungsgemäße Verfahren in Abwesenheit eines Katalysators und/oder ohne jedweden Katalysator durchgeführt werden kann oder aber in Gegenwart eines Katalysators, insbesondere eines Enzyms und/oder eines metallhaltigen und/oder metallbasierten, sauren oder basischen Katalysators, durchgeführt werden kann (insbesondere wobei der Katalysator nach der Umsetzung rezykliert werden kann). Wie zuvor ausführt, kann gemäß einer besonderen Ausführungsform des erfindungsgemäßen Herstellungsverfahrens das Verfahren in Abwesenheit eines Katalysator und/oder ohne jedweden Katalysator durchgeführt werden.

Sofern die Umsetzung in Abwesenheit eines Katalysators und/oder ohne jedweden Katalysator durchgeführt wird, ist es bevorzugt, wenn die Umsetzung bei Temperaturen im Bereich von 20 °C bis 160°C, insbesondere im Bereich von 50 °C bis 150°C, vorzugsweise im Bereich von 70 °C bis 140°C, besonders bevorzugt im Bereich von 80 °C bis 135 °C, ganz besonders bevorzugt im Bereich von 100 °C bis 130 °C, durchgeführt wird.

Im Fall der Umsetzung in Abwesenheit eines Katalysators kann der angewendete Druckbereich in weiten Bereichen variieren. Insbesondere kann die Umsetzung in Abwesenheit eines Katalysators und/oder ohne jedweden Katalysator bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt werden.

Bei der Umsetzung in Abwesenheit eines Katalysators, ist es bevorzugt, wenn die Umsetzung in Gegenwart eines Inertgases, insbesondere in Gegenwart von Helium, Argon oder Stickstoff, bevorzugt in Gegenwart von Stickstoff, durchgeführt wird. Insbesondere können hierdurch unerwünschte Nebenreaktionen, insbesondere aufgrund von Oxidation oder Hydrolyse, verhindert werden.

Alternativ zu dieser besonderen Ausführungsform ist es aber auch möglich, die Umsetzung in Gegenwart eines Enzyms als Katalysator durchzuführen.

Dabei kann das Enzym insbesondere ausgewählt sein aus Synthetasen (Ligasen), Katalasen, Esterasen, Lipasen und deren Kombinationen. Erfindungsgemäß werden als Synthetasen (synonym Ligasen) insbesondere Enzyme aus der Klasse der Ligasen bezeichnet; Ligasen sind Enzyme, welche das Verknüpfen zweier oder mehrerer Moleküle durch eine kovalente Bindung katalysieren. Katalasen im Sinne der vorliegenden Erfindung sind insbesondere Enzyme, welche Wasserstoffperoxid zu Sauerstoff und Wasser umzusetzen imstande sind. Der Begriff der Esterasen bezeichnet insbesondere Enzyme, welche imstande sind, Ester hydrolytisch in Alkohol und Säure aufzuspalten (Verseifung); es handelt sich somit insbesondere um Hydrolasen, wobei fettspaltende Esterasen auch als Lipasen bezeichnet werden. Lipasen im Sinne der vorliegenden Erfindung sind insbesondere Enzyme, welche von Lipiden, wie Glyceriden, freie Fettsäuren abzuspalten imstand sind (Lipolyse).

In diesem Zusammenhang kann sich das als Katalysator eingesetzte Enzym insbesondere ableiten von *Candida antarctica, Mucor miehei (Rhizomucor miehei), Thermomyces lanuginosus, Candida rugosa, Aspergillus oryzae, Pseudomonas cepacia, Pseudomonas fluorescens, Rhizopus delemar* und *Pseudomonas* sp. sowie deren Kombinationen, vorzugsweise von *Candida antarctica, Mucor miehei (Rhizomucor miehei)* und *Thermomyces lanuginosus.*

Gemäß einer besonderen Ausführungsform kann das Enzym in immobilisierter Form, insbesondere immobilisiert auf einem Träger, vorzugsweise auf einem polymeren Träger, bevorzugt auf einem polymeren organischen Träger, besonders bevorzugt mit hydrophoben Eigenschaften, ganz besonders bevorzugt auf einem poly(meth)acrylharzbasierten Träger, eingesetzt werden.

Im Rahmen der vorliegenden Erfindung ist es bevorzugt, wenn im Fall der Verwendung eines Enzyms als Katalysator das Enzym nach der Umsetzung rezykliert wird.

Sofern die Umsetzung im Rahmen des erfindungsgemäßen Herstellungsverfahrens in Gegenwart eines Enzyms als Katalysator durchgeführt wird, ist es bevorzugt, wenn die Umsetzung bei Temperaturen im Bereich von 10 °C bis 80 °C, insbesondere im Bereich von 20 °C bis 80 °C, vorzugsweise im Bereich von 25 °C bis 75 °C, besonders bevorzugt im Bereich von 45 °C bis 75 °C, ganz besonders bevorzugt im Bereich von 50 °C bis 70 °C, durchgeführt wird.

Im Fall der Verwendung eines Enzyms als Katalysator kann die Menge des eingesetzten Enzyms in weiten Bereichen variieren. Insbesondere kann das Enzym in Mengen, bezogen auf die Gesamtmenge der Ausgangsverbindungen (I), (II) und (III), im Bereich von 0,001 Gew.-% bis 20 Gew.-%, insbesondere im Bereich von 0,01 Gew.-% bis 15 Gew.-%, vorzugsweise im Bereich von 0,1 Gew.-% bis 15 Gew.-%, bevorzugt im Bereich von 0,5 Gew.-% bis 10 Gew.-%, eingesetzt werden. Dennoch kann es einzelfallbedingt oder anwendungsbezogen erforderlich sein, von den vorgenannten Mengen abzuweichen, ohne dass der Rahmen der vorliegenden Erfindung verlassen ist.

Wenn gemäß einer besonderen Ausführungsform der vorliegenden Erfindung die Umsetzung in Gegenwart eines Enzyms als Katalysator durchgeführt wird, kann auch der angewendete Druckbereich in weiten Bereichen variieren. Typischerweise kann die Umsetzung in Gegenwart eines Enzyms als Katalysator bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 0,5 bar, durchgeführt werden.

Gemäß der besonderen Ausführungsform der vorliegenden Erfindung, wonach die Umsetzung in Gegenwart eines Enzyms als Katalysator durchgeführt wird, ist es bevorzugt, wenn die Umsetzung in Gegenwart eines Inertgases, insbesondere in Gegenwart von Helium, Argon oder Stickstoff, bevorzugt in Gegenwart von Stickstoff, durchgeführt wird. Wie zuvor bereits im Zusammenhang mit der Umsetzung in Abwesenheit eines Katalysators ausgeführt, können durch die Umsetzung in Gegenwart eines Inertgases unerwünschte Nebenreaktionen, insbesondere aufgrund von Oxidation oder Hydrolyse, verhindert werden.

Gemäß einer weiteren alternativen Ausführungsform der vorliegenden Erfindung kann die Umsetzung in Gegenwart eins metallhaltigen und/oder metallbasierten, sauren oder basischen Katalysators durchgeführt werden.

Gemäß dieser alternativen Ausführungsform der vorliegenden Erfindung, wonach die Umsetzung in Gegenwart eines metallhaltigen und/oder metallbasierten, sauren oder basischen Katalysators durchgeführt wird, kann der Katalysator insbesondere ausgewählt sein aus (i) basischen Katalysatoren, insbesondere Alkali- oder Erdalkalihydroxyden und Alkali- oder Erdalkalialkoholaten, wie NaOH, KOH, LiOH, Ca(OH)₂, NaOMe, KOMe und Na(OBu-tert.), (ii) sauren Katalysatoren, insbesondere Mineralsäuren, und organischen Säuren, wie Schwefelsäure, Salzsäure, Phosphorsäure, Salpetersäure, Sulfonsäuren, Methansulfonsäure, para-Toluolsulfonsäure und Carbonsäuren, (iii) Lewis-Säuren, insbesondere Lewis-Säuren auf der Basis von Titan-, Zinn-, Zink- und Aluminiumverbindungen, wie Titantetrabutylat, Zinnsäuren, Zinkacetat, Aluminiumtrichlorid und Aluminiumtriisopropyl und (iv) heterogenen Katalysatoren, insbesondere auf der Basis von mineralischen Silikaten, Germanaten, Carbonaten und Aluminiumoxiden, wie Zeolithen, Montmorilloniten, Mordeniten, Hydrotalciten und Tonerden, sowie deren Kombinationen.

Bei dieser Ausführungsform kann als Katalysator insbesondere eine Lewis-Säure auf der Basis von Titan-, Zinn-, Zink- und Aluminiumverbindungen, wie Titantetrabutylat, Zinnsäuren, Zinkacetat, Aluminiumtrichlorid und Aluminiumtriisopropyl, eingesetzt werden.

Insbesondere ist es auch bei dieser Ausführungsform bevorzugt, wenn der metallhaltige und/oder metallbasierte saure oder basische Katalysator nach der Umsetzung rezykliert wird.

Auch gemäß der besonderen Ausführungsform der vorliegenden Erfindung, wonach die Umsetzung in Gegenwart eines metallhaltigen und/oder metallbasierten, sauren oder basischen Katalysators erfolgt, können die Temperaturen in weiten Bereichen variiert werden. Insbesondere kann die Umsetzung in Gegenwart eines metallhaltigen und/oder metallbasierten, sauren oder basischen Katalysators bei Temperaturen im Bereich von 20 °C bis 160 °C, insbesondere im Bereich von 50 °C bis 150 °C, vorzugsweise im Bereich von 70 °C bis 140 °C, besonders bevorzugt im Bereich von 80 °C bis 135 °C, ganz besonders bevorzugt im Bereich von 100 °C bis 130 °C, durchgeführt werden.

Weiterhin kann auch bei dieser Ausführungsform der Katalysator (d. h. der metallhaltige und/oder metallbasierte, saure oder basische Katalysator) in weiten Mengenbereichen variiert werden: So kann der Katalysator in Mengen, bezogen auf die Gesamtmenge der Ausgangsverbindungen (I), (II) und (III), im Bereich von 0,01 Gew.-% bis 30 Gew.-%, insbesondere im Bereich von 0,05 Gew.-% bis 15 Gew.-%, vorzugsweise im Bereich von 0,1 Gew.-% bis 15 Gew.-%, bevorzugt im Bereich von 0,2 Gew.-% bis 10 Gew.-%, eingesetzt werden. Dennoch ist es anwendungsbezogen oder einzelfallbedingt möglich, von den vorgenannten Mengen abzuweichen, ohne dass der Rahmen der vorliegenden Erfindung verlassen ist.

Darüber hinaus kann gemäß dieser besonderen Ausführungsform der vorliegenden Erfindung, wonach die Umsetzung in Gegenwart eines metallhaltigen und/oder metallbasierten, sauren oder basischen Katalysators erfolgt, der Druckbereich gleichermaßen in einem weiten Regime variieren: Insbesondere kann die Umsetzung in Gegenwart eines metallhaltigen und/oder metallbasierten, sauren oder basischen Katalysators bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt werden.

Weiterhin ist es auch gemäß dieser besonderen Ausführungsform der vorliegenden Erfindung, wonach die Umsetzung in Gegenwart eines metallhaltigen und/oder metallbasierten, sauren oder basischen Katalysators durchgeführt wird, bevorzugt, wenn die Umsetzung in Gegenwart eines Inertgases, insbesondere in Gegenwart von Helium, Argon oder Stickstoff, bevorzugt in Gegenwart von Stickstoff, durchgeführt werden. Wie zuvor bereits ausgeführt werden durch die Umsetzung in Gegenwart eines Inertgases unerwünschte Nebenreaktionen, insbesondere aufgrund von Oxidation oder Hydrolyse, verhindert.

Gemäß einer besonderen Ausführungsform kann bei der mehrstufigen, insbesondere zweistufigen Vorgehensweise der erste Verfahrensschritt (a) in Abwesenheit eines Katalysators und/oder ohne jedweden Katalysator durchgeführt werden oder aber alternativ in Gegenwart eines Katalysators, insbesondere eines Enzyms und/oder eines metallhaltigen und/oder metallbasierten, sauren oder basischen Katalysators, durchgeführt werden, insbesondere wobei der Katalysator nach Umsetzung rezykliert wird.

Wie zuvor ausführt, kann auch bei der mehrstufigen, insbesondere zweistufigen Vorgehensweise gemäß einer besonderen Ausführungsform des erfindungsgemäßen Herstellungsverfahrens der erste Verfahrensschritt (a) in Abwesenheit eines Katalysators und/oder ohne jedweden Katalysator durchgeführt werden.

Sofern der erste Verfahrensschritt (a) in Abwesenheit eines Katalysators und/oder ohne jedweden Katalysator durchgeführt wird, ist es bevorzugt, wenn der erste Verfahrensschritt (a) in Abwesenheit eines Katalysator und/oder ohne jedweden Katalysator bei Temperaturen im Bereich von 20 °C bis 160 °C, insbesondere im Bereich von 50 °C bis 150 °C, vorzugsweise im Bereich von 70 °C bis 140 °C, besonders bevorzugt im Bereich von 80 °C bis 135 °C, ganz besonders bevorzugt im Bereich von 100 °C bis 130 °C, durchgeführt wird.

Im Fall der Durchführung des ersten Verfahrensschritts (a) in Abwesenheit eines Katalysators kann der angewendete Druckbereich in weiten Bereichen variieren. Insbesondere kann der erste Verfahrensschritt (a) in Abwesenheit eines Katalysator und/oder ohne jedweden Katalysator bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt werden.

Bei der Durchführung des ersten Verfahrensschritts (a) in Abwesenheit eines Katalysators ist es bevorzugt, wenn der erste Verfahrensschritt (a) in Abwesenheit eines Katalysators und/oder ohne jedweden Katalysator in Gegenwart eines Inertgases, insbesondere in Gegenwart von Helium, Argon oder Stickstoff, bevorzugt in Gegenwart von Stickstoff, durchgeführt wird.

Wie zuvor ausgeführt, ist es alternativ zu dieser besonderen Ausführungsform aber auch möglich, bei der mehrstufigen, insbesondere zweistufigen Vorgehensweise den ersten Verfahrensschritt (a) in Gegenwart eines Enzyms als Katalysator durchzuführen.

Dabei kann das Enzym insbesondere ausgewählt sein aus Synthetasen (Ligasen), Katalasen, Esterasen, Lipasen und deren Kombinationen. Erfindungsgemäß werden als Synthetasen (synonym Ligasen) insbesondere Enzyme aus der Klasse der Ligasen bezeichnet; Ligasen sind Enzyme, welche das Verknüpfen zweier oder mehrerer Moleküle durch eine kovalente Bindung katalysieren. Katalasen im Sinne der vorliegenden Erfindung sind insbesondere Enzyme, welche Wasserstoffperoxid zu Sauerstoff und Wasser umzusetzen imstande sind. Der Begriff der Esterasen bezeichnet insbesondere Enzyme, welche imstande sind, Ester hydrolytisch in Alkohol und Säure aufzuspalten (Verseifung); es handelt sich somit insbesondere um Hydrolasen, wobei fettspaltende Esterasen auch als Lipasen bezeichnet werden. Lipasen im Sinne der vorliegenden Erfindung sind insbesondere Enzyme, welche von Lipiden, wie Glyceriden, freie Fettsäuren abzuspalten imstand sind (Lipolyse).

In diesem Zusammenhang kann sich das als Katalysator eingesetzte Enzym insbesondere ableiten von *Candida antarctica, Mucor miehei (Rhizomucor miehei), Thermomyces lanuginosus, Candida rugosa, Aspergillus oryzae, Pseudomonas cepacia, Pseudomonas fluorescens, Rhizopus delemar* und *Pseudomonas* sp. sowie deren Kombinationen, vorzugsweise von *Candida antarctica, Mucor miehei (Rhizomucor miehei)* und *Thermomyces lanuginosus.*

Gemäß einer besonderen Ausführungsform kann das Enzym in immobilisierter Form, insbesondere immobilisiert auf einem Träger, vorzugsweise auf einem polymeren Träger, bevorzugt auf einem polymeren organischen Träger, besonders bevorzugt mit hydrophoben Eigenschaften, ganz besonders bevorzugt auf einem poly(meth)acrylharzbasierten Träger, eingesetzt werden.

Im Rahmen der vorliegenden Erfindung ist es bevorzugt, wenn im Fall der Verwendung eines Enzyms als Katalysator das Enzym nach der Umsetzung rezykliert wird.

Sofern bei der mehrstufigen, insbesondere zweistufigen Vorgehensweise der erste Verfahrensschritt (a) im Rahmen des erfindungsgemäßen Herstellungsverfahrens in Gegenwart eines Enzyms als Katalysator durchgeführt wird, ist es bevorzugt, wenn der erste Verfahrensschritt (a) bei Temperaturen im Bereich von 10 °C bis 80 °C, insbesondere im Bereich von 20 °C bis 80 °C, vorzugsweise im Bereich von 25 °C bis 75 °C, besonders bevorzugt im Bereich von 45 °C bis 75 °C, ganz besonders bevorzugt im Bereich von 50 °C bis 70 °C, durchgeführt wird.

Im Fall der Verwendung eines Enzyms als Katalysator kann die Menge des eingesetzten Enzyms in weiten Bereichen variieren. Insbesondere kann das Enzym in Mengen, bezogen auf die Gesamtmenge der Ausgangsverbindungen (I) und (II), im Bereich von 0,001 Gew.-% bis 20 Gew.-%, insbesondere im Bereich von 0,01 Gew.-% bis 15 Gew.-%, vorzugsweise im Bereich von 0,1 Gew.-% bis 15 Gew.-%, bevorzugt im Bereich von 0,5 Gew.-% bis 10 Gew.-%, eingesetzt werden. Dennoch kann es einzelfallbedingt oder anwendungsbezogen erforderlich sein, von den vorgenannten Mengen abzuweichen, ohne dass der Rahmen der vorliegenden Erfindung verlassen ist.

Wenn gemäß dieser besonderen Ausführungsform der vorliegenden Erfindung der erste Verfahrensschritt (a) in Gegenwart eines Enzyms als Katalysator durchgeführt wird, kann auch der angewendete Druckbereich in weiten Bereichen variieren. Typischerweise kann der erste Verfahrensschritt (a) in Gegenwart eines Enzyms als Katalysator bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 0,5 bar, durchgeführt werden.

Gemäß dieser besonderen Ausführungsform der vorliegenden Erfindung, wonach bei der mehrstufigen, insbesondere zweistufigen Vorgehensweise der erste Verfahrensschritt (a) in Gegenwart eines Enzyms als Katalysator durchgeführt wird, ist es bevorzugt, wenn der erste Verfahrensschritt (a) in Gegenwart eines Inertgases, insbesondere in Gegenwart von Helium, Argon oder Stickstoff, bevorzugt in Gegenwart von Stickstoff, durchgeführt wird. Wie zuvor bereits im Zusammenhang mit der Umsetzung in Abwesenheit eines Katalysators ausgeführt, können durch die Umsetzung in Gegenwart eines Inertgases unerwünschte Nebenreaktionen, insbesondere aufgrund von Oxidation oder Hydrolyse, verhindert werden.

Gemäß einer weiteren alternativen Ausführungsform der vorliegenden Erfindung kann bei der mehrstufigen, insbesondere zweistufigen Vorgehensweise der erste Verfahrensschritt (a) in Gegenwart eins metallhaltigen und/oder metallbasierten, sauren oder basischen Katalysators durchgeführt werden.

Gemäß dieser alternativen Ausführungsform der vorliegenden Erfindung, wonach bei der mehrstufigen, insbesondere zweistufigen Vorgehensweise der erste Verfahrensschritt (a) in Gegenwart eines metallhaltigen und/oder metallbasierten, sauren oder basischen Katalysators durchgeführt wird, kann der Katalysator insbesondere ausgewählt sein aus (i) basischen Katalysatoren, insbesondere Alkali- oder Erdalkalihydroxyden und Alkali- oder Erdalkalialkoholaten, wie NaOH, KOH, LiOH, Ca(OH)₂, NaOMe, KOMe und Na(OBu-tert.), (ii) sauren Katalysatoren, insbesondere Mineralsäuren, und organischen Säuren, wie Schwefelsäure, Salzsäure, Phosphorsäure, Salpetersäure, Sulfonsäuren, Methansulfonsäure, para-Toluolsulfonsäure und Carbonsäuren, (iii) Lewis-Säuren, insbesondere Lewis-Säuren auf der Basis von Titan-, Zinn-, Zink- und Aluminiumverbindungen, wie Titantetrabutylat, Zinnsäuren, Zinkacetat, Aluminiumtrichlorid und Aluminiumtriisopropyl und (iv) heterogenen Katalysatoren, insbesondere auf der Basis von mineralischen Silikaten, Germanaten, Carbonaten und Aluminiumoxiden, wie Zeolithen, Montmorilloniten, Mordeniten, Hydrotalciten und Tonerden, sowie deren Kombinationen.

Bei dieser Ausführungsform kann als Katalysator insbesondere eine Lewis-Säure auf der Basis von Titan-, Zinn-, Zink- und Aluminiumverbindungen, wie Titantetrabutylat, Zinnsäuren, Zinkacetat, Aluminiumtrichlorid und Aluminiumtriisopropyl, eingesetzt werden.

Insbesondere ist es auch bei dieser Ausführungsform bevorzugt, wenn der metallhaltige und/oder metallbasierte saure oder basische Katalysator nach der Umsetzung rezykliert wird.

Auch gemäß der besonderen Ausführungsform der vorliegenden Erfindung, wonach bei der mehrstufigen, insbesondere zweistufigen Vorgehensweise der erste Verfahrensschritt (a) in Gegenwart eines metallhaltigen und/oder metallbasierten, sauren oder basischen Katalysators erfolgt, können die Temperaturen in weiten Bereichen variiert werden. Insbesondere kann der erste Verfahrensschritt (a) in Gegenwart eines metallhaltigen und/oder metallbasierten, sauren oder basischen Katalysators bei Temperaturen im Bereich von 20 °C bis 160 °C, insbesondere im Bereich von 50 °C bis 150 °C, vorzugsweise im Bereich von 70 °C bis 140 °C, besonders bevorzugt im Bereich von 80 °C bis 135 °C, ganz besonders bevorzugt im Bereich von 100 °C bis 130 °C, durchgeführt werden.

Weiterhin kann auch bei dieser Ausführungsform der Katalysator (d. h. der metallhaltige und/oder metallbasierte, saure oder basische Katalysator) in weiten Mengenbereichen variiert werden: So kann der Katalysator in Mengen, bezogen auf die Gesamtmenge der Ausgangsverbindungen (I) und (II), im Bereich von 0,01 Gew.-% bis 30 Gew.-%, insbesondere im Bereich von 0,05 Gew.-% bis 15 Gew.-%, vorzugsweise im Bereich von 0,1 Gew.-% bis 15 Gew.-%, bevorzugt im Bereich von 0,2 Gew.-% bis 10 Gew.-%, eingesetzt werden. Dennoch ist es anwendungsbezogen oder einzelfallbedingt möglich, von den vorgenannten Mengen abzuweichen, ohne dass der Rahmen der vorliegenden Erfindung verlassen ist.

Darüber hinaus kann gemäß dieser besonderen Ausführungsform der vorliegenden Erfindung, wonach bei der mehrstufigen, insbesondere zweistufigen Vorgehensweise der erste Verfahrensschritt (a) in Gegenwart eines metallhaltigen und/oder metallbasierten, sauren oder basischen Katalysators erfolgt, der Druckbereich gleichermaßen in einem weiten Regime variieren: Insbesondere kann der erste Verfahrensschritt (a) in Gegenwart eines metallhaltigen und/oder metallbasierten, sauren oder basischen Katalysators bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt werden.

Weiterhin ist es auch gemäß dieser besonderen Ausführungsform der vorliegenden Erfindung, wonach bei der mehrstufigen, insbesondere zweistufigen Vorgehensweise der erste Verfahrensschritt (a) in Gegenwart eines metallhaltigen und/oder metallbasierten, sauren oder basischen Katalysators durchgeführt wird, bevorzugt, wenn die Umsetzung in Gegenwart eines Inertgases, insbesondere in Gegenwart von Helium, Argon oder Stickstoff, bevorzugt in Gegenwart von Stickstoff, durchgeführt werden. Wie zuvor bereits ausgeführt werden durch die Umsetzung in Gegenwart eines Inertgases unerwünschte Nebenreaktionen, insbesondere aufgrund von Oxidation oder Hydrolyse, verhindert.

Darüber hinaus kann gemäß einer weiteren besonderen Ausführungsform der vorliegenden Erfindung bei der mehrstufigen, insbesondere zweistufigen Vorgehensweise der zweite Verfahrensschritt (b) in Abwesenheit eines Katalysators und/oder ohne jedweden Katalysator durchgeführt werden oder aber in Gegenwart eines Katalysators, insbesondere eines Enzyms und/oder eines metallhaltigen und/oder metallbasierten, sauren oder basischen Katalysators, durchgeführt werden (insbesondere wobei der Katalysator nach Umsetzung rezykliert werden kann).

Wie zuvor ausführt, kann gemäß einer besonderen Ausführungsform des erfindungsgemäßen Herstellungsverfahrens auch der zweite Verfahrensschritt (b) bei der mehrstufigen, insbesondere zweistufigen Vorgehensweise in Abwesenheit eines Katalysators und/oder ohne jedweden Katalysator durchgeführt werden.

Sofern bei der mehrstufigen, insbesondere zweistufigen Vorgehensweise der zweite Verfahrensschritt (b) in Abwesenheit eines Katalysators und/oder ohne jedweden Katalysator durchgeführt wird, ist es bevorzugt, wenn der zweite Verfahrensschritt (b) in Abwesenheit eines Katalysator und/oder ohne jedweden Katalysator bei Temperaturen im Bereich von 20 °C bis 160 °C, insbesondere im Bereich von 50 °C bis 150 °C, vorzugsweise im Bereich von 70 °C bis 140 °C, besonders bevorzugt im Bereich von 80 °C bis 135 °C, ganz besonders bevorzugt im Bereich von 100 °C bis 130 °C, durchgeführt wird.

Im Fall der Durchführung des zweiten Verfahrensschritts (b) in Abwesenheit eines Katalysators kann der angewendete Druckbereich in weiten Bereichen variieren. Insbesondere kann der zweite Verfahrensschritt (b) in Abwesenheit eines Katalysator und/oder ohne jedweden Katalysator bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt werden.

Bei der Durchführung des zweiten Verfahrensschritts (b) in Abwesenheit eines Katalysators, ist es bevorzugt, wenn der zweite Verfahrensschritt (b) in Gegenwart eines Inertgases, insbesondere in Gegenwart von Helium, Argon oder Stickstoff, bevorzugt in Gegenwart von Stickstoff, durchgeführt wird. Insbesondere können hierdurch unerwünschte Nebenreaktionen, insbesondere aufgrund von Oxidation oder Hydrolyse, verhindert werden.

Alternativ zu dieser besonderen Ausführungsform ist es aber auch möglich, bei der mehrstufigen, insbesondere zweistufigen Vorgehensweise den zweiten Verfahrensschritt (b) in Gegenwart eines Enzyms als Katalysator durchzuführen.

Dabei kann das Enzym insbesondere ausgewählt sein aus Synthetasen (Ligasen), Katalasen, Esterasen, Lipasen und deren Kombinationen. Erfindungsgemäß werden als Synthetasen (synonym Ligasen) insbesondere Enzyme aus der Klasse der Ligasen bezeichnet; Ligasen sind Enzyme, welche das Verknüpfen zweier oder mehrerer Moleküle durch eine kovalente Bindung katalysieren. Katalasen im Sinne der vorliegenden Erfindung sind insbesondere Enzyme, welche Wasserstoffperoxid zu Sauerstoff und Wasser umzusetzen imstande sind. Der Begriff der Esterasen bezeichnet insbesondere Enzyme, welche imstande sind, Ester hydrolytisch in Alkohol und Säure aufzuspalten (Verseifung); es handelt sich somit insbesondere um Hydrolasen, wobei fettspaltende Esterasen auch als Lipasen bezeichnet werden. Lipasen im Sinne der vorliegenden Erfindung sind insbesondere Enzyme, welche von Lipiden, wie Glyceriden, freie Fettsäuren abzuspalten imstand sind (Lipolyse).

In diesem Zusammenhang kann sich das als Katalysator eingesetzte Enzym insbesondere ableiten von *Candida antarctica, Mucor miehei (Rhizomucor miehei), Thermomyces lanuginosus, Candida rugosa, Aspergillus oryzae, Pseudomonas cepacia, Pseudomonas fluorescens, Rhizopus delemar* und *Pseudomonas* sp. sowie deren Kombinationen, vorzugsweise von *Candida antarctica, Mucor miehei* (*Rhizomucor miehei*) und *Thermomyces lanuginosus.*

Gemäß einer besonderen Ausführungsform kann das Enzym in immobilisierter Form, insbesondere immobilisiert auf einem Träger, vorzugsweise auf einem polymeren Träger, bevorzugt auf einem polymeren organischen Träger, besonders bevorzugt mit hydrophoben Eigenschaften, ganz besonders bevorzugt auf einem poly(meth)acrylharzbasierten Träger, eingesetzt werden.

Im Rahmen der vorliegenden Erfindung ist es bevorzugt, wenn im Fall der Verwendung eines Enzyms als Katalysator das Enzym nach der Umsetzung rezykliert wird.

Sofern bei der mehrstufigen, insbesondere zweistufigen Vorgehensweise der zweite Verfahrensschritt (b) im Rahmen des erfindungsgemäßen Herstellungsverfahrens in Gegenwart eines Enzyms als Katalysator durchgeführt wird, ist es bevorzugt, wenn der zweite Verfahrensschritt (b) in Gegenwart eines Enzyms als Katalysator bei Temperaturen im Bereich von 10 °C bis 80 °C, insbesondere im Bereich von 20 °C bis 80 °C, vorzugsweise im Bereich von 25 °C bis 75 °C, besonders bevorzugt im Bereich von 45 °C bis 75 °C, ganz besonders bevorzugt im Bereich von 50 °C bis 70 °C, durchgeführt wird.

Im Fall der Verwendung eines Enzyms als Katalysator kann die Menge des eingesetzten Enzyms in weiten Bereichen variieren. Insbesondere kann das Enzym in Mengen, bezogen auf die Gesamtmenge der Ausgangsverbindungen (IV') und (III), im Bereich von 0,001 Gew.-% bis 20 Gew.-%, insbesondere im Bereich von 0,01 Gew.-% bis 15 Gew.-%, vorzugsweise im Bereich von 0,1 Gew.-% bis 15 Gew.-%, bevorzugt im Bereich von 0,5 Gew.-% bis 10 Gew.-%, eingesetzt werden. Dennoch kann es einzelfallbedingt oder anwendungsbezogen erforderlich sein, von den vorgenannten Mengen abzuweichen, ohne dass der Rahmen der vorliegenden Erfindung verlassen ist.

Wenn gemäß einer besonderen Ausführungsform der vorliegenden Erfindung bei der mehrstufigen, insbesondere zweistufigen Vorgehensweise der zweite Verfahrensschritt (b) in Gegenwart eines Enzyms als Katalysator durchgeführt wird, kann auch der angewendete Druckbereich in weiten Bereichen variieren. Typischerweise kann der zweite Verfahrensschritt (b) in Gegenwart eines Enzyms als Katalysator bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 0,5 bar, durchgeführt werden.

Gemäß der besonderen Ausführungsform der vorliegenden Erfindung, wonach der zweite Verfahrensschritt (b) in Gegenwart eines Enzyms als Katalysator durchgeführt wird, ist es bevorzugt, wenn der zweite Verfahrensschritt (b) in Gegenwart eines Enzyms in Gegenwart eines Inertgases, insbesondere in Gegenwart von Helium, Argon oder Stickstoff, bevorzugt in Gegenwart von Stickstoff, durchgeführt wird. Wie zuvor bereits im Zusammenhang mit der Umsetzung in Abwesenheit eines Katalysators ausgeführt, können durch die Umsetzung in Gegenwart eines Inertgases unerwünschte Nebenreaktionen, insbesondere aufgrund von Oxidation oder Hydrolyse, verhindert werden.

Gemäß einer weiteren alternativen Ausführungsform der vorliegenden Erfindung kann bei der mehrstufigen, insbesondere zweistufigen Vorgehensweise der zweite Verfahrensschritt (b) in Gegenwart eins metallhaltigen und/oder metallbasierten, sauren oder basischen Katalysators durchgeführt werden.

Gemäß dieser alternativen Ausführungsform der vorliegenden Erfindung, wonach die Umsetzung in Gegenwart eines metallhaltigen und/oder metallbasierten, sauren oder basischen Katalysators durchgeführt wird, kann der Katalysator insbesondere ausgewählt sein aus (i) basischen Katalysatoren, insbesondere Alkali- oder Erdalkalihydroxyden und Alkali- oder Erdalkalialkoholaten, wie NaOH, KOH, LiOH, Ca(OH)₂, NaOMe, KOMe und Na(OBu-tert.), (ii) sauren Katalysatoren, insbesondere Mineralsäuren, und organischen Säuren, wie Schwefelsäure, Salzsäure, Phosphorsäure, Salpetersäure, Sulfonsäuren, Methansulfonsäure, para-Toluolsulfonsäure und Carbonsäuren, (iii) Lewis-Säuren, insbesondere Lewis-Säuren auf der Basis von Titan-, Zinn-, Zink- und Aluminiumverbindungen, wie Titantetrabutylat, Zinnsäuren, Zinkacetat, Aluminiumtrichlorid und Aluminiumtriisopropyl und (iv) heterogenen Katalysatoren, insbesondere auf der Basis von mineralischen Silikaten, Germanaten, Carbonaten und Aluminiumoxiden, wie Zeolithen, Montmorilloniten, Mordeniten, Hydrotalciten und Tonerden, sowie deren Kombinationen.

Bei dieser Ausführungsform kann als Katalysator insbesondere eine Lewis-Säure auf der Basis von Titan-, Zinn-, Zink- und Aluminiumverbindungen, wie Titantetrabutylat, Zinnsäuren, Zinkacetat, Aluminiumtrichlorid und Aluminiumtriisopropyl, eingesetzt werden.

Insbesondere ist es auch bei dieser Ausführungsform bevorzugt, wenn der metallhaltige und/oder metallbasierte saure oder basische Katalysator nach der Umsetzung rezykliert wird.

Auch gemäß der besonderen Ausführungsform der vorliegenden Erfindung, wonach bei der mehrstufigen, insbesondere zweistufigen Vorgehensweise der zweite Verfahrensschritt (b) in Gegenwart eines metallhaltigen und/oder metallbasierten, sauren oder basischen Katalysators erfolgt, können die Temperaturen in weiten Bereichen variiert werden. Insbesondere kann der zweite Verfahrensschritt (b) in Gegenwart eines metallhaltigen und/oder metallbasierten, sauren oder basischen Katalysators bei Temperaturen im Bereich von 20 °C bis 160 °C, insbesondere im Bereich von 50 °C bis 150 °C, vorzugsweise im Bereich von 70 °C bis 140 °C, besonders bevorzugt im Bereich von 80 °C bis 135 °C, ganz besonders bevorzugt im Bereich von 100 °C bis 130 °C, durchgeführt werden.

Weiterhin kann auch bei dieser Ausführungsform der Katalysator (d. h. der metallhaltige und/oder metallbasierte, saure oder basische Katalysator) in weiten Mengenbereichen variiert werden: So kann der Katalysator in Mengen, bezogen auf die Gesamtmenge der Ausgangsverbindungen (IV') und (III), im Bereich von 0,01 Gew.-% bis 30 Gew.-%, insbesondere im Bereich von 0,05 Gew.-% bis 15 Gew.-%, vorzugsweise im Bereich von 0,1 Gew.-% bis 15 Gew.-%, bevorzugt im Bereich von 0,2 Gew.-% bis 10 Gew.-%, eingesetzt werden. Dennoch ist es anwendungsbezogen oder einzelfallbedingt möglich, von den vorgenannten Mengen abzuweichen, ohne dass der Rahmen der vorliegenden Erfindung verlassen ist.

Darüber hinaus kann gemäß dieser besonderen Ausführungsform der vorliegenden Erfindung, wonach bei der mehrstufigen, insbesondere zweistufigen Vorgehensweise der zweite Verfahrensschritt (b) in Gegenwart eines metallhaltigen und/oder metallbasierten, sauren oder basischen Katalysators erfolgt, der Druckbereich gleichermaßen in einem weiten Regime variieren: Insbesondere kann der zweite Verfahrensschritt (b) in Gegenwart eines metallhaltigen und/oder metallbasierten, sauren oder basischen Katalysators bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt werden.

Weiterhin ist es auch gemäß dieser besonderen Ausführungsform der vorliegenden Erfindung, wonach bei der mehrstufigen, insbesondere zweistufigen Vorgehensweise der zweite Verfahrensschritt (b) in Gegenwart eines metallhaltigen und/oder metallbasierten, sauren oder basischen Katalysators durchgeführt wird, bevorzugt, wenn der zweite Verfahrensschritt (b) in Gegenwart eines Inertgases, insbesondere in Gegenwart von Helium, Argon oder Stickstoff, bevorzugt in Gegenwart von Stickstoff, durchgeführt wird. Wie zuvor bereits ausgeführt werden durch die Umsetzung in Gegenwart eines Inertgases unerwünschte Nebenreaktionen, insbesondere aufgrund von Oxidation oder Hydrolyse, verhindert.

Was die Mengen an Edukten bzw. Ausgangsverbindungen insgesamt anbelangt, so können diese in weiten Bereichen variiert werden.

Unter Berücksichtigung von Verfahrensökonomie und Optimierung des Verfahrensablaufs, insbesondere im Hinblick auf die Minimierung von Nebenprodukten, ist es vorteilhaft, wenn im ersten Verfahrensschritt (a) die Ausgangsverbindungen (I) und (II) in einem (I) / (II)-Molverhältnis von etwa 1 : 1 eingesetzt werden.

Gleichermaßen unter Berücksichtigung von Verfahrensökonomie und Optimierung des Verfahrensablaufs, insbesondere im Hinblick auf die Minimierung von Nebenprodukten, ist es vorteilhaft, wenn im zweiten Verfahrensschritt (b) die Ausgangsverbindung (III) und das Reaktionszwischenprodukt (IV') in einem (III) / (IV')-Molverhältnis im Bereich von (1/n) : 1 bis 1 : 1, eingesetzt werden, wobei n die Anzahl der Hydroxylgruppen des Polyglycerins (III) bezeichnet und vorzugsweise im Bereich von 4 bis 9 liegt.

Im Rahmen des erfindungsgemäßen Verfahrens wird bei Verwendung der Polycarbonsäure (II) in Form der freien Säure bei der Umsetzung gleichzeitig Wasser gebildet. Erfindungsgemäß wird das Wasser der Umsetzung kontinuierlich entzogen, insbesondere mittels vorzugsweise kontinuierlicher, insbesondere destillativer oder adsorptiver Entfernung.

Im Rahmen des erfindungsgemäßen Verfahrens werden bei Verwendung der Polycarbonsäure (II) in Form des Anhydrids die entsprechende freie Polycarbonsäure (II) und Wasser gebildet. Erfindungsgemäß wird die entstehende freie Polycarbonsäure (II) weiter umgesetzt oder nach erfolgter Reaktion entfernt und gegebenenfalls rezykliert, insbesondere in Abhängigkeit von den Mengen und/oder Mengenverhältnissen der eingesetzten Ausgangsverbindungen (I), (II) und (III), und das Wasser wird der Umsetzung kontinuierlich entzogen, insbesondere mittels vorzugsweise kontinuierlicher, insbesondere destillativer oder adsorptiver Entfernung.

Bei der Verwendung von inneren bzw. cyclischen Anhydriden (wie beispielsweise Bernsteinsäureanhydrid oder Maleinsäureanhydrid) wird jedoch der Ring geöffnet und kein Abspaltprodukt gebildet, so dass das Reaktionsprodukt eine endständige freie Säure aufweist. Dieser Verlauf ist im Folgenden am Beispiel der Reaktion von Maleinsäureanhydrid mit 3-Hydroxybuttersäureethylester als Veresterungsalkohol dargestellt:

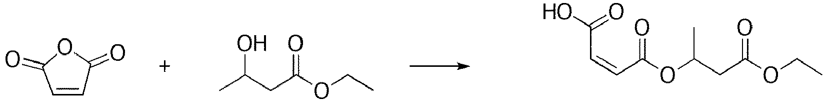

Alternativ kann die Polycarbonsäure (II) aber auch in Form eines Esters eingesetzt werden. Im Rahmen des erfindungsgemäßen Verfahrens werden bei Verwendung der Polycarbonsäure (II) in Form des Esters der entsprechende Esteralkohol und Wasser gebildet. Erfindungsgemäß werden der entstehende Esteralkohol und Wasser der Umsetzung kontinuierlich entzogen, insbesondere mittels vorzugsweise kontinuierlicher, insbesondere destillativer oder adsorptiver Entfernung.

Durch die kontinuierliche Entfernung der Nebenprodukte (d. h. Wasser und/oder Esteralkohol) wird das chemische Gleichgewicht verschoben, wodurch die Ausbeute bzw. Produktbildung erhöht und die Nebenproduktbildung minimiert wird.

Im Rahmen des erfindungsgemäßen Herstellungsverfahren kann die Zusammensetzung des Reaktionsprodukts, insbesondere das Vorhandensein verschiedener Polyglycerinester von mit Oxobutanol veresterten Polycarbonsäuren, und deren jeweiliger Anteil im Fall eines Gemischs mittels der Umsetzungsbedingungen kontrolliert und/oder gesteuert werden kann, insbesondere durch Auswahl der Umsetzungstemperatur (Reaktionstemperatur) und/oder Auswahl des Umsetzungsdrucks (Reaktionsdrucks) und/oder Abwesenheit oder Vorsehen eines Katalysators und dessen Auswahl in Bezug auf Art und/oder Menge und/oder Auswahl der Mengen der Ausgangsverbindungen (Edukte) und/oder Vorsehen der Entfernung der gegebenenfalls gebildeten Nebenprodukte.

Somit ist es möglich, die Zusammensetzung des Produkts bzw. Produktgemischs je nach Anwendung maßzuschneidern; insbesondere kann beispielsweise die Dichte an Ketokörpern (d. h. Oxobutanolen bzw. 3-Hydroxybutanoaten) pro Molekül zielgerichtet eingestellt werden.

Im Anschluss an die Umsetzung kann das erhaltene Reaktionsprodukt weiteren üblichen bzw. an sich bekannten Aufreinigungs- bzw. Aufarbeitungsschritten unterzogen werden.

In diesem Zusammenhang kann das erhaltene Reaktionsprodukt nach erfolgter Umsetzung beispielsweise fraktioniert werden, insbesondere destillativ fraktioniert werden.

Auch können nichtumgesetzte Ausgangsverbindungen (I) und/oder (II) und/oder (III) aus dem Reaktionsprodukt abgetrennt und anschließend gegebenenfalls rezykliert werden.

Gemäß einer besonderen Ausführungsform des erfindungsgemäßen Herstellungsverfahrens kann insbesondere derart vorgegangen werden, dass im Reaktionsprodukt nach erfolgter Umsetzung noch vorhandene Hydroxylgruppen und/oder Carboxylgruppen zumindest teilweise, vorzugsweise vollständig, funktionalisiert, insbesondere verestert werden.

Mit anderen Worten kann sich der Umsetzung eine teilweise, insbesondere vollständige Funktionalisierung, insbesondere Veresterung, noch vorhandener Hydroxylgruppen und/oder Carboxylgruppen anschließen.

Bei dieser besonderen Ausführungsform des erfindungsgemäßen Verfahrens kann die Funktionalisierung, insbesondere die Veresterung noch vorhandener Hydroxylgruppen und/oder Carboxylgruppen, durch Reaktion mit einem Carbonsäureanhydrid von beispielsweise C₂-C₃₀-Carbonsäuren oder mit C₂-C₃₀-Fettsäuren oder mit C₂-C₃₀-Fettalkoholen erfolgen. Dabei kann es sich um lineare oder verzweigte, gesättigte oder ein- oder mehrfach ungesättigte C₂-C₃₀-Carbonsäureanhydride oder C₂-C₃₀-Fettsäuren oder C₂-C₃₀-Fettalkohole handeln. In diesem Zusammenhang können noch vorhandene Hydroxylgruppen insbesondere mit Carbonsäureanhydriden oder Fettsäuren umgesetzt werden und noch vorhandene Carboxylgruppen können insbesondere mit Fettalkoholen umgesetzt werden.

Wenn das erfindungsgemäße Verfahren zweistufig durchgeführt wird, können als Reaktionszwischenprodukt (IV') des Verfahrensschritts (a) ein oder mehrere Polycarbonsäureester von Oxobutanol, insbesondere ein oder mehrere Polycarbonsäureester von 3-Hydroxybutanoat, bevorzugt ein oder mehrere Polycarbonsäureester von 4-Oxo-2-butanol, vorzugsweise ein oder mehrere Polycarbonsäureester von 4-Oxo-4-(C₁-C₅-alkoxy)-2-butanol oder von 4-Oxo-4-(hydroxy-C₃-C₅-alkoxy)-2-butanol, erhalten werden und/oder erhältlich sein.

Insbesondere können gemäß der Ausführungsform des erfindungsgemäßen Verfahrens, wonach das Verfahren zweistufig durchgeführt wird, als Reaktionszwischenprodukt (IV') des Verfahrensschritts (a) ein oder mehrere Polycarbonsäureester von Oxobutanol, insbesondere ein oder mehrere Polycarbonsäureester von 3-Hydroxybutanoat, bevorzugt ein oder mehrere Polycarbonsäureester von 4-Oxo-2-butanol, vorzugsweise ein oder mehrere Polycarbonsäureester von 4-Oxo-4-(C₁-C₅-alkoxy)-2-butanol oder von 4-Oxo-4-(hydroxy-C₃-C₅-alkoxy)-2-butanol, der allgemeinen Formel (IVa')

R¹-O-C(O)-CH₂-CH(CH₃)-O-(O)C-X-COOH (IVa')

erhalten werden und/oder erhältlich sein, wobei in der allgemeinen Formel (IVa')
- R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt;
- X einen 1 bis 10, vorzugsweise 2 bis 6 Kohlenstoffatome enthaltenden und gegebenenfalls ein- oder mehrfach substituierten, insbesondere mit einem oder mehreren Hydroxyl-Resten und/oder Resten -O - C(O) - CH₂ - CH(OH) - CH₃ und/oder Carboxyl-Resten und/oder Resten - C(O) - O - CH(CH₃) - CH₂ -C(O)OR¹ mit R¹ wie zuvor definiert substituierten, gesättigten oder ungesättigten organischen Rest darstellt.

Insbesondere ist es dabei bevorzugt, wenn der Rest - COOH endständig ist und/oder ein primärer Rest ist.

Darüber hinaus kann es bevorzugt sein, wenn das Verfahren zweistufig durchgeführt wird, dass als Reaktionszwischenprodukt (IV') des Verfahrensschritts (a) ein oder mehrere Polycarbonsäureester von Oxobutanol, insbesondere ein oder mehrere Polycarbonsäureester von 3-Hydroxybutanoat, bevorzugt ein oder mehrere Polycarbonsäureester von 4-Oxo-2-butanol, vorzugsweise ein oder mehrere Polycarbonsäureester von 4-Oxo-4-(C₁-C₅-alkoxy)-2-butanol oder von 4-Oxo-4-(hydroxy-C₃-C₅-alkoxy)-2-butanol, der allgemeinen Formel (IVa')

R¹-O-C(O)-CH₂-CH(CH₃)-O-(O)C-X-COOH (IVa')

erhalten werden und/oder erhältlich sind, wobei in der allgemeinen Formel (IVa')
- R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt;
- X einen 2 bis 6 Kohlenstoffatome enthaltenden und gegebenenfalls ein- oder mehrfach substituierten, insbesondere mit einem oder mehreren Hydroxyl-Resten und/oder Resten - O - C(O) - CH₂ - CH(OH) - CH₃ und/oder Carboxyl-Resten und/oder Resten - C(O) - O - CH(CH₃) - CH₂ - C(O)OR¹ mit R¹ wie zuvor definiert substituierten, gesättigten oder ungesättigten organischen Rest darstellt;
insbesondere wobei der Rest - COOH endständig ist und/oder ein primärer Rest ist.

Weiterhin kann es auch bevorzugt sein, wenn das Verfahren zweistufig durchgeführt wird, dass als Reaktionszwischenprodukt (IV') des Verfahrensschritts (a) ein oder mehrere Polycarbonsäureester von Oxobutanol, insbesondere ein oder mehrere Polycarbonsäureester von 3-Hydroxybutanoat, bevorzugt ein oder mehrere Polycarbonsäureester von 4-Oxo-2-butanol, vorzugsweise ein oder mehrere Polycarbonsäureester von 4-Oxo-4-(C₁-C₅-alkoxy)-2-butanol oder von 4-Oxo-4-(hydroxy-C₃-C₅-alkoxy)-2-butanol, der allgemeinen Formel (IVa')

R¹-O-C(O)-CH₂-CH(CH₃)-O-(O)C-X-COOH (IVa')

erhalten werden und/oder erhältlich sind, wobei in der allgemeinen Formel (IVa')
- R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt;
- X einen 2 bis 6 Kohlenstoffatome enthaltenden und gegebenenfalls ein- oder mehrfach substituierten, insbesondere mit einem oder mehreren Hydroxyl-Resten und/ Carboxyl-Resten substituierten, gesättigten oder ungesättigten organischen Rest darstellt;
insbesondere wobei der Rest - COOH endständig ist und/oder ein primärer Rest ist.

Gemäß der Ausführungsform des erfindungsgemäßen Verfahrens, wonach das Verfahren zweistufig durchgeführt wird, können als Reaktionszwischenprodukt (IV') des Verfahrensschritts (a) ein oder mehrere Polycarbonsäureester von Oxobutanol, insbesondere ein oder mehrere Polycarbonsäureester von 3-Hydroxybutanoat, bevorzugt ein oder mehrere Polycarbonsäureester von 4-Oxo-2-butanol, vorzugsweise ein oder mehrere Polycarbonsäureester von 4-Oxo-4-(C₁-C₅-alkoxy)-2-butanol oder von 4-Oxo-4-(hydroxy-C₃-C₅-alkoxy)-2-butanol, der allgemeinen Formel (IVb')

CH₃-CH(OR²)-CH₂-C(O)OR¹ (IVb')

erhalten werden und/oder erhältlich sein, wobei in der allgemeinen Formel (Illb')
- der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und
- der Rest R² abgeleitet ist von einer Polycarbonsäure, ausgewählt aus der Gruppe von Bernsteinsäure, Weinsäure, Citronensäure, Äpfelsäure, Adipinsäure, Fumarsäure und Maleinsäure sowie deren Kombinationen oder Mischungen, insbesondere ausgewählt aus der Gruppe von Bernsteinsäure, Weinsäure, Citronensäure, Äpfelsäure, Adipinsäure und Fumarsäure sowie deren Kombinationen oder Mischungen bevorzugt ausgewählt aus der Gruppe von Bernsteinsäure und Adipinsäure und deren Anhydriden sowie deren Kombinationen oder Mischungen;
insbesondere wobei im Fall der Citronensäure eine weitere Carboxylgruppe mit einem Rest - CH(CH₃) - CH₂ - C(O)OR¹ mit R¹ wie zuvor definiert verestert ist.

In diesem Zusammenhang bedeutet "abgeleitet von", dass der Rest R² aus den genannten Carbonsäuren gebildet wird; insbesondere wird durch Veresterung der Wasserstoff des Carboxyls verestert; d. h. also, es liegt jeweils der Carboxylatrest der entsprechenden Säure als Rest R² vor (d. h. also der Rest R² ist ein Succinat-Rest im Fall der Bernsteinsäure, ein Tartrat-Rest im Fall der Weinsäure, ein Citrat-Rest im Fall der Citronensäure, ein Malat-Rest im Fall der Äpfelsäure, ein Adipat-Rest im Fall der Adipinsäure, ein Fumarat-Rest im Fall der Fumarsäure und ein Maleat-Rest im Fall der Maleinsäure).

Weiterhin können gemäß der Ausführungsform des erfindungsgemäßen Verfahrens, wonach das Verfahren zweistufig durchgeführt wird, als Reaktionszwischenprodukt (IV') des Verfahrensschritts (a) ein oder mehrere Polycarbonsäureester von Oxobutanol, insbesondere ein oder mehrere Polycarbonsäureester von 3-Hydroxybutanoat, bevorzugt ein oder mehrere Polycarbonsäureester von 4-Oxo-2-butanol, vorzugsweise ein oder mehrere Polycarbonsäureester von 4-Oxo-4-(C₁-C₅-alkoxy)-2-butanol oder von 4-Oxo-4-(hydroxy-C₃-C₅-alkoxy)-2-butanol, der allgemeinen Formel (IVb')

CH₃-CH(OR²)-CH₂-C(O)OR¹ (IVb')

erhalten werden und/oder erhältlich sein, wobei in der allgemeinen Formel (IVb')
- der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und
- der Rest R² einen oder mehrere der folgenden Reste darstellt 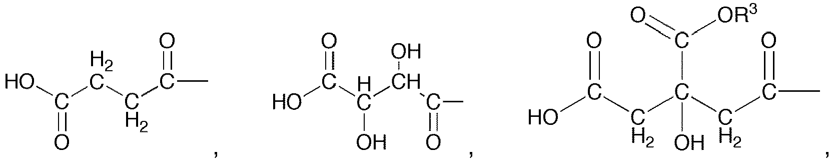 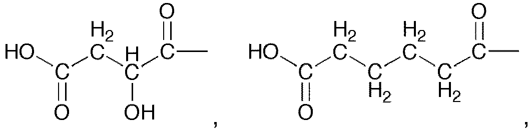 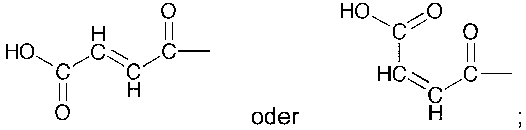 wobei in den vorstehenden Resten der Rest R³ Wasserstoff oder einen Rest - CH(CH₃) - CH₂ - C(O)OR¹ mit R¹ wie zuvor definiert darstellt.

Darüber hinaus kann gemäß der Ausführungsform des erfindungsgemäßen Verfahrens, wonach das Verfahren zweistufig durchgeführt wird, als Reaktionszwischenprodukt (IV') des Verfahrensschritts (a) ein Gemisch von mindestens zwei voneinander verschiedenen Polycarbonsäureestern von Oxobutanol, insbesondere Polycarbonsäureestern von 3-Hydroxybutanoat, bevorzugt Polycarbonsäureestern von 4-Oxo-2-butanol, vorzugsweise Polycarbonsäureestern von 4-Oxo-4-(C₁-C₅-alkoxy)-2-butanol oder von 4-Oxo-4-(hydroxy-C₃-C₅-alkoxy)-2-butanol, insbesondere wie zuvor definiert, erhalten werden und/oder erhältlich sein.

Im Rahmen des erfindungsgemäßen Verfahrens können als Reaktionsprodukt (IV) ein oder mehrere Polyglycerinester von mit Oxobutanol veresterten Polycarbonsäuren, insbesondere ein oder mehrere Polyglycerinester von mit 4-Oxo-2-butanol veresterten Polycarbonsäuren, vorzugsweise ein oder mehrere ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, der allgemeinen Formel (IVa)

R⁴O-CH₂-CH(OR⁴)-CH₂-[O-CH₂-CH(OR⁴)-CH₂]ₚ-OR⁴ (IVa)

erhalten werden und/oder erhältlich sein,
wobei in der allgemeinen Formel (IVa) der Rest R⁴, unabhängig voneinander, darstellt:
   - Wasserstoff;
   - einen Rest R¹- O - C(O) - CH₂ - CH(CH₃) - O - (O)C - X - C(O) -, wobei der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und wobei X einen 1 bis 10, vorzugsweise 2 bis 6 Kohlenstoffatome enthaltenden und gegebenenfalls ein- oder mehrfach substituierten, insbesondere mit einem oder mehreren Hydroxyl-Resten und/oder Resten - O - C(O) - CH₂ - CH(OH) - CH₃ und/oder Carboxyl-Resten und/oder Resten - C(O) - O - CH(CH₃) - CH₂ - C(O)OR¹ mit R¹ wie zuvor definiert substituierten, gesättigten oder ungesättigten organischen Rest darstellt;
   - einen Rest H₃C - CH(OH) - CH₂ - C(O) -;
   - einen Rest HOOC - X - C(O) -, wobei X einen 1 bis 10, vorzugsweise 2 bis 6 Kohlenstoffatome enthaltenden und gegebenenfalls ein- oder mehrfach substituierten, insbesondere mit einem oder mehreren Hydroxyl-Resten und/oder Resten - O - C(O) - CH₂ - CH(OH) - CH₃ und/oder Carboxyl-Resten und/oder Resten - C(O) - O - CH(CH₃) - CH₂ - C(O)OR¹ mit R¹ wie zuvor definiert substituierten, gesättigten oder ungesättigten organischen Rest darstellt;
jedoch mit der Maßgabe, dass mindestens ein Rest R⁴, insbesondere mindestens zwei Reste R⁴, einen Rest R¹ - O - C(O) - CH₂ - CH(CH₃) - O - (O)C - X-C(O) -, wie zuvor definiert, darstellt; und
wobei in der allgemeinen Formel (IVa) die Variable p eine ganze Zahl von 1 bis 6, insbesondere von 1 bis 4, vorzugsweise 1 oder 2, besonders bevorzugt 1, darstellt.

Insbesondere können bei dem erfindungsgemäßen Herstellungsverfahren als Reaktionsprodukt (IV) ein oder mehrere Polyglycerinester von mit Oxobutanol veresterten Polycarbonsäuren, insbesondere ein oder mehrere Polyglycerinester von mit 4-Oxo-2-butanol veresterten Polycarbonsäuren, vorzugsweise ein oder mehrere ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, der allgemeinen Formel (IVa)

R⁴O-CH₂-CH(OR⁴)-CH₂-[O-CH₂-CH(OR⁴)-CH₂]ₚ-OR⁴ (IVa)

erhalten werden und/oder erhältlich sein,
wobei in der allgemeinen Formel (IVa) der Rest R⁴, unabhängig voneinander, darstellt:
   - Wasserstoff;
   - einen Rest R¹ - O - C(O) - CH₂ - CH(CH₃) - O - (O)C - X - C(O) -, wobei der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und wobei X einen 1 bis 10, vorzugsweise 2 bis 6 Kohlenstoffatome enthaltenden und gegebenenfalls ein- oder mehrfach substituierten, insbesondere mit einem oder mehreren Hydroxyl-Resten und/oder Resten - O - C(O) - CH₂ - CH(OH) - CH₃ und/oder Carboxyl-Resten und/oder Resten - C(O) - O - CH(CH₃) - CH₂ - C(O)OR¹ mit R¹ wie zuvor definiert substituierten, gesättigten oder ungesättigten organischen Rest darstellt;
jedoch mit der Maßgabe, dass mindestens ein Rest R⁴, insbesondere mindestens zwei Reste R⁴, einen Rest R¹ - O - C(O) - CH₂ - CH(CH₃) - O - (O)C - X - C(O) -, wie zuvor definiert, darstellt; und
wobei in der allgemeinen Formel (IVa) die Variable p eine ganze Zahl von 1 bis 6, insbesondere von 1 bis 4, vorzugsweise 1 oder 2, besonders bevorzugt 1, darstellt.

Gemäß einer besonderen Ausführungsform des erfindungsgemäßen Verfahrens kann es bevorzugt sein, wenn als Reaktionsprodukt (IV) ein oder mehrere Polyglycerinester von mit Oxobutanol veresterten Polycarbonsäuren, insbesondere ein oder mehrere Polyglycerinester von mit 4-Oxo-2-butanol veresterten Polycarbonsäuren, vorzugsweise ein oder mehrere ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, der allgemeinen Formel (IVa)

R⁴O-CH₂-CH(OR⁴)-CH₂-[O-CH₂-CH(OR⁴)-CH₂]ₚ-OR⁴ (IVa)

erhalten werden und/oder erhältlich sind,
wobei in der allgemeinen Formel (IVa) der Rest R⁴, unabhängig voneinander, darstellt:
   - Wasserstoff;
   - einen Rest R¹ - O - C(O) - CH₂ - CH(CH₃) - O - (O)C - X - C(O) -, wobei der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und wobei X einen 1 bis 10, vorzugsweise 2 bis 6 Kohlenstoffatome enthaltenden und gegebenenfalls ein- oder mehrfach substituierten, insbesondere mit einem oder mehreren Hydroxyl-Resten und/oder Carboxyl-Resten substituierten, gesättigten oder ungesättigten organischen Rest darstellt;
jedoch mit der Maßgabe, dass mindestens ein Rest R⁴, insbesondere mindestens zwei Reste R⁴, einen Rest R¹ - O - C(O) - CH₂ - CH(CH₃) - O - (O)C - X - C(O) -, wie zuvor definiert, darstellt; und
wobei in der allgemeinen Formel (IVa) die Variable p eine ganze Zahl von 1 bis 6, insbesondere von 1 bis 4, vorzugsweise 1 oder 2, besonders bevorzugt 1, darstellt.

Gemäß einer weiteren besonderen Ausführungsform des erfindungsgemäßen Verfahren können als Reaktionsprodukt (IV) ein oder mehrere Polyglycerinester von mit Oxobutanol veresterten Polycarbonsäuren, insbesondere ein oder mehrere Polyglycerinester von mit 4-Oxo-2-butanol veresterten Polycarbonsäuren, vorzugsweise ein oder mehrere ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, der allgemeinen Formel (IVb)

R⁵O-CH₂-CH(OR⁵)-CH₂-[O-CH₂-CH(OR⁵)-CH₂]ₚ-OR⁵ (IVb)

erhalten werden und/oder erhältlich sein,
wobei in der allgemeinen Formel (IVb) der Rest R⁵, unabhängig voneinander, darstellt:
   - Wasserstoff;
   - einen Rest R¹ - O - C(O) - CH₂ - CH(CH₃) - O - Y -, wobei der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und wobei Y abgeleitet ist von einer Polycarbonsäure, ausgewählt aus der Gruppe von Bernsteinsäure, Weinsäure, Citronensäure, Äpfelsäure, Adipinsäure, Fumarsäure und Maleinsäure sowie deren Kombinationen oder Mischungen, insbesondere ausgewählt aus der Gruppe von Bernsteinsäure, Weinsäure, Citronensäure, Äpfelsäure, Adipinsäure und Fumarsäure sowie deren Kombinationen oder Mischungen, bevorzugt ausgewählt aus der Gruppe von Bernsteinsäure und Adipinsäure und deren Anhydriden sowie deren Kombinationen oder Mischungen insbesondere wobei im Fall der Citronensäure die vorhandene weitere Carboxylgruppe mit einem Rest - CH(CH₃) - CH₂ - C(O)OR¹ mit R¹ wie zuvor definiert verestert ist;
   - einen Rest H₃C - CH(OH) - CH₂ - C(O) -;
   - einen Rest - Y - OH mit Y wie zuvor definiert;
jedoch mit der Maßgabe, dass mindestens ein Rest R⁵, insbesondere mindestens zwei Reste R⁵, einen Rest R¹ - O - C(O) - CH₂ - CH(CH₃) - O - Y - mit R¹ und Y jeweils wie zuvor definiert darstellt; und
wobei in der allgemeinen Formel (IVb) die Variable p eine ganze Zahl von 1 bis 6, insbesondere von 1 bis 4, vorzugsweise 1 oder 2, besonders bevorzugt 1, darstellt.

In diesem Zusammenhang bedeutet "abgeleitet von", dass Y aus den genannten Carbonsäuren gebildet wird; insbesondere wird durch Veresterung der Wasserstoff des Carboxyls verestert; d. h. also, es liegt jeweils der Carboxylatrest der entsprechenden Säure als Y vor (d. h. also Y ist ein Succinat-Rest im Fall der Bernsteinsäure, ein Tartrat-Rest im Fall der Weinsäure, ein Citrat-Rest im Fall der Citronensäure, ein Malat-Rest im Fall der Äpfelsäure, ein Adipat-Rest im Fall der Adipinsäure, ein Fumarat-Rest im Fall der Fumarsäure und ein Maleat-Rest im Fall der Maleinsäure).

In diesem Zusammenhang bedeutet "verestert mit", dass im Fall der Citronensäure in der weiteren, insbesondere dritten, Carboxylgruppe der Wasserstoff durch einen Rest - CH(CH₃) - CH₂ - C(O)OR¹ ersetzt wird.

Gemäß einer wiederum weiteren besonderen Ausführungsform des erfindungsgemäßen Verfahrens können als Reaktionsprodukt (IV) ein oder mehrere Polyglycerinester von mit Oxobutanol veresterten Polycarbonsäuren, insbesondere ein oder mehrere Polyglycerinester von mit 4-Oxo-2-butanol veresterten Polycarbonsäuren, vorzugsweise ein oder mehrere ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, der allgemeinen Formel (IVb)

R⁵O-CH₂-CH(OR⁵)-CH₂-[O-CH₂-CH(OR⁵)-CH₂]ₚ-OR⁵ (IVb)

erhalten werden und/oder erhältlich sein,
wobei in der allgemeinen Formel (IVb) der Rest R⁵, unabhängig voneinander, darstellt:
   - Wasserstoff;
   - einen Rest R¹ - O - C(O) - CH₂ - CH(CH₃) - O - Y -, wobei der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und
      wobei Y einen der folgenden Reste darstellt: 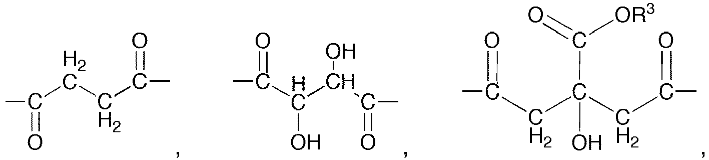 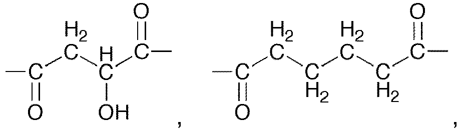 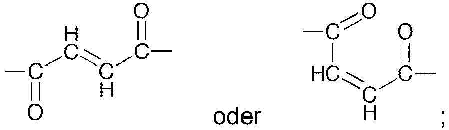 wobei in den vorstehenden Resten der Rest R³ Wasserstoff oder einen Rest - CH(CH₃) - CH₂ - C(O)OR¹ mit R¹ wie zuvor definiert darstellt;
   - einen Rest H₃C - CH(OH) - CH₂ - C(O) -;
   - einen Rest Y - OH mit Y wie zuvor definiert;
jedoch mit der Maßgabe, dass mindestens ein Rest R⁵, insbesondere mindestens zwei Reste R⁵, einen Rest R¹ - O - C(O) - CH₂ - CH(CH₃) - O - Y - mit R¹ und Y jeweils wie zuvor definiert darstellt; und
wobei in der allgemeinen Formel (IVb) die Variable p eine ganze Zahl von 1 bis 6, insbesondere von 1 bis 4, vorzugsweise 1 oder 2, besonders bevorzugt 1, darstellt.

Darüber hinaus können gemäß einer besonderen Ausführungsform des erfindungsgemäßen Verfahrens als Reaktionsprodukt (IV) ein oder mehrere Polyglycerinester von mit Oxobutanol veresterten Polycarbonsäuren, insbesondere ein oder mehrere Polyglycerinester von mit 4-Oxo-2-butanol veresterten Polycarbonsäuren, vorzugsweise ein oder mehrere ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, der allgemeinen Formel (IVb)

R⁵O-CH₂-CH(OR⁵)-CH₂-[O-CH₂-CH(OR⁵)-CH₂]ₚ-OR⁵ (IVb)

erhalten werden und/oder erhältlich sein,
wobei in der allgemeinen Formel (IVb) der Rest R⁵, unabhängig voneinander, darstellt:
   - Wasserstoff;
   - einen Rest R¹ - O - C(O) - CH₂ - CH(CH₃) - O - Y -, wobei der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und
      wobei Y einen der folgenden Reste darstellt: 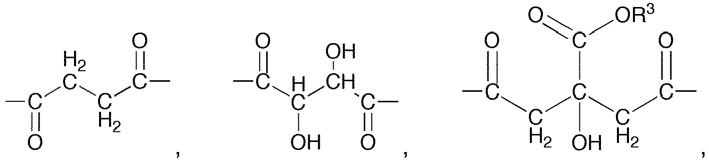 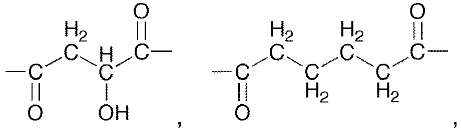 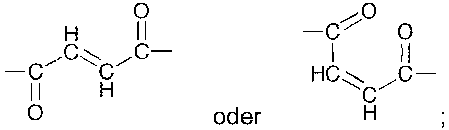 wobei in den vorstehenden Resten der Rest R³ Wasserstoff oder einen Rest - CH(CH₃) - CH₂ - C(O)OR¹ mit R¹ wie zuvor definiert darstellt;
jedoch mit der Maßgabe, dass mindestens ein Rest R⁵, insbesondere mindestens zwei Reste R⁵, einen Rest R¹ - O - C(O) - CH₂ - CH(CH₃) - O - Y - mit R¹ und Y jeweils wie zuvor definiert darstellt; und
wobei in der allgemeinen Formel (IVb) die Variable p eine ganze Zahl von 1 bis 6, insbesondere von 1 bis 4, vorzugsweise 1 oder 2, besonders bevorzugt 1, darstellt.

Darüber hinaus können gemäß einer weiteren besonderen Ausführungsform des erfindungsgemäßen Verfahrens als Reaktionsprodukt (IV) ein oder mehrere Polyglycerinester von mit Oxobutanol veresterten Polycarbonsäuren, insbesondere ein oder mehrere Polyglycerinester von mit 4-Oxo-2-butanol veresterten Polycarbonsäuren, vorzugsweise ein oder mehrere ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, der allgemeinen Formel (IVc)

R⁴O-CH₂-CH(OR⁴)-CH₂-O-CH₂-CH(OR⁴)-CH₂-OR⁴ (IVc)

erhalten werden und/oder erhältlich sein,
wobei in der allgemeinen Formel (IVc) der Rest R⁴, unabhängig voneinander, darstellt:
   - Wasserstoff;
   - einen Rest R¹ - O - C(O) - CH₂ - CH(CH₃) - O - (O)C - X - C(O) -, wobei der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und wobei X einen 1 bis 10, vorzugsweise 2 bis 6 Kohlenstoffatome enthaltenden und gegebenenfalls ein- oder mehrfach substituierten, insbesondere mit einem oder mehreren Hydroxyl-Resten und/oder Resten - O - C(O) - CH₂ - CH(OH) - CH₃ und/oder Carboxyl-Resten und/oder Resten - C(O) - O - CH(CH₃) - CH₂ - C(O)OR¹ mit R¹ wie zuvor definiert substituierten, gesättigten oder ungesättigten organischen Rest darstellt;
   - einen Rest H₃C - CH(OH) - CH₂ - C(O) -;
   - einen Rest HOOC - X - C(O) -, wobei X einen 1 bis 10, vorzugsweise 2 bis 6 Kohlenstoffatome enthaltenden und gegebenenfalls ein- oder mehrfach substituierten, insbesondere mit einem oder mehreren Hydroxyl-Resten und/oder Resten - O - C(O) - CH₂ - CH(OH) - CH₃ und/oder Carboxyl-Resten und/oder Resten - C(O) - O - CH(CH₃) - CH₂ - C(O)OR¹ mit R¹ wie zuvor definiert substituierten, gesättigten oder ungesättigten organischen Rest darstellt;
jedoch mit der Maßgabe, dass mindestens ein Rest R⁴, insbesondere mindestens zwei Reste R⁴, einen Rest R¹ - O - C(O) - CH₂ - CH(CH₃) - O - (O)C - X - C(O) -, wie zuvor definiert, darstellt.

Insbesondere können gemäß einer besonderen Ausführungsform des erfindungsgemäßen Verfahrens als Reaktionsprodukt (IV) ein oder mehrere Polyglycerinester von mit Oxobutanol veresterten Polycarbonsäuren, insbesondere ein oder mehrere Polyglycerinester von mit 4-Oxo-2-butanol veresterten Polycarbonsäuren, vorzugsweise ein oder mehrere ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, der allgemeinen Formel (IVc)

R⁴O-CH₂-CH(OR⁴)-CH₂-O-CH₂-CH(OR⁴)-CH₂-OR⁴ (IVc)

erhalten werden und/oder erhältlich sein,
wobei in der allgemeinen Formel (IVc) der Rest R⁴, unabhängig voneinander, darstellt:
   - Wasserstoff;
   - einen Rest R¹ - O - C(O) - CH₂ - CH(CH₃) - O - (O)C - X - C(O) -, wobei der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und wobei X einen 1 bis 10, vorzugsweise 2 bis 6 Kohlenstoffatome enthaltenden und gegebenenfalls ein- oder mehrfach substituierten, insbesondere mit einem oder mehreren Hydroxyl-Resten und/oder Resten - O - C(O) - CH₂ - CH(OH) - CH₃ und/oder Carboxyl-Resten und/oder Resten - C(O) - O - CH(CH₃) - CH₂ - C(O)OR¹ mit R¹ wie zuvor definiert substituierten, gesättigten oder ungesättigten organischen Rest darstellt;
jedoch mit der Maßgabe, dass mindestens ein Rest R⁴, insbesondere mindestens zwei Reste R⁴, einen Rest R¹ - O - C(O) - CH₂ - CH(CH₃) - O - (O)C - X - C(O) -, wie zuvor definiert, darstellt.

Weiterhin können gemäß einer zusätzlichen besonderen Ausführungsform des erfindungsgemäßen Verfahrens als Reaktionsprodukt (IV) ein oder mehrere Polyglycerinester von mit Oxobutanol veresterten Polycarbonsäuren, insbesondere ein oder mehrere Polyglycerinester von mit 4-Oxo-2-butanol veresterten Polycarbonsäuren, vorzugsweise ein oder mehrere ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, der allgemeinen Formel (IVc)

R⁴O-CH₂-CH(OR⁴)-CH₂-O-CH₂-CH(OR⁴)-CH₂-OR⁴ (IVc)

erhalten werden und/oder erhältlich sein,
wobei in der allgemeinen Formel (IVc) der Rest R⁴, unabhängig voneinander, darstellt:
   - Wasserstoff;
   - einen Rest R¹ - O - C(O) - CH₂ - CH(CH₃) - O - (O)C - X - C(O) -, wobei der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und wobei X einen 1 bis 10, vorzugsweise 2 bis 6 Kohlenstoffatome enthaltenden und gegebenenfalls ein- oder mehrfach substituierten, insbesondere mit einem oder mehreren Hydroxyl-Resten und/oder Carboxyl-Resten substituierten, gesättigten oder ungesättigten organischen Rest darstellt;
jedoch mit der Maßgabe, dass mindestens ein Rest R⁴, insbesondere mindestens zwei Reste R⁴, einen Rest R¹ - O - C(O) - CH₂ - CH(CH₃) - O - (O)C - X - C(O) -, wie zuvor definiert, darstellt.

Insbesondere können gemäß einer besonderen Ausführungsform des erfindungsgemäßen Verfahrens als Reaktionsprodukt (IV) ein oder mehrere Polyglycerinester von mit Oxobutanol veresterten Polycarbonsäuren, insbesondere ein oder mehrere Polyglycerinester von mit 4-Oxo-2-butanol veresterten Polycarbonsäuren, vorzugsweise ein oder mehrere ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, der allgemeinen Formel (IVd)

R⁵O-CH₂-CH(OR⁵)-CH₂-O-CH₂-CH(OR⁵)-CH₂-OR⁵ (IVd)

erhalten werden und/oder erhältlich sein,
wobei in der allgemeinen Formel (IVd) der Rest R⁵, unabhängig voneinander, darstellt:
   - Wasserstoff;
   - einen Rest R¹ - O - C(O) - CH₂ - CH(CH₃) - O - Y -, wobei der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und wobei Y abgeleitet ist von einer Polycarbonsäure, ausgewählt aus der Gruppe von Bernsteinsäure, Weinsäure, Citronensäure, Äpfelsäure, Adipinsäure, Fumarsäure und Maleinsäure sowie deren Kombinationen oder Mischungen, insbesondere ausgewählt aus der Gruppe von Bernsteinsäure, Weinsäure, Citronensäure, Äpfelsäure, Adipinsäure und Fumarsäure sowie deren Kombinationen oder Mischungen, bevorzugt ausgewählt aus der Gruppe von Bernsteinsäure und Adipinsäure und deren Anhydriden sowie deren Kombinationen oder Mischungen insbesondere wobei im Fall der Citronensäure die vorhandene weitere Carboxylgruppe mit einem Rest - CH(CH₃) - CH₂ - C(O)OR¹ mit R¹ wie zuvor definiert verestert ist;
   - einen Rest H₃C - CH(OH) - CH₂ - C(O) -;
   - einen Rest Y - OH mit Y wie zuvor definiert;
jedoch mit der Maßgabe, dass mindestens ein Rest R⁵, insbesondere mindestens zwei Reste R⁵, einen Rest R¹ - O - C(O) - CH₂ - CH(CH₃) - O - Y - mit R¹ und Y jeweils wie zuvor definiert darstellt.

In diesem Zusammenhang bedeutet "abgeleitet von", dass Y aus den genannten Carbonsäuren gebildet wird; insbesondere wird durch Veresterung der Wasserstoff des Carboxyls verestert; d. h. also, es liegt jeweils der Carboxylatrest der entsprechenden Säure als Y vor (d. h. also Y ist ein Succinat-Rest im Fall der Bernsteinsäure, ein Tartrat-Rest im Fall der Weinsäure, ein Citrat-Rest im Fall der Citronensäure, ein Malat-Rest im Fall der Äpfelsäure, ein Adipat-Rest im Fall der Adipinsäure, ein Fumarat-Rest im Fall der Fumarsäure und ein Maleat-Rest im Fall der Maleinsäure).

In diesem Zusammenhang bedeutet "verestert mit", dass im Fall der Citronensäure in der weiteren, insbesondere dritten, Carboxylgruppe der Wasserstoff durch einen Rest - CH(CH₃) - CH₂ - C(O)OR¹ ersetzt wird.

Weiterhin können gemäß einer besonderen Ausführungsform des erfindungsgemäßen Verfahrens als Reaktionsprodukt (IV) ein oder mehrere Polyglycerinester von mit Oxobutanol veresterten Polycarbonsäuren, insbesondere ein oder mehrere Polyglycerinester von mit 4-Oxo-2-butanol veresterten Polycarbonsäuren, vorzugsweise ein oder mehrere ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, der allgemeinen Formel (IVd)

R⁵O-CH₂-CH(OR⁵)-CH₂-O-CH₂-CH(OR⁵)-CH₂-OR⁵ (IVd)

erhalten werden und/oder erhältlich sein,
wobei in der allgemeinen Formel (IVd) der Rest R⁵, unabhängig voneinander, darstellt:
   - Wasserstoff;
   - einen Rest R¹ - O - C(O) - CH₂ - CH(CH₃) - O - Y -, wobei der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und wobei Y einen der folgenden Reste darstellt 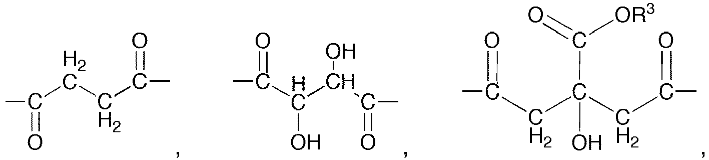 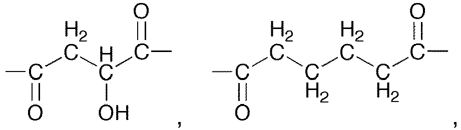 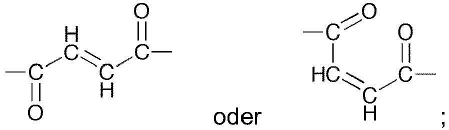 wobei in den vorstehenden Resten der Rest R³ Wasserstoff oder einen Rest - CH(CH₃) - CH₂ - C(O)OR¹ mit R¹ wie zuvor definiert darstellt;
   - einen Rest H₃C - CH(OH) - CH₂ - C(O) -;
   - einen Rest Y - OH mit Y wie zuvor definiert;
jedoch mit der Maßgabe, dass mindestens ein Rest R⁵, insbesondere mindestens zwei Reste R⁵, einen Rest R' - O - C(O) - CH₂ - CH(CH₃) - O - Y - mit R¹ und Y jeweils wie zuvor definiert darstellt.

Darüber hinaus können gemäß einer besonderen Ausführungsform des erfindungsgemäßen Verfahrens als Reaktionsprodukt (IV) ein oder mehrere Polyglycerinester von mit Oxobutanol veresterten Polycarbonsäuren, insbesondere ein oder mehrere Polyglycerinester von mit 4-Oxo-2-butanol veresterten Polycarbonsäuren, vorzugsweise ein oder mehrere ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, der allgemeinen Formel (IVd)

R⁵O-CH₂-CH(OR⁵)-CH₂-O-CH₂-CH(OR⁵)-CH₂-OR⁵ (IVd)

erhalten werden und/oder erhältlich sein,
wobei in der allgemeinen Formel (IVd) der Rest R⁵, unabhängig voneinander, darstellt:
   - Wasserstoff;
   - einen Rest R¹ - O - C(O) - CH₂ - CH(CH₃) - O - Y -, wobei der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und wobei Y einen der folgenden Reste darstellt 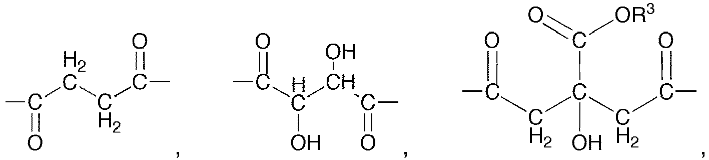 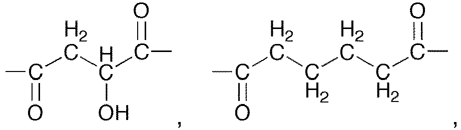 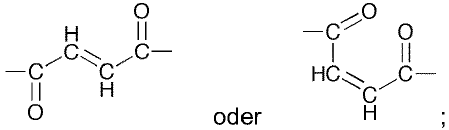 wobei in den vorstehenden Resten der Rest R³ Wasserstoff oder einen Rest - CH(CH₃) - CH₂ - C(O)OR¹ mit R¹ wie zuvor definiert darstellt;
jedoch mit der Maßgabe, dass mindestens ein Rest R⁵, insbesondere mindestens zwei Reste R⁵, einen Rest R¹ - O - C(O) - CH₂ - CH(CH₃) - O - Y - mit R¹ und Y jeweils wie zuvor definiert darstellt.

Gemäß einer wiederum weiteren besonderen Ausführungsform des erfindungsgemäßen Verfahrens kann als Reaktionsprodukt (IV) ein Gemisch von mindestens zwei voneinander verschiedenen Polyglycerinestern von mit Oxobutanol veresterten Polycarbonsäuren, insbesondere Polyglycerinestern von mit 4-Oxo-2-butanol veresterten Polycarbonsäuren, vorzugsweise ein- oder mehrwertigen Polyglycerinestern von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, erhalten werden und/oder erhältlich sein.

Weiterer Gegenstand - gemäß einem **zweiten** Aspekt der vorliegenden Erfindung - ist ein Reaktionszwischenprodukt in Form eines Polycarbonsäureesters von Oxobutanol, insbesondere Polycarbonsäureesters von 3-Hydroxybutanoat, bevorzugt Polycarbonsäureesters von 4-Oxo-2-butanol, vorzugsweise Polycarbonsäureesters von 4-Oxo-4-(C₁-C₅-alkoxy)-2-butanol oder von 4-Oxo-4-(hydroxy-C₃-C₅-alkoxy)-2-butanol, welches erhältlich ist nach dem zuvor beschriebenen Verfahren, insbesondere erhältlich ist als Reaktionszwischenprodukt (IV') des ersten Verfahrensschritts (a) des zuvor beschriebenen Verfahrens.

Gemäß einer besonderen Ausführungsform kann das Reaktionszwischenprodukt insbesondere ein Gemisch von mindestens zwei voneinander verschiedenen Polycarbonsäureestern von Oxobutanol, wie zuvor definiert, umfassen.

Gegenstand der vorliegenden Erfindung gemäß diesem Erfindungsaspekt ist aber auch ein Reaktionsprodukt in Form eines Polyglycerinesters von mit Oxobutanol veresterten Polycarbonsäuren, insbesondere Polyglycerinesters von mit 4-Oxo-2-butanol veresterten Polycarbonsäuren, vorzugsweise ein- oder mehrwertigen Polyglycerinesters von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, welches erhältlich ist nach dem zuvor beschriebenen Verfahren.

Gegenstand der vorliegenden Erfindung gemäß diesem Erfindungsaspekt ist aber auch ein Polyglycerinester von mit Oxobutanol veresterten Polycarbonsäuren, wobei der Polyglycerinester von mit Oxobutanol veresterten Polycarbonsäuren, insbesondere der Polyglycerinester von mit 4-Oxo-2-butanol veresterten Polycarbonsäuren, vorzugsweise der ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, der allgemeinen Formel (IVa)

R⁴O-CH₂-CH(OR⁴)-CH₂-[O-CH₂-CH(OR⁴)-CH₂]ₚ-OR⁴ (IVa)

entspricht,
wobei in der allgemeinen Formel (IVa) der Rest R⁴, unabhängig voneinander, darstellt:
   - Wasserstoff;
   - einen Rest R¹ - O - C(O) - CH₂ - CH(CH₃) - O - (O)C - X - C(O) -, wobei der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und wobei X einen 1 bis 10, vorzugsweise 2 bis 6 Kohlenstoffatome enthaltenden und gegebenenfalls ein- oder mehrfach substituierten, insbesondere mit einem oder mehreren Hydroxyl-Resten und/oder Resten - O - C(O) - CH₂ - CH(OH) - CH₃ und/oder Carboxyl-Resten und/oder Resten - C(O) - O - CH(CH₃) - CH₂ - C(O)OR¹ mit R¹ wie zuvor definiert substituierten, gesättigten oder ungesättigten organischen Rest darstellt;
   - einen Rest H₃C - CH(OH) - CH₂ - C(O) -;
   - einen Rest HOOC - X - C(O) -, wobei X einen 1 bis 10, vorzugsweise 2 bis 6 Kohlenstoffatome enthaltenden und gegebenenfalls ein- oder mehrfach substituierten, insbesondere mit einem oder mehreren Hydroxyl-Resten und/oder Resten - O - C(O) - CH₂ - CH(OH) - CH₃ und/oder Carboxyl-Resten und/oder Resten - C(O) - O - CH(CH₃) - CH₂ - C(O)OR¹ mit R¹ wie zuvor definiert substituierten, gesättigten oder ungesättigten organischen Rest darstellt;
jedoch mit der Maßgabe, dass mindestens ein Rest R⁴, insbesondere mindestens zwei Reste R⁴, einen Rest R¹ - O - C(O) - CH₂ - CH(CH₃) - O - (O)C - X - C(O) -, wie zuvor definiert, darstellt; und
wobei in der allgemeinen Formel (IVa) die Variable p eine ganze Zahl von 1 bis 6, insbesondere von 1 bis 4, vorzugsweise 1 oder 2, besonders bevorzugt 1, darstellt.

Gemäß einer wiederum weiteren besonderen Ausführungsform kann der erfindungsgemäße bzw. erfindungsgemäß hergestellte Polyglycerinester von mit Oxobutanol veresterten Polycarbonsäuren, insbesondere der Polyglycerinester von mit 4-Oxo-2-butanol veresterten Polycarbonsäuren, vorzugsweise der ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, der allgemeinen Formel (IVa)

R⁴O-CH₂-CH(OR⁴)-CH₂-[O-CH₂-CH(OR⁴)-CH₂]ₚ-OR⁴ (IVa)

entsprechen,
wobei in der allgemeinen Formel (IVa) der Rest R⁴, unabhängig voneinander, darstellt:
   - Wasserstoff;
   - einen Rest R¹ - O - C(O) - CH₂ - CH(CH₃) - O - (O)C - X - C(O) -, wobei der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und wobei X einen 1 bis 10, vorzugsweise 2 bis 6 Kohlenstoffatome enthaltenden und gegebenenfalls ein- oder mehrfach substituierten, insbesondere mit einem oder mehreren Hydroxyl-Resten und/oder Resten - O - C(O) - CH₂ - CH(OH) - CH₃ und/oder Carboxyl-Resten und/oder Resten - C(O) - O - CH(CH₃) - CH₂ - C(O)OR¹ mit R¹ wie zuvor definiert substituierten, gesättigten oder ungesättigten organischen Rest darstellt;
jedoch mit der Maßgabe, dass mindestens ein Rest R⁴, insbesondere mindestens zwei Reste R⁴, einen Rest R¹ - O - C(O) - CH₂ - CH(CH₃) - O - (O)C - X - C(O) -, wie zuvor definiert, darstellt; und
wobei in der allgemeinen Formel (IVa) die Variable p eine ganze Zahl von 1 bis 6, insbesondere von 1 bis 4, vorzugsweise 1 oder 2, besonders bevorzugt 1, darstellt.

Gemäß einer weiteren besonderen Ausführungsform der vorliegenden Erfindung kann der erfindungsgemäße bzw. erfindungsgemäß hergestellte Polyglycerinester von mit Oxobutanol veresterten Polycarbonsäuren, insbesondere der Polyglycerinester von mit 4-Oxo-2-butanol veresterten Polycarbonsäuren, vorzugsweise der ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, der allgemeinen Formel (IVa)

R⁴O-CH₂-CH(OR⁴)-CH₂-[O-CH₂-CH(OR⁴)-CH₂]ₚ-OR⁴ (IVa)

entsprechen,
wobei in der allgemeinen Formel (IVa) der Rest R⁴, unabhängig voneinander, darstellt:
   - Wasserstoff;
   - einen Rest R¹ - O - C(O) - CH₂ - CH(CH₃) - O - (O)C - X - C(O) -, wobei der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und wobei X einen 1 bis 10, vorzugsweise 2 bis 6 Kohlenstoffatome enthaltenden und gegebenenfalls ein- oder mehrfach substituierten, insbesondere mit einem oder mehreren Hydroxyl-Resten und/oder Carboxyl-Resten substituierten, gesättigten oder ungesättigten organischen Rest darstellt;
jedoch mit der Maßgabe, dass mindestens ein Rest R⁴, insbesondere mindestens zwei Reste R⁴, einen Rest R¹ - O - C(O) - CH₂ - CH(CH₃) - O - (O)C - X - C(O) -, wie zuvor definiert, darstellt; und
wobei in der allgemeinen Formel (IVa) die Variable p eine ganze Zahl von 1 bis 6, insbesondere von 1 bis 4, vorzugsweise 1 oder 2, besonders bevorzugt 1, darstellt.

Gemäß einer weiteren besonderen Ausführungsform der vorliegenden Erfindung kann der erfindungsgemäße bzw. erfindungsgemäß hergestellte Polyglycerinester von mit Oxobutanol veresterten Polycarbonsäuren, insbesondere der Polyglycerinester von mit 4-Oxo-2-butanol veresterten Polycarbonsäuren, vorzugsweise der ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, der allgemeinen Formel (IVb)

R⁵O-CH₂-CH(OR⁵)-CH₂-[O-CH₂-CH(OR⁵)-CH₂]ₚ-OR⁵ (IVb)

entsprechen,
wobei in der allgemeinen Formel (IVb) der Rest R⁵, unabhängig voneinander, darstellt:
   - Wasserstoff;
   - einen Rest R¹ - O - C(O) - CH₂ - CH(CH₃) - O - Y -, wobei der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und wobei Y abgeleitet ist von einer Polycarbonsäure, ausgewählt aus der Gruppe von Bernsteinsäure, Weinsäure, Citronensäure, Äpfelsäure, Adipinsäure, Fumarsäure und Maleinsäure sowie deren Kombinationen oder Mischungen, insbesondere ausgewählt aus der Gruppe von Bernsteinsäure, Weinsäure, Citronensäure, Äpfelsäure, Adipinsäure und Fumarsäure sowie deren Kombinationen oder Mischungen, bevorzugt ausgewählt aus der Gruppe von Bernsteinsäure und Adipinsäure und deren Anhydriden sowie deren Kombinationen oder Mischungen insbesondere wobei im Fall der Citronensäure die vorhandene weitere Carboxylgruppe mit einem Rest - CH(CH₃) - CH₂ - C(O)OR¹ mit R¹ wie zuvor definiert verestert ist;
   - einen Rest H₃C - CH(OH) - CH₂ - C(O) -;
   - einen Rest - Y - OH mit Y wie zuvor definiert;
jedoch mit der Maßgabe, dass mindestens ein Rest R⁵, insbesondere mindestens zwei Reste R⁵, einen Rest R¹ - O - C(O) - CH₂ - CH(CH₃) - O - Y - mit R¹ und Y jeweils wie zuvor definiert darstellt; und
wobei in der allgemeinen Formel (IVb) die Variable p eine ganze Zahl von 1 bis 6, insbesondere von 1 bis 4, vorzugsweise 1 oder 2, besonders bevorzugt 1, darstellt.

In diesem Zusammenhang bedeutet "abgeleitet von", dass Y aus den genannten Carbonsäuren gebildet wird; insbesondere wird durch Veresterung der Wasserstoff des Carboxyls verestert; d. h. also, es liegt jeweils der Carboxylatrest der entsprechenden Säure als Y vor (d. h. also Y ist ein Succinat-Rest im Fall der Bernsteinsäure, ein Tartrat-Rest im Fall der Weinsäure, ein Citrat-Rest im Fall der Citronensäure, ein Malat-Rest im Fall der Äpfelsäure, ein Adipat-Rest im Fall der Adipinsäure, ein Fumarat-Rest im Fall der Fumarsäure und ein Maleat-Rest im Fall der Maleinsäure).

In diesem Zusammenhang bedeutet "verestert mit", dass im Fall der Citronensäure in der weiteren, insbesondere dritten, Carboxylgruppe der Wasserstoff durch einen Rest - CH(CH₃) - CH₂ - C(O)OR¹ ersetzt wird.

Gemäß einer bevorzugten Ausführungsform der vorliegenden Erfindung kann der erfindungsgemäße bzw. erfindungsgemäß hergestellte Polyglycerinester von mit Oxobutanol veresterten Polycarbonsäuren, insbesondere der Polyglycerinester von mit 4-Oxo-2-butanol veresterten Polycarbonsäuren, vorzugsweise der ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, der allgemeinen Formel (IVb)

R⁵O-CH₂-CH(OR⁵)-CH₂-[O-CH₂-CH(OR⁵)-CH₂]ₚ-OR⁵ (IVb)

entsprechen,
wobei in der allgemeinen Formel (IVb) der Rest R⁵, unabhängig voneinander, darstellt:
   - Wasserstoff;
   - einen Rest R¹ - O - C(O) - CH₂ - CH(CH₃) - O - Y -, wobei der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und
      wobei Y einen der folgenden Reste darstellt: 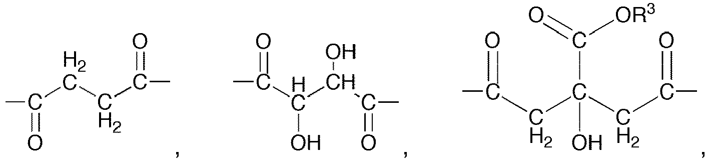 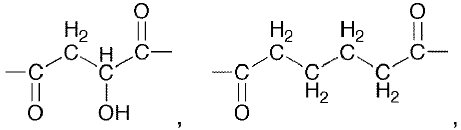 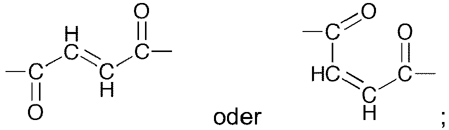 wobei in den vorstehenden Resten der Rest R³ Wasserstoff oder einen Rest - CH(CH₃) - CH₂ - C(O)OR¹ mit R¹ wie zuvor definiert darstellt;
   - einen Rest H₃C - CH(OH) - CH₂ - C(O) -;
   - einen Rest Y - OH mit Y wie zuvor definiert;
jedoch mit der Maßgabe, dass mindestens ein Rest R⁵, insbesondere mindestens zwei Reste R⁵, einen Rest R¹ - O - C(O) - CH₂ - CH(CH₃) - O - Y - mit R¹ und Y jeweils wie zuvor definiert darstellt; und
wobei in der allgemeinen Formel (IVb) die Variable p eine ganze Zahl von 1 bis 6, insbesondere von 1 bis 4, vorzugsweise 1 oder 2, besonders bevorzugt 1, darstellt.

Gemäß einer weiteren bevorzugten Ausführungsform dieses Erfindungsaspekts kann der erfindungsgemäße bzw. erfindungsgemäß hergestellte Polyglycerinester von mit Oxobutanol veresterten Polycarbonsäuren, insbesondere der Polyglycerinester von mit 4-Oxo-2-butanol veresterten Polycarbonsäuren, vorzugsweise der ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, der allgemeinen Formel (IVb)

R⁵O-CH₂-CH(OR⁵)-CH₂-[O-CH₂-CH(OR⁵)-CH₂]ₚ-OR⁵ (IVb)

entsprechen,
wobei in der allgemeinen Formel (IVb) der Rest R⁵, unabhängig voneinander, darstellt:
   - Wasserstoff;
   - einen Rest R¹ - O - C(O) - CH₂ - CH(CH₃) - O - Y -, wobei der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und
      wobei Y einen der folgenden Reste darstellt: 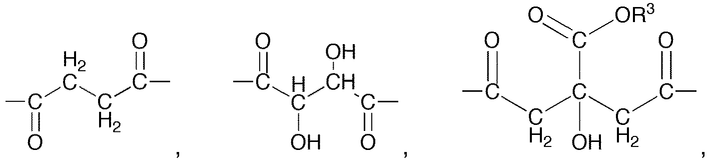 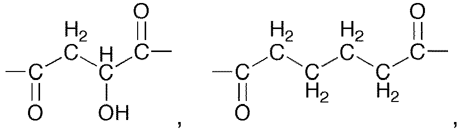 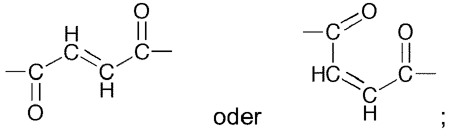 wobei in den vorstehenden Resten der Rest R³ Wasserstoff oder einen Rest - CH(CH₃) - CH₂ - C(O)OR¹ mit R¹ wie zuvor definiert darstellt;
jedoch mit der Maßgabe, dass mindestens ein Rest R⁵, insbesondere mindestens zwei Reste R⁵, einen Rest R¹ - O - C(O) - CH₂ - CH(CH₃) - O - Y - mit R¹ und Y jeweils wie zuvor definiert darstellt; und
wobei in der allgemeinen Formel (IVb) die Variable p eine ganze Zahl von 1 bis 6, insbesondere von 1 bis 4, vorzugsweise 1 oder 2, besonders bevorzugt 1, darstellt.

Weiterhin kann gemäß einer besonderen Ausführungsform dieses Erfindungsaspekts der erfindungsgemäße bzw. erfindungsgemäß hergestellte Polyglycerinester von mit Oxobutanol veresterten Polycarbonsäuren, insbesondere der Polyglycerinester von mit 4-Oxo-2-butanol veresterten Polycarbonsäuren, vorzugsweise der ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, der allgemeinen Formel (IVc)

R⁴O-CH₂-CH(OR⁴)-CH₂-O-CH₂-CH(OR⁴)-CH₂-OR⁴ (IVc)

entsprechen,
wobei in der allgemeinen Formel (IVc) der Rest R⁴, unabhängig voneinander, darstellt:
   - Wasserstoff;
   - einen Rest R¹ - O - C(O) - CH₂ - CH(CH₃) - O - (O)C - X - C(O) -, wobei der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und wobei X einen 1 bis 10, vorzugsweise 2 bis 6 Kohlenstoffatome enthaltenden und gegebenenfalls ein- oder mehrfach substituierten, insbesondere mit einem oder mehreren Hydroxyl-Resten und/oder Resten - O - C(O) - CH₂ - CH(OH) - CH₃ und/oder Carboxyl-Resten und/oder Resten - C(O) - O - CH(CH₃) - CH₂ - C(O)OR¹ mit R¹ wie zuvor definiert substituierten, gesättigten oder ungesättigten organischen Rest darstellt;
   - einen Rest H₃C - CH(OH) - CH₂ - C(O) -;
   - einen Rest HOOC - X - C(O) -, wobei X einen 1 bis 10, vorzugsweise 2 bis 6 Kohlenstoffatome enthaltenden und gegebenenfalls ein- oder mehrfach substituierten, insbesondere mit einem oder mehreren Hydroxyl-Resten und/oder Resten - O - C(O) - CH₂ - CH(OH) - CH₃ und/oder Carboxyl-Resten und/oder Resten - C(O) - O - CH(CH₃) - CH₂ - C(O)OR¹ mit R¹ wie zuvor definiert substituierten, gesättigten oder ungesättigten organischen Rest darstellt;
jedoch mit der Maßgabe, dass mindestens ein Rest R⁴, insbesondere mindestens zwei Reste R⁴, einen Rest R¹ - O - C(O) - CH₂ - CH(CH₃) - O - (O)C - X - C(O) -, wie zuvor definiert, darstellt.

Darüber hinaus kann gemäß einer weiteren besonderen Ausführungsform dieses Erfindungsaspekts der erfindungsgemäße bzw. erfindungsgemäß hergestellte Polyglycerinester von mit Oxobutanol veresterten Polycarbonsäuren, insbesondere der Polyglycerinester von mit 4-Oxo-2-butanol veresterten Polycarbonsäuren, vorzugsweise der ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, der allgemeinen Formel (IVc)

R⁴O-CH₂-CH(OR⁴)-CH₂-O-CH₂-CH(OR⁴)-CH₂-OR⁴ (IVc)

entsprechen,
wobei in der allgemeinen Formel (IVc) der Rest R⁴, unabhängig voneinander, darstellt:
   - Wasserstoff;
   - einen Rest R¹ - O - C(O) - CH₂ - CH(CH₃) - O - (O)C - X - C(O) -, wobei der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und wobei X einen 1 bis 10, vorzugsweise 2 bis 6 Kohlenstoffatome enthaltenden und gegebenenfalls ein- oder mehrfach substituierten, insbesondere mit einem oder mehreren Hydroxyl-Resten und/oder Resten - O - C(O) - CH₂ - CH(OH) - CH₃ und/oder Carboxyl-Resten und/oder Resten - C(O) - O - CH(CH₃) - CH₂ - C(O)OR¹ mit R¹ wie zuvor definiert substituierten, gesättigten oder ungesättigten organischen Rest darstellt;
jedoch mit der Maßgabe, dass mindestens ein Rest R⁴, insbesondere mindestens zwei Reste R⁴, einen Rest R¹ - O - C(O) - CH₂ - CH(CH₃) - O - (O)C - X - C(O) -, wie zuvor definiert, darstellt.

Auch kann gemäß einer wiederum weiteren besonderen Ausführungsform dieses Erfindungsaspekts der erfindungsgemäße bzw. erfindungsgemäß hergestellte Polyglycerinester von mit Oxobutanol veresterten Polycarbonsäuren, insbesondere der Polyglycerinester von mit 4-Oxo-2-butanol veresterten Polycarbonsäuren, vorzugsweise der ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, der allgemeinen Formel (IVc)

R⁴O-CH₂-CH(OR⁴)-CH₂-O-CH₂-CH(OR⁴)-CH₂-OR⁴ (IVc)

entsprechen,
wobei in der allgemeinen Formel (IVc) der Rest R⁴, unabhängig voneinander, darstellt:
   - Wasserstoff;
   - einen Rest R¹ - O - C(O) - CH₂ - CH(CH₃) - O - (O)C - X - C(O) -, wobei der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und wobei X einen 1 bis 10, vorzugsweise 2 bis 6 Kohlenstoffatome enthaltenden und gegebenenfalls ein- oder mehrfach substituierten, insbesondere mit einem oder mehreren Hydroxyl-Resten und/oder Carboxyl-Resten substituierten, gesättigten oder ungesättigten organischen Rest darstellt;
jedoch mit der Maßgabe, dass mindestens ein Rest R⁴, insbesondere mindestens zwei Reste R⁴, einen Rest R¹ - O - C(O) - CH₂ - CH(CH₃) - O - (O)C - X - C(O) -, wie zuvor definiert, darstellt.

Darüber hinaus kann es gemäß einer besonderen Ausführungsform dieses Erfindungsaspekts vorgesehen sein, dass der erfindungsgemäße bzw. erfindungsgemäß hergestellte Polyglycerinester von mit Oxobutanol veresterten Polycarbonsäuren, insbesondere der Polyglycerinester von mit 4-Oxo-2-butanol veresterten Polycarbonsäuren, vorzugsweise der ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, der allgemeinen Formel (IVd)

R⁵O-CH₂-CH(OR⁵)-CH₂-O-CH₂-CH(OR⁵)-CH₂-OR⁵ (IVd)

entspricht,
wobei in der allgemeinen Formel (IVd) der Rest R⁵, unabhängig voneinander, darstellt:
   - Wasserstoff;
   - einen Rest R¹ - O - C(O) - CH₂ - CH(CH₃) - O - Y -, wobei der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und wobei Y abgeleitet ist von einer Polycarbonsäure, ausgewählt aus der Gruppe von Bernsteinsäure, Weinsäure, Citronensäure, Äpfelsäure, Adipinsäure, Fumarsäure und Maleinsäure sowie deren Kombinationen oder Mischungen, insbesondere ausgewählt aus der Gruppe von Bernsteinsäure, Weinsäure, Citronensäure, Äpfelsäure, Adipinsäure und Fumarsäure sowie deren Kombinationen oder Mischungen, bevorzugt ausgewählt aus der Gruppe von Bernsteinsäure und Adipinsäure und deren Anhydriden sowie deren Kombinationen oder Mischungen insbesondere wobei im Fall der Citronensäure die vorhandene weitere Carboxylgruppe mit einem Rest - CH(CH₃) - CH₂ - C(O)OR¹ mit R¹ wie zuvor definiert verestert ist;
   - einen Rest H₃C - CH(OH) - CH₂ - C(O) -;
   - einen Rest Y - OH mit Y wie zuvor definiert;
jedoch mit der Maßgabe, dass mindestens ein Rest R⁵, insbesondere mindestens zwei Reste R⁵, einen Rest R¹ - O - C(O) - CH₂ - CH(CH₃) - O - Y - mit R¹ und Y jeweils wie zuvor definiert darstellt.

In diesem Zusammenhang bedeutet "abgeleitet von", dass Y aus den genannten Carbonsäuren gebildet wird; insbesondere wird durch Veresterung der Wasserstoff des Carboxyls verestert; d. h. also, es liegt jeweils der Carboxylatrest der entsprechenden Säure als Y vor (d. h. also Y ist ein Succinat-Rest im Fall der Bernsteinsäure, ein Tartrat-Rest im Fall der Weinsäure, ein Citrat-Rest im Fall der Citronensäure, ein Malat-Rest im Fall der Äpfelsäure, ein Adipat-Rest im Fall der Adipinsäure, ein Fumarat-Rest im Fall der Fumarsäure und ein Maleat-Rest im Fall der Maleinsäure).

In diesem Zusammenhang bedeutet "verestert mit", dass im Fall der Citronensäue in der weiteren, insbesondere dritten, Carboxylgruppe der Wasserstoff durch einen Rest - CH(CH₃) - CH₂ - C(O)OR¹ ersetzt wird.

Auch kann es gemäß einer weiteren besonderen Ausführungsform dieses Erfindungsaspekts vorgesehen sein, dass der erfindungsgemäße bzw. erfindungsgemäß hergestellte Polyglycerinester von mit Oxobutanol veresterten Polycarbonsäuren, insbesondere der Polyglycerinester von mit 4-Oxo-2-butanol veresterten Polycarbonsäuren, vorzugsweise der ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, der allgemeinen Formel (IVd)

R⁵O-CH₂-CH(OR⁵)-CH₂-O-CH₂-CH(OR⁵)-CH₂-OR⁵ (IVd)

entspricht,
wobei in der allgemeinen Formel (IVd) der Rest R⁵, unabhängig voneinander, darstellt:
   - Wasserstoff;
   - einen Rest R¹ - O - C(O) - CH₂ - CH(CH₃) - O - Y -, wobei der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und wobei Y einen der folgenden Reste darstellt 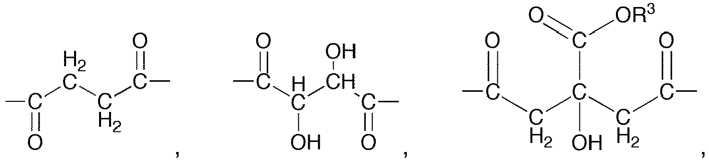 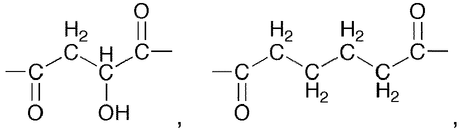 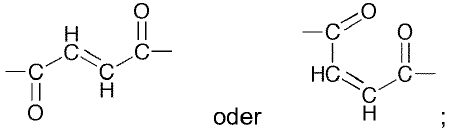 wobei in den vorstehenden Resten der Rest R³ Wasserstoff oder einen Rest - CH(CH₃) - CH₂ - C(O)OR¹ mit R¹ wie zuvor definiert darstellt;
   - einen Rest H₃C - CH(OH) - CH₂ - C(O) -;
   - einen Rest Y - OH mit Y wie zuvor definiert;
jedoch mit der Maßgabe, dass mindestens ein Rest R⁵, insbesondere mindestens zwei Reste R⁵, einen Rest R¹ - O - C(O) - CH₂ - CH(CH₃) - O - Y - mit R¹ und Y jeweils wie zuvor definiert darstellt.

Darüber hinaus kann gemäß einer besonderen Ausführungsform dieses Erfindungsaspekts der erfindungsgemäße bzw. erfindungsgemäß hergestellte Polyglycerinester von mit Oxobutanol veresterten Polycarbonsäuren, insbesondere der Polyglycerinester von mit 4-Oxo-2-butanol veresterten Polycarbonsäuren, vorzugsweise der ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, der allgemeinen Formel (IVd)

R⁵O-CH₂-CH(OR⁵)-CH₂-O-CH₂-CH(OR⁵)-CH₂-OR⁵ (IVd)

entsprechen,
wobei in der allgemeinen Formel (IVd) der Rest R⁵, unabhängig voneinander, darstellt:
   - Wasserstoff;
   - einen Rest R¹ - O - C(O) - CH₂ - CH(CH₃) - O - Y -, wobei der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und wobei Y einen der folgenden Reste darstellt 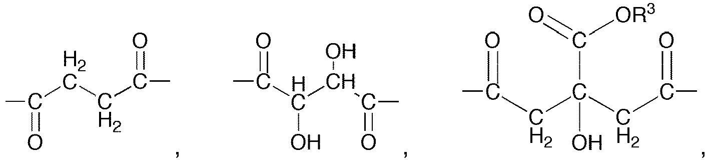 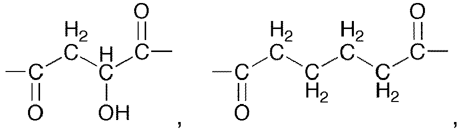 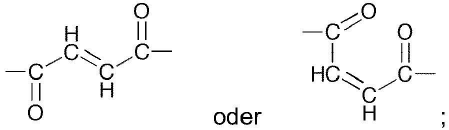 wobei in den vorstehenden Resten der Rest R³ Wasserstoff oder einen Rest - CH(CH₃) - CH₂ - C(O)OR¹ mit R¹ wie zuvor definiert darstellt;
jedoch mit der Maßgabe, dass mindestens ein Rest R⁵, insbesondere mindestens zwei Reste R⁵, einen Rest R¹ - O - C(O) - CH₂ - CH(CH₃) - O - Y - mit R¹ und Y jeweils wie zuvor definiert darstellt.

Darüber hinaus ist weiterer Gegenstand der vorliegenden Erfindung gemäß einer besonderen Ausführungsform dieses Erfindungsaspekts ein erfindungsgemäßes Gemisch, umfassend mindestens zwei voneinander verschiedene erfindungsgemäße bzw. erfindungsgemäß hergestellte Polyglycerinester von mit Oxobutanol veresterten Polycarbonsäuren, insbesondere Polyglycerinester von mit 4-Oxo-2-butanol veresterten Polycarbonsäuren, vorzugsweise ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, insbesondere wie zuvor definiert.

Das nach dem erfindungsgemäßen Verfahren erhältliche bzw. erfindungsgemäße Reaktionsprodukt, wie zuvor definiert, und/oder der nach dem erfindungsgemäßen Herstellungsverfahren erhältliche bzw. erfindungsgemäße Polyglycerinester von mit Oxobutanol veresterten Polycarbonsäuren, insbesondere der Polyglycerinester von mit 4-Oxo-2-butanol veresterten Polycarbonsäuren, vorzugsweise der ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, wie zuvor definiert, und/oder das nach dem erfindungsgemäßen Herstellungsverfahren erhältliche bzw. erfindungsgemäße Gemisch, wie zuvor definiert, weist gegenüber dem Stand der Technik eine Vielzahl von Vorteilen und Besonderheiten auf:
Wie die Anmelderin überraschend herausgefunden hat, eignet sich das nach dem erfindungsgemäßen Verfahren erhältliche bzw. erfindungsgemäße Reaktionsprodukt, wie zuvor definiert, und/oder der nach dem erfindungsgemäßen Herstellungsverfahren erhältliche bzw. erfindungsgemäße Polyglycerinester von mit Oxobutanol veresterten Polycarbonsäuren, insbesondere der Polyglycerinester von mit 4-Oxo-2-butanol veresterten Polycarbonsäuren, vorzugsweise der ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, wie zuvor definiert, und/oder das nach dem erfindungsgemäßen Herstellungsverfahren erhältliche bzw. erfindungsgemäße Gemisch, wie zuvor definiert, insbesondere als Präkursor bzw. Metabolit von 3-Hydroxybuttersäure bzw. deren Salzen, da dieses bzw. dieser einerseits physiologisch, insbesondere im Magen/Darm-Trakt, zu 3-Hydroxybuttersäure bzw. deren Salzen umgesetzt wird und andererseits gleichzeitig eine gute physiologische Kompatibilität bzw. Verträglichkeit aufweist, insbesondere im Hinblick auf Nichttoxizität und akzeptable organoleptische Eigenschaften.

Darüber hinaus ist das nach dem erfindungsgemäßen Verfahren erhältliche bzw. erfindungsgemäße Reaktionsprodukt, wie zuvor definiert, und/oder der nach dem erfindungsgemäßen Herstellungsverfahren erhältliche bzw. erfindungsgemäße Polyglycerinester von mit Oxobutanol veresterten Polycarbonsäuren, insbesondere Polyglycerinester von mit 4-Oxo-2-butanol veresterten Polycarbonsäuren, vorzugsweise ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, wie zuvor definiert, und/oder das nach dem erfindungsgemäßen Herstellungsverfahren erhältliche bzw. erfindungsgemäße Gemisch, wie zuvor definiert, ohne Weiteres auf synthetischem Wege auch in großtechnischem Maßstab zugänglich bzw. verfügbar, und zwar auch mit der erforderlichen pharmazeutischen bzw. pharmakologischen Qualität.

Zudem kann das nach dem erfindungsgemäßen Verfahren erhältliche bzw. erfindungsgemäße Reaktionsprodukt, wie zuvor definiert, und/oder der nach dem erfindungsgemäßen Herstellungsverfahren erhältliche bzw. erfindungsgemäße Polyglycerinester von mit Oxobutanol veresterten Polycarbonsäuren, insbesondere Polyglycerinester von mit 4-Oxo-2-butanol veresterten Polycarbonsäuren, vorzugsweise ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, wie zuvor definiert, und/oder das nach dem erfindungsgemäßen Herstellungsverfahren erhältliche bzw. erfindungsgemäße Gemisch, wie zuvor definiert, erforderlichenfalls in enantiomerenreiner bzw. enantiomerenangereicherter Form bereitgestellt werden.

Das nach dem erfindungsgemäßen Verfahren erhältliche bzw. erfindungsgemäße Reaktionsprodukt, wie zuvor definiert, und/oder der nach dem erfindungsgemäßen Herstellungsverfahren erhältliche bzw. erfindungsgemäße Polyglycerinester von mit Oxobutanol veresterten Polycarbonsäuren, insbesondere Polyglycerinester von mit 4-Oxo-2-butanol veresterten Polycarbonsäuren, vorzugsweise ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, wie zuvor definiert, und/oder das nach dem erfindungsgemäßen Herstellungsverfahren erhältliche bzw. erfindungsgemäße Gemisch, wie zuvor definiert, stellt somit ein effizientes pharmakologisches Wirkstoff-Target im Rahmen einer Ketokörper-Therapie des menschlichen oder tierischen Körpers dar.

Nachfolgend werden noch die übrigen Erfindungsaspekte weiterführende erläutert und im Detail beschrieben.

Weiterer Gegenstand der vorliegenden Erfindung - gemäß einem **dritten** Aspekt der vorliegenden Erfindung - ist eine pharmazeutische Zusammensetzung, insbesondere ein Arzneimittel oder Medikament, welche(s) ein nach dem erfindungsgemäßen Herstellungsverfahren erhältliches bzw. erfindungsgemäßes Reaktionsprodukt bzw. einen erfindungsgemäßen bzw. erfindungsgemäß hergestellten Polyglycerinester von mit Oxobutanol veresterten Polycarbonsäuren, insbesondere einen Polyglycerinester von mit 4-Oxo-2-butanol veresterten Polycarbonsäuren, vorzugsweise einen ein- oder mehrwertigen Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, wie zuvor definiert, und/oder ein nach dem erfindungsgemäßen Herstellungsverfahren erhältliches bzw. erfindungsgemäßes Gemisch, wie zuvor definiert, umfasst.

Insbesondere betrifft die vorliegende Erfindung gemäß diesem Erfindungsaspekt eine pharmazeutische Zusammensetzung zur prophylaktischen und/oder therapeutischen Behandlung bzw. zur Verwendung bei der prophylaktischen und/oder therapeutischen Behandlung von Erkrankungen des menschlichen oder tierischen Körpers. Hierbei kann es sich insbesondere um Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien, handeln.

Wiederum weiterer Gegenstand der vorliegenden Erfindung - gemäß einem **vierten** Aspekt der vorliegenden Erfindung - ist ein nach dem erfindungsgemäßen Herstellungsverfahren erhältliches bzw. erfindungsgemäßes Reaktionsprodukt bzw. erfindungsgemäßer bzw. erfindungsgemäß hergestellter Polyglycerinester von mit Oxobutanol veresterten Polycarbonsäuren, insbesondere Polyglycerinester von mit 4-Oxo-2-butanol veresterten Polycarbonsäuren, vorzugsweise ein- oder mehrwertiger Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, wie zuvor definiert, und/oder ein nach dem erfindungsgemäßen Herstellungsverfahren erhältliches bzw. erfindungsgemäßes Gemisch, wie zuvor definiert, zur prophylaktischen und/oder therapeutischen Behandlung bzw. zur Verwendung bei der prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

Auch kann die Verwendung eines nach dem erfindungsgemäßen Herstellungsverfahren erhältlichen bzw. erfindungsgemäßen Reaktionsprodukts bzw. eines erfindungsgemäßen bzw. erfindungsgemäß hergestellten Polyglycerinesters von mit Oxobutanol veresterten Polycarbonsäuren, insbesondere eines Polyglycerinesters von mit 4-Oxo-2-butanol veresterten Polycarbonsäuren, vorzugsweise eines ein- oder mehrwertigen Polyglycerinesters von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, wie zuvor definiert, und/oder eines nach dem erfindungsgemäßen Herstellungsverfahren erhältlichen bzw. erfindungsgemäßen Gemischs, wie zuvor definiert, zur prophylaktischen und/oder therapeutischen Behandlung bzw. zur Herstellung eines Arzneimittels zur prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien, vorgesehen sein.

Darüber hinaus kann die Verwendung eines nach dem erfindungsgemäßen Herstellungsverfahren erhältlichen bzw. erfindungsgemäßen Reaktionsprodukts bzw. eines erfindungsgemäßen bzw. erfindungsgemäß hergestellten Polyglycerinesters von mit Oxobutanol veresterten Polycarbonsäuren, insbesondere eines Polyglycerinesters von mit 4-Oxo-2-butanol veresterten Polycarbonsäuren, vorzugsweise eines ein- oder mehrwertigen Polyglycerinesters von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, wie zuvor definiert, und/oder eines nach dem erfindungsgemäßen Herstellungsverfahren erhältlichen bzw. erfindungsgemäßen Gemischs, wie zuvor definiert, zur prophylaktischen und/oder therapeutischen Behandlung bzw. zur Herstellung eines Arzneimittels zur prophylaktischen und/oder therapeutischen Behandlung bzw. zur Anwendung von/bei katabolen Stoffwechsellagen, wie Hunger, Diäten oder kohlenhydratarmer Ernährung, vorgesehen sein.

Gleichermaßen weiterer Gegenstand der vorliegenden Erfindung - gemäß einem **fünften** Aspekt der vorliegenden Erfindung - ist ein Nahrungsmittel- und/oder Lebensmittelerzeugnis, welches ein nach dem erfindungsgemäßen Herstellungsverfahren erhältliches bzw. erfindungsgemäßes Reaktionsprodukt bzw. einen erfindungsgemäßen bzw. erfindungsgemäß hergestellten Polyglycerinester von mit Oxobutanol veresterten Polycarbonsäuren, insbesondere einen Polyglycerinester von mit 4-Oxo-2-butanol veresterten Polycarbonsäuren, vorzugsweise einen ein- oder mehrwertigen Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, wie zuvor definiert, und/oder ein nach dem erfindungsgemäßen Herstellungsverfahren erhältliches bzw. erfindungsgemäßes Gemisch, wie zuvor definiert, umfasst.

Gemäß einer besonderen Ausführungsform kann das Nahrungsmittel- und/oder Lebensmittelerzeugnis insbesondere ein Nahrungsergänzungsmittel, ein funktionelles Lebensmittel (*Functional Food*), ein *Novel Food,* ein Lebensmittelzusatzstoff, ein Nahrungszusatz, ein diätetisches Lebensmittel, ein Power-Snack, ein Appetitzügler oder ein Kraft- und/oder Ausdauersport-Supplement sein.

Des Weiteren kann die Verwendung eines nach dem erfindungsgemäßen Herstellungsverfahren erhältlichen Reaktionsprodukts bzw. eines erfindungsgemäßen bzw. erfindungsgemäß hergestellten Polyglycerinesters von mit Oxobutanol veresterten Polycarbonsäuren, insbesondere eines Polyglycerinesters von mit 4-Oxo-2-butanol veresterten Polycarbonsäuren, vorzugsweise eines ein- oder mehrwertigen Polyglycerinesters von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, wie zuvor definiert, und/oder eines nach dem erfindungsgemäßen Herstellungsverfahren erhältlichen Gemischs, wie zuvor definiert, in einem Nahrungsmittel- und/oder Lebensmittelerzeugnis, vorgesehen sein.

Dabei kann das Nahrungsmittel- und/oder Lebensmittelerzeugnis insbesondere ein Nahrungsergänzungsmittel, ein funktionelles Lebensmittel (*Functional Food*), ein *Novel Food,* ein Lebensmittelzusatzstoff, ein Nahrungszusatz, ein diätetisches Lebensmittel, ein Power-Snack, ein Appetitzügler oder ein Kraft- und/oder Ausdauersport-Supplement sein.

Weitere Ausgestaltungen, Abwandlungen und Variationen der vorliegenden Erfindung sind für den Fachmann beim Lesen der Beschreibung ohne Weiteres erkennbar oder realisierbar, ohne dass er dabei den Rahmen der vorliegenden Erfindung verlässt.

Die vorliegende Erfindung wird anhand der nachfolgenden Ausführungsbeispiele veranschaulicht, welche die vorliegende Erfindung jedoch keinesfalls beschränken sollen, sondern lediglich die beispielhafte und nichtlimitierende Durchführungsweise der Ausgestaltung der vorliegenden Erfindung erläutern sollen.

### AUSFÜHRUNGSBEISPIELE:

### Verwendete Abkürzungen

| | |
|---|---|
| • 3-BHB-EE = BHB-EE | 3-Hydroxybuttersäureethylester (= Ethyl-3-hydroxybutanoat oder 4-Ethoxy-4-oxobutan-2-ol) |
| • 3-BHB = BHB | 3-Hydroxybuttersäure |
| • BS-(BHB-EE)-Monoester | Bernsteinsäuremonoester von Ethyl-3-hydroxybutanoat |
| • BS-(BHB-EE)-Monoester | Bernsteinsäurediester von Ethyl-3-hydroxybutanoat |
| • PG(2)-(BS-(BHB-EE))-Monoester | Diglycerinmonoester von mit Ethyl-3-hydroxybutanoat veresterter Bernsteinsäure |
| • PG(2)-(BS-(BHB-EE))-Diester | Diglycerindiester von mit Ethyl-3-hydroxybutanoat veresterter Bernsteinsäure |
| • PG(2)-(BS-(BHB-EE))-Polyester | Diglycerinpolyester von mit Ethyl-3-hydroxybutanoat veresterter Bernsteinsäure |

### I. Herstellungsbeispiele einer besonderen Ausführungsform des erfindungsgemäßen Verfahrens als Eintopfsynthese ohne die Verwendung eines Katalysators

### I. 1. Herstellung von Diglycerinestern von mit Ethyl-3-hydroxybutanoat veresterter Bernsteinsäure unter Verwendung von Bernsteinsäureanhydrid

In einem 500-ml-Mehrhalskolben mit Dephlegmator (Partialkondensator) und Destillationsbrücke werden 132 g (R)/(S)-3-Hydroxybuttersäureethylester (3-BHB-EE = Ethyl-3-hydroxybutanoat oder 4-Ethoxy-4-oxobutan-2-ol) (racemisch), 100 g Bernsteinsäureanhydrid und 40 g Diglycerin vorgelegt. Das Reaktionsgemisch wird unter Rühren bei 120 °C und unter N₂ für 7 h zur Reaktion gebracht und das entstehende Reaktionswasser wird kontinuierlich destillativ entfernt. Anschließend werden überschüssige 3-Hydroxybuttersäureethylester sowie überschüssiger Bernsteinsäureester von Ethyl-3-hydroxybutanoat unter Vakuum destillativ entfernt. Das gewünschte Reaktionsproduktgemisch wird dann als Rückstand erhalten. Der erhaltene Rückstand kann bei Bedarf für 2 bis 4 Stunden im Vakuum mit Wasserdampf behandelt werden.

Es wird ein Gemisch aus ein-, zwei-, drei- und vierwertigen Estern des Diglycerins (d. h. Diglycerinmonoester, Diglycerindiester, Diglycerintriester und Diglycerintetraester) von mit Ethyl-3-hydroxybutanoat veresterter Bernsteinsäure erhalten (siehe auch nachfolgendes Reaktionsschema).

Als Nebenprodukte werden unter anderem sehr geringe Mengen von Diglycerinestern von 3-Hydroxybuttersäure und Diglycerinestern von Bernsteinsäure gebildet.

Der Diglycerinmonoester von mit Ethyl-3-hydroxybutanoat veresterter Bernsteinsäure kann synonym auch als 1-[3-(2,3-dihydroxypropoxy)-2-hydroxypropyl] 4-(4-ethoxy-4-oxobutan-2-yl)-butandioat bezeichnet werden.

Der Diglycerindiester von mit Ethyl-3-hydroxybutanoat veresterter Bernsteinsäure kann synonym auch als 1-(4-ethoxy-4-oxobutan-2-yl) 4-{3-[3-({4-[(4-ethoxy-4-oxobutan-2-yl)oxy]-4-oxobutanoyl}oxy)-2-hydroxypropoxy]-2-hydroxypropyl}-butan-dioat bezeichnet werden.

Der Diglycerintriester von mit Ethyl-3-hydroxybutanoat veresterter Bernsteinsäure kann synonym auch als 1-{3-[2,3-bis({4-[(4-ethoxy-4-oxobutan-2-yl)oxy]-4-oxobutanoyl}oxy)propoxy]-2-hydroxypropyl}-4-(4-ethoxy-4-oxobutan-2-yl)-butan-dioat bezeichnet werden.

Der Diglycerintetraester von mit Ethyl-3-hydroxybutanoat veresterter Bernsteinsäure kann synonym auch als 1-{1-[2,3-bis({4-[(4-ethoxy-4-oxobutan-2-yl)oxy]-4-oxobutanoyl}oxy)propoxy]-3-({4-[(4-ethoxy-4-oxobutan-2-yl)oxy]-4-oxobutanoyl}-oxy)propan-2-yl}-4-(4-ethoxy-4-oxobutan-2-yl)-butanedioate bezeichnet werden.

Die Charakterisierung erfolgt mittels Massenspektrometrie (MS), Gelpermeationschromatographie (GPC) und Protonenresonanzspektroskopie (¹H-NMR).

Im Folgenden ist der Reaktionsverlauf schematisch dargestellt, wobei im Fall der Nebenprodukte freie Hydroxylgruppen gegebenenfalls auch (ganz oder teilweise) durch einen Rest - C(O) - CH₂ - CH(OH) - CH₃ (Veresterung mit Ethyl-3-hydroxybutanoat) oder einen Rest - C(O) - CH₂ - CH₂ - COOH (Veresterung mit Bernsteinsäureanhydrid) verestert sein können:

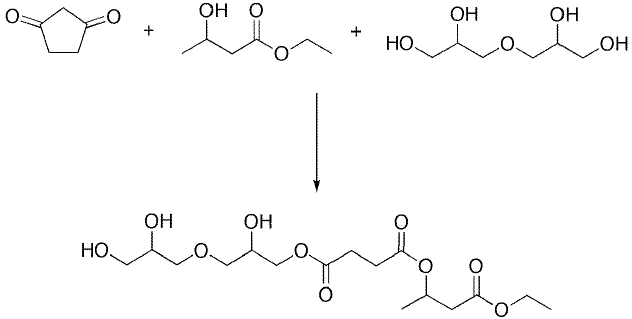

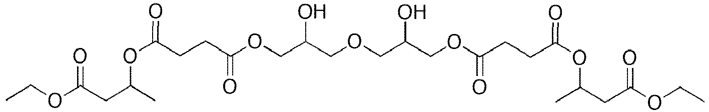

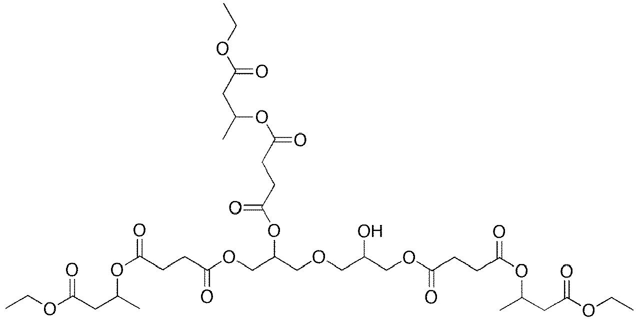

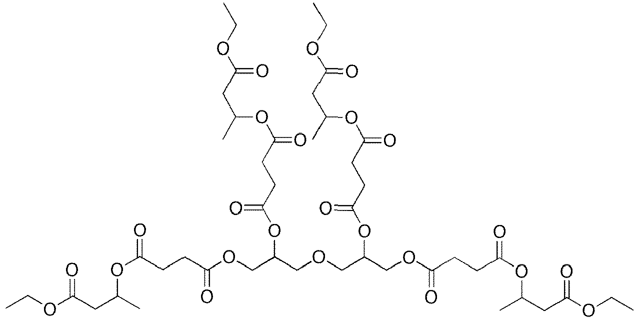

### I. 2. Herstellung von Diglycerinestern von mit Ethyl-3-hydroxybutanoat veresterter Bernsteinsäure unter Verwendung der freien Bernsteinsäure

In einem 500-ml-Mehrhalskolben mit Dephlegmator (Partialkondensator) und Destillationsbrücke werden 132 g (R)/(S)-3-Hydroxybuttersäureethylester (3-BHB-EE = Ethyl-3-hydroxybutanoat oder 4-Ethoxy-4-oxobutan-2-ol) (racemisch), 118 g Bernsteinsäure und 40 g Diglycerin vorgelegt. Das Reaktionsgemisch wird unter Rühren bei 120 °C und unter N₂ für 7 h zur Reaktion gebracht und das entstehende Reaktionswasser wird kontinuierlich destillativ entfernt. Anschließend werden überschüssiger 3-Hydroxybuttersäureethylester sowie überschüssiger Bernsteinsäureester von Ethyl-3-hydroxybutanoat unter Vakuum destillativ entfernt. Der erhaltene Rückstand wird bei Bedarf für 2 bis 4 Stunden im Vakuum mit Wasserdampf behandelt.

Es wird ein Gemisch aus ein-, zwei-, drei- und vierwertigen Diglycerinestern (d. h. Diglycerinmonoester, Diglycerindiester, Diglycerintriester und Diglycerintetraester) von mit Ethyl-3-hydroxybutanoat veresterter Bernsteinsäure erhalten.

Als Nebenprodukte werden unter anderem sehr geringe Mengen von Diglycerinestern von 3-Hydroxybuttersäure und Diglycerinestern von Bernsteinsäure gebildet.

Die Charakterisierung erfolgt mittels Massenspektrometrie (MS), Gelpermeationschromatographie (GPC) und Protonenresonanzspektroskopie (¹H-NMR).

### I. 3. Herstellung von Diglycerinestern von mit Ethyl-3-hydroxybutanoat veresterter Bernsteinsäure unter Verwendung von Bernsteinsäurediethylester

In einem 500-ml-Mehrhalskolben mit Dephlegmator (Partialkondensator) und Destillationsbrücke werden 132 g (R)/(S)-3-Hydroxybuttersäureethylester (3-BHB-EE = Ethyl-3-hydroxybutanoat oder 4-Ethoxy-4-oxobutan-2-ol) (racemisch), 174 g Bernsteinsäurediethylester und 40 g Diglycerin vorgelegt. Das Reaktionsgemisch wird unter Rühren bei 120 °C und unter N₂ für 7 h zur Reaktion gebracht und das entstehende Ethanol wird kontinuierlich destillativ entfernt. Anschließend werden überschüssiger 3-Hydroxybuttersäureethylester sowie überschüssiger Bernsteinsäureester von Ethyl-3-hydroxybutanoat unter Vakuum destillativ entfernt. Der erhaltene Rückstand wird bei Bedarf für 2 bis 4 Stunden im Vakuum mit Wasserdampf behandelt.

Es wird ein Gemisch aus ein-, zwei-, drei- und vierwertigen Diglycerinestern (d. h. Diglycerinmonoester, Diglycerindiester, Diglycerintriester und Diglycerintetraester) von mit Ethyl-3-hydroxybutanoat veresterter Bernsteinsäure erhalten.

Als Nebenprodukte werden unter anderem sehr geringe Mengen von Diglycerinestern von 3-Hydroxybuttersäure und Diglycerinestern von Bernsteinsäure gebildet.

Die Charakterisierung erfolgt mittels Massenspektrometrie (MS), Gelpermeationschromatographie (GPC) und Protonenresonanzspektroskopie (¹H-NMR).

### I. 4. Herstellung weiterer Diglycerinestern von mit Ethyl-3-hydroxybutanoat veresterter Polycarbonsäuren

Die zuvor durchgeführten Synthesebeispiele gemäß I.1., I.2. und I.3. werden erneut durchgeführt, jedoch unter Verwendung der folgenden Polycarbonsäuren (erste Verfahrensvariante jeweils als freie Säuren) und deren jeweiligen Anhydriden (zweite Verfahrensvariante) sowie deren jeweiligen Ethylester (dritte Verfahrensvariante): Weinsäure, Citronensäure, Äpfelsäure, Adpinsäure, Fumarsäure und Maleinsäure. Es werden jeweils vergleichbare Ergebnisse erzielt.

### II. Herstellungsbeispiele einer besonderen Ausführungsform des erfindungsgemäßen Verfahrens als zweistufige Synthese ohne die Verwendung eines Katalysators

### II. 1. Herstellung von Diglycerinestern von mit Ethyl-3-hydroxybutanoat veresterter Bernsteinsäure unter Verwendung von Bernsteinsäureanhydrid

In einem 500-ml-Mehrhalskolben mit Dephlegmator (Partialkondensator) und Destillationsbrücke werden 132 g (R)/(S)-3-Hydroxybuttersäureethylester (3-BHB-EE = Ethyl-3-hydroxybutanoat oder 4-Ethoxy-4-oxobutan-2-ol) (racemisch) und 100 g Bernsteinsäureanhydrid vorgelegt. Das Reaktionsgemisch wird unter Rühren bei 120 °C und unter N₂ für 7 h zur Reaktion gebracht. Nach Ablauf der Reaktionszeit wird eine Probe zur Analyse genommen. Anschließend wird 40 g Diglycerin zugegeben und das Reaktionsgemisch wird unter Rühren bei 120 °C und unter N₂ für weitere 7 h zur Reaktion gebracht und das entstehende Reaktionswasser wird kontinuierlich destillativ entfernt. Nach Ablauf der Reaktionszeit wird eine weitere Probe zur Analyse genommen.

Anschließend werden überschüssiger 3-Hydroxybuttersäureethylester sowie überschüssiger Bernsteinsäureester von Ethyl-3-hydroxybutanoat unter Vakuum destillativ entfernt. Der erhaltene Rückstand wird bei Bedarf für 2 bis 4 Stunden im Vakuum mit Wasserdampf behandelt.

Es wird wie im Fall der einstufigen Synthese ein Gemisch aus ein-, zwei-, drei- und vierwertigen Diglycerinestern (= Estern des Dyglycerins) (d. h. Diglycerinmonoester, Diglycerindiester, Diglycerintriester und Diglycerintetraester) von mit Ethyl-3-hydroxybutanoat veresterter Bernsteinsäure erhalten. Als Nebenprodukte werden unter anderem sehr geringe Mengen von Diglycerinestern von 3-Hydroxybuttersäure und Diglycerinestern von Bernsteinsäure gebildet.

Die Charakterisierung erfolgt mittels Massenspektrometrie (MS), Gelpermeationschromatographie (GPC) und Protonenresonanzspektroskopie (¹H-NMR).

Die jeweils nach Beendigung des ersten und des zweiten Reaktionsschrittes genommenen Proben werden mittels GC (Gaschromatographie) analysiert; die GC-Flächenanalysen sind in Tabelle 1 zusammengefasst.

**Tabelle 1: GC-Flächenanalyse [%] der Umsetzung von Bernsteinsäureanhydrid, Ethyl-3-hydroxybutanoat und Diglycerin**

| | **nach dem ersten Verfahrensschritt** | **nach dem zweiten Verfahrensschritt** |
|---|---|---|
| **Bernsteinsäureanhydrid** | 3,1 | 0,1 |
| **Bernsteinsäure** | 2,2 | 1,1 |
| **3-BHB-EE** | 19,2 | 7,5 |
| **BS-(BHB-EE)-Monoester** | 65,2 | 44,9 |
| **BS-(BHB-EE)-Diester** | 1,3 | 3,6 |
| **Diglycerin** | - | 7,6 |
| **PG(2)-(BS-(BHB-EE))-Monoester** | - | 12,6 |
| **PG(2)-(BS-(BHB-EE))-Monoester** | - | 6,7 |
| **PG(2)-(BS-(BHB-EE))-Monoester** | - | 6,2 |
| **Nebenprodukte** * | 0,8 | 6,5 |
| **Unbekannt** | 8,2 | 3,2 |

| | | |
|---|---|---|
| * u. a. Monoethylsuccinat, BHB-Dimer, BHB-Diglycerinester, Diglycerinestern von 3-Hydroxybuttersäure und Diglycerinestern von Bernsteinsäure | | |

Im Folgenden ist der Reaktionsverlauf schematisch dargestellt (wobei im Fall der Nebenprodukte freie Hydroxylgruppen gegebenenfalls auch, ganz oder teilweise durch einen Rest - C(O) - CH₂ - CH(OH) - CH₃ (Veresterung mit Ethyl-3-hydroxybutanoat) oder einen Rest - C(O) - CH₂ - CH₂ - COOH (Veresterung mit Bernsteinsäureanhydrid) verestert sein können):

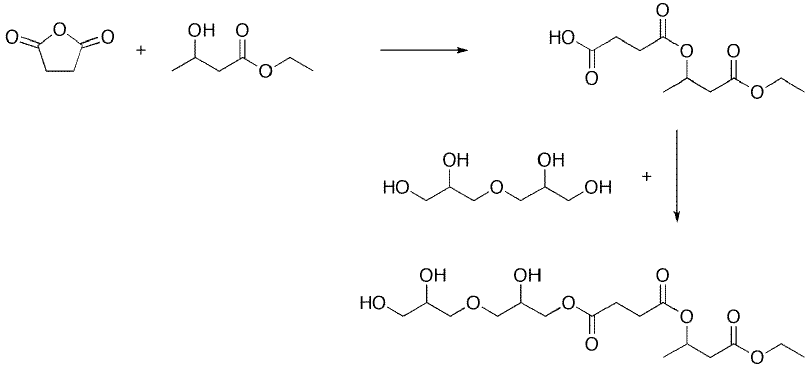

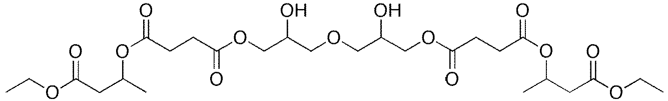

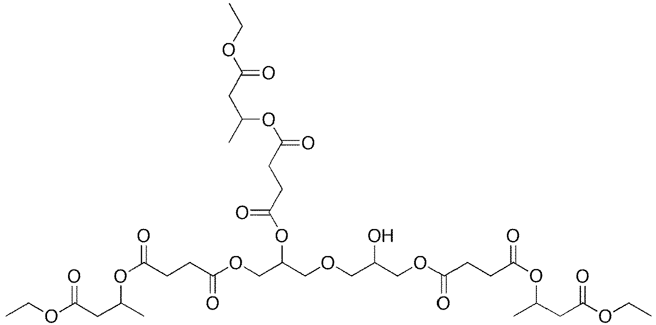

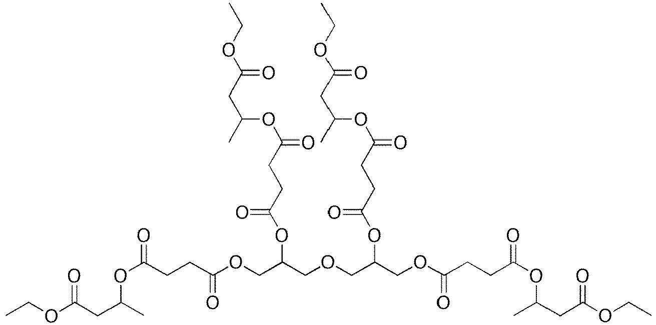

### II. 2. Herstellung von Diglycerinestern von mit Ethyl-3-hydroxybutanoat veresterter Bernsteinsäure unter Verwendung der freien Bernsteinsäure

In einem 500-ml-Mehrhalskolben mit Dephlegmator (Partialkondensator) und Destillationsbrücke werden 132 g (R)/(S)-3-Hydroxybuttersäureethylester (3-BHB-EE = Ethyl-3-hydroxybutanoat oder 4-Ethoxy-4-oxobutan-2-ol) (racemisch) und 118 g Bernsteinsäure vorgelegt. Das Reaktionsgemisch wird unter Rühren bei 120 °C und unter N₂ für 7 h zur Reaktion gebracht und das entstehende Reaktionswasser wird kontinuierlich destillativ entfernt. Anschließend wird 40 g Diglycerin zugegeben und das Reaktionsgemisch wird unter Rühren bei 120 °C und unter N₂ für weitere 7 h zur Reaktion gebracht und das entstehende Reaktionswasser wird kontinuierlich destillativ entfernt.

Anschließend werden überschüssiger 3-Hydroxybuttersäureethylester sowie überschüssiger Bernsteinsäureester von Ethyl-3-hydroxybutanoat unter Vakuum destillativ entfernt. Der erhaltene Rückstand wird bei Bedarf für 2 bis 4 Stunden im Vakuum mit Wasserdampf behandelt.

Es wird ein Gemisch aus ein-, zwei-, drei- und vierwertigen Diglycerinestern (d. h. Diglycerinmonoester, Diglycerindiester, Diglycerintriester und Diglycerintetraester) von mit Ethyl-3-hydroxybutanoat veresterter Bernsteinsäure erhalten.

Als Nebenprodukte werden unter anderem sehr geringe Mengen von Diglycerinestern von 3-Hydroxybuttersäure und Diglycerinestern von Bernsteinsäure gebildet.

Die Charakterisierung erfolgt mittels Massenspektrometrie (MS), Gelpermeationschromatographie (GPC) und Protonenresonanzspektroskopie (¹H-NMR).

### II. 3. Herstellung von Diglycerinestern von mit Ethyl-3-hydroxybutanoat veresterter Bernsteinsäure unter Verwendung von Bernsteinsäurediethylester

In einem 500-ml-Mehrhalskolben mit Dephlegmator (Partialkondensator) und Destillationsbrücke werden 132 g (R)/(S)-3-Hydroxybuttersäureethylester (3-BHB-EE = Ethyl-3-hydroxybutanoat oder 4-Ethoxy-4-oxobutan-2-ol) (racemisch) und 174 g Bernsteinsäurediethylester vorgelegt. Das Reaktionsgemisch wird unter Rühren bei 120 °C und unter N₂ für 7 h zur Reaktion gebracht und das entstehende Ethanol wird kontinuierlich destillativ entfernt. Anschließend wird 40 g Diglycerin zugegeben und das Reaktionsgemisch wird unter Rühren bei 120 °C und unter N₂ für weitere 7 h zur Reaktion gebracht und das entstehende Ethanol wird kontinuierlich destillativ entfernt.

Anschließend werden überschüssiger 3-Hydroxybuttersäureethylester sowie überschüssiger Bernsteinsäureester von Ethyl-3-hydroxybutanoat unter Vakuum destillativ entfernt. Der erhaltene Rückstand wird bei Bedarf für 2 bis 4 Stunden im Vakuum mit Wasserdampf behandelt.

Es wird ein Gemisch aus ein-, zwei-, drei- und vierwertigen Diglycerinestern (d. h. Diglycerinmonoester, Diglycerindiester, Diglycerintriester und Diglycerintetraester) von mit Ethyl-3-hydroxybutanoat veresterter Bernsteinsäure erhalten.

Als Nebenprodukte werden unter anderem sehr geringe Mengen von Diglycerinestern von 3-Hydroxybuttersäure und Diglycerinestern von Bernsteinsäure gebildet.

Die Charakterisierung erfolgt mittels Massenspektrometrie (MS), Gelpermeationschromatographie (GPC) und Protonenresonanzspektroskopie (¹H-NMR).

### II. 4. Herstellung weiterer Diglycerinestern von mit Ethyl-3-hydroxybutanoat veresterter Polycarbonsäuren

Die zuvor durchgeführten Synthesebeispiele gemäß II.1., II.2. und II.3. werden erneut durchgeführt, jedoch unter Verwendung der folgenden Polycarbonsäuren und deren jeweiligen Anhydriden sowie Ethylester: Weinsäure, Citronensäure, Äpfelsäure, Adpinsäure, Fumarsäure und Maleinsäure. Es werden vergleichbare Ergebnisse erzielt.

### III. Herstellungsbeispiele einer alternativen besonderen Ausführungsform des erfindungsgemäßen Verfahrens unter Verwendung eines Metallkatalysators

Alle zuvor unter Abschnitt I. und Abschnitt II. beschriebenen chemischen Synthesebeispiele werden erneut durchgeführt, jedoch unter Zugabe von Titantetrabutylat als Katalysator (Titan(IV)-Katalysator). Der Titan(IV)-Katalysator wird zusammen mit den weiteren Reaktanden im Kolben vorgelegt. Im Anschluss entspricht der Reaktionsverlauf den zuvor beschriebenen Beispielen. Es werden vergleichbare Ergebnisse erzielt. Der Katalysator wird nach Reaktionsende abgetrennt und rezykliert.

### IV. Herstellungsbeispiele einer weiteren alternativen besonderen Ausführungsform des erfindungsgemäßen Verfahrens unter Verwendung eines Enzymkatalysators

Alle zuvor unter Abschnitt I. und Abschnitt **II.** beschriebenen chemischen Synthesebeispiele werden erneut durchgeführt, jedoch unter Zugabe eines immobilisierten Enzyms (CALB-Lipase auf Polymerträger, abgeleitet von *Candida antarctica,* z. B. Novozym^{®}435 von der Fa. Sigma-Aldrich bzw. Merck oder Lipozym^{®}435 von der Fa. Strem Chemicals, Inc.) als Katalysators und bei 70 °C unter Vakuum. Es werden vergleichbare Ergebnisse erzielt. Das Enzym wird nach Reaktionsende abgetrennt und rezykliert.

### V. Physiologische Anwendungsversuche: in-vitro-Verdauversuche

### V. 1. Verdauversuche (Spalt- bzw. Spaltungsversuche) von erfindungsgemäßen Carbonsäureestern von 3-Hydroxybutanoat

Mittels Spaltungsversuchen wird gezeigt, dass erfindungsgemäß hergestellte Polyglycerinester von mit Ethyl-3-Hydroxybutanoat veresterten Polycarbonsäuren bzw. deren Gemische (vgl. zuvor beschriebene Versuche gemäß I., II. III. und IV.), einschließlich der Reaktionsnebenprodukte, im menschlichen gastrointestinalen Trakt gespalten werden können.

Als Ausgangsgemisch werden jeweils aufgereinigte, nach dem erfindungsgemäßen Verfahren erhaltene Reaktionsprodukte eingesetzt:
- Diglycerinestergemisch von mit Ethyl-3-hydroxybutanoat veresterter Weinsäure,
- Diglycerinestergemisch von mit Ethyl-3-hydroxybutanoat veresterter Citronensäure,
- Diglycerinestergemisch von mit Ethyl-3-hydroxybutanoat veresterter Äpfelsäure,
- Diglycerinestergemisch von mit Ethyl-3-hydroxybutanoat veresterter Adpinsäure,
- Diglycerinestergemisch von mit Ethyl-3-hydroxybutanoat veresterter Fumarsäure und
- Diglycerinestergemisch von mit Ethyl-3-hydroxybutanoat veresterter Maleinsäure.

Für die Spaltungsversuche unter körpernahen Bedingungen werden zwei Medien untersucht:
- FaSSGF, welches den Magen simuliert
- FaSSIF, welches den Darmtrakt simuliert.

Beide Medien stammen von der Firma Biorelevant^{®}, Ltd., Großbritannien. Zusätzlich wird in einigen Experimenten beiden Medien jeweils Schweine-Pankrease zugesetzt (Panzytrat^{®}40.000, Fa. Allergan).

Die Ergebnisse der Spaltungsversuche in einem FaSSGF- bzw. FaSSIF-Medium mit Panzytrat^{®} und ohne Panzytrat^{®} (jeweils 35 °C, 24 h) zeigen, dass die Proben unter FaSSGF-Bedingungen mit Panzytrat^{®} und ohne Panzytrat^{®} hydrolysieren; dies liegt hauptsächlich am niedrigen pH-Wert (pH = 1,6) des Mediums. Bei FaSSIF-Bedingungen findet eine geringere Umsetzung unter Verwendung von Panzytrat^{®} statt.

Bei allen Spaltungsversuchen ist zu erkennen, dass die Spaltung in Form einer Kaskade verläuft (d. h. der Diglycerintetraester wird zum Diglycerintriester, der Diglycerintriester wird zum Diglycerindiester etc.). Weiterhin wird das Ethyl-3-hydroxybutanoat von der Polycarbonsäure abgespalten und anschließend in die freie Buttersäure und Ethanol gespalten. Insgesamt werden somit die vom Körper verwertbaren Polycarbonsäuren und die freie Buttersäure freigesetzt und weiterhin wird der untoxische, physiologisch kompatible Träger Diglycerin freigesetzt, welcher vom Körper ausgeschieden wird.

Insgesamt liegt somit ein Retardierungseffekt vor (d. h. die Freisetzung der 3-Hydroxybuttersäure und der Polycarbonsäure erfolgen verzögert bzw. kontinuierlich über einen längeren Zeitraum).

### V. 2. Weitere Verdauversuche (Spaltungsversuche) von erfindungsgemäßen Carbonsäuren von 3-Hydroxybutanoat

### Spaltunqsversuche mit Pankreatin

2 g eines wie zuvor beschrieben hergestellten Diglycerinester von mit Ethyl-3-hydroxybutanoat veresterten Polycarbonsäuren bzw. deren Mischung (Mischung aus entsprechenden Diglycerinmono-, Diglycerindi-, Diglycerintri- und/oder Diglycerintetraester von mit 3-Hydroxybutanoat veresterten Polycarbonsäuren) werden in 50 g Wasser gelöst und mit 0,5 g (1 Gew.-%) Pankreatin versetzt. Das Pankreatin wird in Form des kommerziell verfügbaren Produkts Panzytrat^{®} 40.000 von der Fa. Allergan eingesetzt. Das Ganze wird auf einer Heizplatte bei 50 °C gerührt; der Reaktionsverlauf wird mittels kontinuierlicher Erfassung der Säurezahl über die Zeit ermittelt und verfolgt. Die Säurezahl steigt über den Beobachtungszeitraum an (Spaltung der Diglycerinester von mit 3-Hydroxybutanoat veresterten Polycarbonsäuren). Der Umsatz/Zeit-Verlauf der wässrigen Spaltung der erfindungsgemäßen Diglycerinester von mit 3-Hydroxybutanoat veresterten Polycarbonsäuren mittels Pankreatin, einschließlich Zunahme der Säurezahl über die Zeit, belegt die gewünschte Zersetzung des Eduktgemischs zu der freien Polycarbonsäure und der freien 3-Hydroxybuttersäure. Dies wird durch entsprechende Analytik bestätigt. Der Versuch belegt, dass das erfindungsgemäße Ausgangsgemisch ein geeigneter physiologischer Präkursor für 3-Hydroxybuttersäure bzw. deren Ester (3-Hydroxybutanoate) für die entsprechenden Ketokörpertherapien darstellt.

### V. 3. Schlussfolgerungen

Die zuvor geschilderten Spaltungsversuche belegen, dass die Diglycerinester von mit 3-Hydroxybutanoat veresterten Polycarbonsäuren effiziente Präkursoren bzw. Metabolite der freien Hydroxybuttersäure bzw. deren Estern (hier: Ethylester) darstellen, insbesondere im Hinblick auf ihre beabsichtigte Wirkung, welche in physiologisch verträglicher bzw. physiologisch kompatibler Form vorliegen. Gleichermaßen werden ebenfalls metabolisch verwertbare bzw. umsetzbare, im natürlichen Stoffwechsel (z. B. Citratzyklus) vorkommende Polycarbonsäuren bzw. deren Derivate, insbesondere Salze, gebildet (z. B. Citronensäure bzw. Citrate, Äpfelsäure bzw. Malate, Weinsäure bzw. Tartrate etc.). Das Diglycerin dient als physiologisch kompatibles, nicht-toxisches Träger- bzw. Carriermolekül für die Anbindung einer Vielzahl an Wirkstoffmolekülen (= mit 3-Hydroxybutanoat veresterte Polycarbonsäuren), welches aber selbst ohne Weiteres ausgeschieden werden kann; es resultiert eine hohe Wirkstoffdichte einerseits und eine gewünschte kontrollierte, insbesondere retardierte Freisetzung andererseits.

### VI. Weitere Testungen (Oraanoleptik und Toxizität)

Weitere Versuche und Testreihen werden in Bezug auf Organoleptik und Toxizität der erfindungsgemäßen Diglycerinester von mit 3-Hydroxybutanoat veresterten Polycarbonsäuren durchgeführt. Diese zeigen, dass die erfindungsgemäßen Diglycerinester von mit 3-Hydroxybutanoat veresterten Polycarbonsäuren organoleptisch akzeptabel und kompatibel sind, insbesondere im Vergleich zu reiner 3-Hydroxybuttersäure sowie deren Salzen und Estern signifikant verbesserte organoleptische Eigenschaften aufweisen, sowie zudem keine für die Anwendung entgegenstehende Toxizität aufweisen.

## Patentansprüche

1. Verfahren zur Herstellung von Polyglycerinestern von mit Oxobutanol veresterten Polycarbonsäuren, insbesondere ein- oder mehrwertigen Polyglycerinestern von mit 4-Oxo-4-(C₁-C₅-alkoxy)-2-butanol oder mit 4-Oxo-4-(hydroxy-C₃-C₅-alkoxy)-2-butanol veresterten Polycarbonsäuren,
wobei
(i) mindestens ein Oxobutanol, insbesondere ein 3-Hydroxybutanoat, bevorzugt ein 4-Oxo-2-butanol, vorzugsweise ein 4-Oxo-4-(C₁-C₅-alkoxy)-2-butanol oder ein 4-Oxo-4-(hydroxy-C₃-C₅-alkoxy)-2-butanol, der allgemeinen Formel (I)
CH₃-CH(OH)-CH₂-C(O)OR¹ (I)
wobei in der allgemeinen Formel (I) der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt,
(ii) mindestens eine Polycarbonsäure (II), insbesondere mindestens eine mindestens zwei Carboxylgruppen enthaltende Polycarbonsäure, wobei die Polycarbonsäure (II) in Form der freien Polycarbonsäure, in Form eines Salzes der Polycarbonsäure, in Form eines Polycarbonsäureesters oder in Form des Polycarbonsäureanhydrids eingesetzt wird, und
(iii) mindestens ein Polyglycerin (III)
miteinander umgesetzt und/oder zur Reaktion gebracht werden,
wobei das Verfahren einstufig oder aber mehrstufig durchgeführt wird, und
wobei das Verfahren in Abwesenheit von Lösemitteln und/oder ohne jedwedes Lösemittel durchgeführt wird, und
wobei bei Verwendung der Polycarbonsäure (II) in Form der freien Säure bei der Umsetzung gleichzeitig Wasser gebildet wird, wobei das Wasser der Umsetzung kontinuierlich entzogen wird; und/oder
wobei bei Verwendung der Polycarbonsäure (II) in Form des Anhydrids die entsprechende freie Polycarbonsäure (II) und Wasser gebildet werden, wobei die entstehende freie Polycarbonsäure (II) weiter umgesetzt wird oder nach erfolgter Reaktion entfernt und gegebenenfalls rezykliert wird und wobei das Wasser der Umsetzung kontinuierlich entzogen wird; und/oder
wobei bei Verwendung der Polycarbonsäure (II) in Form des Esters der entsprechende Esteralkohol und Wasser gebildet werden, wobei der entstehende Esteralkohol und Wasser der Umsetzung kontinuierlich entzogen werden,
so dass als Reaktionsprodukt (IV) ein oder mehrere Polyglycerinester von mit Oxobutanol veresterten Polycarbonsäuren, insbesondere ein oder mehrere Polyglycerinester von mit 4-Oxo-2-butanol veresterten Polycarbonsäuren, vorzugsweise ein oder mehrere ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, erhalten werden.

2. Verfahren nach Anspruch 1,
wobei das Verfahren mehrstufig, insbesondere zweistufig, durchgeführt wird,
wobei
(a) in einem ersten Verfahrensschritt (a) mindestens ein Oxobutanol, insbesondere ein 3-Hydroxybutanoat, bevorzugt ein 4-Oxo-2-butanol, vorzugsweise ein 4-Oxo-4-(C₁-C₅-alkoxy)-2-butanol oder ein 4-Oxo-4-(hydroxy-C₃-C₅-alkoxy)-2-butanol, der allgemeinen Formel (I)
CH₃-CH(OH)-CH₂-C(O)OR¹ (I)
wobei in der allgemeinen Formel (I) der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt,
mit mindestens einer Polycarbonsäure (II), insbesondere mit mindestens einer mindestens zwei Carboxylgruppen enthaltenden Polycarbonsäure, umgesetzt und/oder zur Reaktion gebracht wird, insbesondere in einer Veresterungsreaktion und/oder unter Veresterungsbedingungen umgesetzt und/oder zur Reaktion gebracht wird,
insbesondere sodass als Reaktionszwischenprodukt (IV') des Verfahrensschritts (a) ein oder mehrere Polycarbonsäureester von Oxobutanol, insbesondere ein oder mehrere Polycarbonsäureester von 3-Hydroxybutanoat, bevorzugt ein oder mehrere Polycarbonsäureester von 4-Oxo-2-butanol, vorzugsweise ein oder mehrere Polycarbonsäureester von 4-Oxo-4-(C₁-C₅-alkoxy)-2-butanol oder von 4-Oxo-4-(hydroxy-C₃-C₅-alkoxy)-2-butanol, erhalten werden; und
(b) nachfolgend in einem zweiten Verfahrensschritt (b) das in Verfahrensschritt (a) erhaltene Reaktionszwischenprodukt (IV') mit mindestens einem Polyglycerin (III) umgesetzt und/oder zur Reaktion gebracht wird, insbesondere in einer Veresterungsreaktion und/oder unter Veresterungsbedingungen umgesetzt und/oder zur Reaktion gebracht wird.

3. Verfahren nach einem der vorangehenden Ansprüche,
wobei die Polycarbonsäure (II) ausgewählt ist aus der Gruppe von Bernsteinsäure, Weinsäure, Citronensäure, Äpfelsäure, Adipinsäure, Fumarsäure und Maleinsäure und deren Anhydriden sowie deren Kombinationen oder Mischungen, insbesondere ausgewählt ist aus der Gruppe von Bernsteinsäure, Weinsäure, Citronensäure, Äpfelsäure, Adipinsäure und Fumarsäure und deren Anhydriden sowie deren Kombinationen oder Mischungen, bevorzugt ausgewählt ist aus der Gruppe von Bernsteinsäure und Adipinsäure und deren Anhydriden sowie deren Kombinationen oder Mischungen; und/oder
wobei die Polycarbonsäure (II) eine natürlich vorkommende Carbonsäure oder deren Anhydrid oder Derivat, insbesondere Umsetzungsprodukt, ist, insbesondere eine im menschlichen und/oder tierischen Stoffwechsel vorkommende Carbonsäure oder deren Anhydrid oder Derivat, insbesondere Umsetzungsprodukt; und/oder
wobei die Polycarbonsäure (II) ein lebensmittelrechtlich zugelassener Inhaltsstoff, insbesondere Zusatzstoff, ist.

4. Verfahren nach einem der vorangehenden Ansprüche,
wobei das Polyglycerin der allgemeinen Formel (Illa)
HO-CH₂-CH(OH)-CH₂-[O-CH₂-CH(OH)-CH₂]ₚ-OH (IIIa)
entspricht, wobei in der allgemeinen Formel (IIIa) die Variable p eine ganze Zahl von 1 bis 6, insbesondere von 1 bis 4, vorzugsweise 1 oder 2, besonders bevorzugt 1, darstellt; und/oder
wobei das Polyglycerin (III) ein Diglycerin der Formel (IIIb)
HO-CH₂-CH(OH)-CH₂-O-CH₂-CH(OH)-CH₂-OH (IIIb)
ist; und/oder
wobei das Polyglycerin (III) kein Propan-1,2,3-triol (Glycerin) ist.

5. Verfahren nach einem der vorangehenden Ansprüche,
wobei im Reaktionsprodukt nach erfolgter Umsetzung noch vorhandene Hydroxylgruppen und/oder Carboxylgruppen zumindest teilweise, vorzugsweise vollständig, funktionalisiert, insbesondere verestert werden; und/oder
wobei sich der Umsetzung eine teilweise, insbesondere vollständige Funktionalisierung, insbesondere Veresterung, noch vorhandener Hydroxylgruppen und/oder Carboxylgruppen anschließt.

6. Reaktionszwischenprodukt in Form eines Polycarbonsäureesters von Oxobutanol, insbesondere Polycarbonsäureesters von 3-Hydroxybutanoat, bevorzugt Polycarbonsäureesters von 4-Oxo-2-butanol, vorzugsweise Polycarbonsäureesters von 4-Oxo-4-(C₁-C₅-alkoxy)-2-butanol oder von 4-Oxo-4-(hydroxy-C₃-C₅-alkoxy)-2-butanol, erhältlich nach einem Verfahren gemäß einem der vorangehenden Ansprüche, insbesondere erhältlich als Reaktionszwischenprodukt (IV') des ersten Verfahrensschritts (a) des Verfahrens gemäß einem der vorangehenden Ansprüche.

7. Reaktionsprodukt in Form eines Polyglycerinesters von mit Oxobutanol veresterten Polycarbonsäuren, insbesondere Polyglycerinesters von mit 4-Oxo-2-butanol veresterten Polycarbonsäuren, vorzugsweise ein- oder mehrwertigen Polyglycerinesters von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, erhältlich nach einem Verfahren gemäß einem der vorangehenden Ansprüche.

8. Polyglycerinester von mit Oxobutanol veresterten Polycarbonsäuren,
wobei der Polyglycerinester von mit Oxobutanol veresterten Polycarbonsäuren, insbesondere Polyglycerinester von mit 4-Oxo-2-butanol veresterten Polycarbonsäuren, vorzugsweise ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, der allgemeinen Formel (IVa)
R⁴O-CH₂-CH(OR⁴)-CH₂-[O-CH₂-CH(OR⁴)-CH₂]ₚ-OR⁴ (IVa)
entspricht,
wobei in der allgemeinen Formel (IVa) der Rest R⁴, unabhängig voneinander, darstellt:
• Wasserstoff;
• einen Rest R¹ - O - C(O) - CH₂ - CH(CH₃) - O - (O)C - X - C(O) -, wobei der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und wobei X einen 1 bis 10, vorzugsweise 2 bis 6 Kohlenstoffatome enthaltenden und gegebenenfalls ein- oder mehrfach substituierten, insbesondere mit einem oder mehreren Hydroxyl-Resten und/oder Resten - O - C(O) - CH₂ - CH(OH) - CH₃ und/oder Carboxyl-Resten und/oder Resten - C(O) - O - CH(CH₃) - CH₂ - C(O)OR¹ mit R¹ wie zuvor definiert substituierten, gesättigten oder ungesättigten organischen Rest darstellt;
• einen Rest H₃C - CH(OH) - CH₂ - C(O) -;
• einen Rest HOOC - X - C(O) -, wobei X einen 1 bis 10, vorzugsweise 2 bis 6 Kohlenstoffatome enthaltenden und gegebenenfalls ein- oder mehrfach substituierten, insbesondere mit einem oder mehreren Hydroxyl-Resten und/oder Resten - O - C(O) - CH₂ - CH(OH) - CH₃ und/oder Carboxyl-Resten und/oder Resten - C(O) - O - CH(CH₃) - CH₂ - C(O)OR¹ mit R¹ wie zuvor definiert substituierten, gesättigten oder ungesättigten organischen Rest darstellt;
jedoch mit der Maßgabe, dass mindestens ein Rest R⁴, insbesondere mindestens zwei Reste R⁴, einen Rest R¹ - O - C(O) - CH₂ - CH(CH₃) - O - (O)C - X - C(O) -, wie zuvor definiert, darstellt; und
wobei in der allgemeinen Formel (IVa) die Variable p eine ganze Zahl von 1 bis 6, insbesondere von 1 bis 4, vorzugsweise 1 oder 2, besonders bevorzugt 1, darstellt.

9. Polyglycerinester von mit Oxobutanol veresterten Polycarbonsäuren nach Anspruch 7 oder 8,
wobei der Polyglycerinester von mit Oxobutanol veresterten Polycarbonsäuren, insbesondere Polyglycerinester von mit 4-Oxo-2-butanol veresterten Polycarbonsäuren, vorzugsweise ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, der allgemeinen Formel (IVa)
R⁴O-CH₂-CH(OR⁴)-CH₂-[O-CH₂-CH(OR⁴)-CH₂]ₚ-OR⁴ (IVa)
entspricht,
wobei in der allgemeinen Formel (IVa) der Rest R⁴, unabhängig voneinander, darstellt:
• Wasserstoff;
• einen Rest R¹ - O - C(O) - CH₂ - CH(CH₃) - O - (O)C - X - C(O) -, wobei der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und wobei X einen 1 bis 10, vorzugsweise 2 bis 6 Kohlenstoffatome enthaltenden und gegebenenfalls ein- oder mehrfach substituierten, insbesondere mit einem oder mehreren Hydroxyl-Resten und/oder Resten - O - C(O) - CH₂ - CH(OH) - CH₃ und/oder Carboxyl-Resten und/oder Resten - C(O) - O - CH(CH₃) - CH₂ - C(O)OR¹ mit R¹ wie zuvor definiert substituierten, gesättigten oder ungesättigten organischen Rest darstellt;
jedoch mit der Maßgabe, dass mindestens ein Rest R⁴, insbesondere mindestens zwei Reste R⁴, einen Rest R¹ - O - C(O) - CH₂ - CH(CH₃) - O - (O)C - X - C(O) -, wie zuvor definiert, darstellt; und
wobei in der allgemeinen Formel (IVa) die Variable p eine ganze Zahl von 1 bis 6, insbesondere von 1 bis 4, vorzugsweise 1 oder 2, besonders bevorzugt 1, darstellt.

10. Polyglycerinester von mit Oxobutanol veresterten Polycarbonsäuren nach einem der Ansprüche 7 bis 9,
wobei der Polyglycerinester von mit Oxobutanol veresterten Polycarbonsäuren, insbesondere Polyglycerinester von mit 4-Oxo-2-butanol veresterten Polycarbonsäuren, vorzugsweise ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, der allgemeinen Formel (IVa)
R⁴O-CH₂-CH(OR⁴)-CH₂-[O-CH₂-CH(OR⁴)-CH₂]ₚ-OR⁴ (IVa)
entspricht,
wobei in der allgemeinen Formel (IVa) der Rest R⁴, unabhängig voneinander, darstellt:
• Wasserstoff;
• einen Rest R¹ - O - C(O) - CH₂ - CH(CH₃) - O - (O)C - X - C(O) -, wobei der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und wobei X einen 1 bis 10, vorzugsweise 2 bis 6 Kohlenstoffatome enthaltenden und gegebenenfalls ein- oder mehrfach substituierten, insbesondere mit einem oder mehreren Hydroxyl-Resten und/oder Carboxyl-Resten substituierten, gesättigten oder ungesättigten organischen Rest darstellt;
jedoch mit der Maßgabe, dass mindestens ein Rest R⁴, insbesondere mindestens zwei Reste R⁴, einen Rest R¹ - O - C(O) - CH₂ - CH(CH₃) - O - (O)C - X - C(O) -, wie zuvor definiert, darstellt; und
wobei in der allgemeinen Formel (IVa) die Variable p eine ganze Zahl von 1 bis 6, insbesondere von 1 bis 4, vorzugsweise 1 oder 2, besonders bevorzugt 1, darstellt.

11. Polyglycerinester von mit Oxobutanol veresterten Polycarbonsäuren nach einem der Ansprüche 7 bis 10,
wobei der Polyglycerinester von mit Oxobutanol veresterten Polycarbonsäuren, insbesondere Polyglycerinester von mit 4-Oxo-2-butanol veresterten Polycarbonsäuren, vorzugsweise ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, der allgemeinen Formel (IVb)
R⁵O-CH₂-CH(OR⁵)-CH₂-[O-CH₂-CH(OR⁵)-CH₂]ₚ-OR⁵ (IVb)
entspricht,
wobei in der allgemeinen Formel (IVb) der Rest R⁵, unabhängig voneinander, darstellt:
• Wasserstoff;
• einen Rest R¹ - O - C(O) - CH₂ - CH(CH₃) - O - Y -, wobei der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und wobei Y abgeleitet ist von einer Polycarbonsäure, ausgewählt aus der Gruppe von Bernsteinsäure, Weinsäure, Citronensäure, Äpfelsäure, Adipinsäure, Fumarsäure und Maleinsäure sowie deren Kombinationen oder Mischungen, insbesondere ausgewählt aus der Gruppe von Bernsteinsäure, Weinsäure, Citronensäure, Äpfelsäure, Adipinsäure und Fumarsäure sowie deren Kombinationen oder Mischungen, bevorzugt ausgewählt aus der Gruppe von Bernsteinsäure und Adipinsäure und deren Anhydriden sowie deren Kombinationen oder Mischungen insbesondere wobei im Fall der Citronensäure die vorhandene weitere Carboxylgruppe mit einem Rest - CH(CH₃) - CH₂ - C(O)OR¹ mit R¹ wie zuvor definiert verestert ist;
• einen Rest H₃C - CH(OH) - CH₂ - C(O) -;
• einen Rest - Y - OH mit Y wie zuvor definiert;
jedoch mit der Maßgabe, dass mindestens ein Rest R⁵, insbesondere mindestens zwei Reste R⁵, einen Rest R¹ - O - C(O) - CH₂ - CH(CH₃) - O - Y - mit R¹ und Y jeweils wie zuvor definiert darstellt; und
wobei in der allgemeinen Formel (IVb) die Variable p eine ganze Zahl von 1 bis 6, insbesondere von 1 bis 4, vorzugsweise 1 oder 2, besonders bevorzugt 1, darstellt.

12. Polyglycerinester von mit Oxobutanol veresterten Polycarbonsäuren nach einem der Ansprüche 7 bis 11,
wobei der Polyglycerinester von mit Oxobutanol veresterten Polycarbonsäuren, insbesondere Polyglycerinester von mit 4-Oxo-2-butanol veresterten Polycarbonsäuren, vorzugsweise ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, der allgemeinen Formel (IVb)
R⁵O-CH₂-CH(OR⁵)-CH₂-[O-CH₂-CH(OR⁵)-CH₂]ₚ-OR⁵ (IVb)
entspricht,
wobei in der allgemeinen Formel (IVb) der Rest R⁵, unabhängig voneinander, darstellt:
• Wasserstoff;
• einen Rest R¹ - O - C(O) - CH₂ - CH(CH₃) - O - Y -, wobei der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und
wobei Y einen der folgenden Reste darstellt: 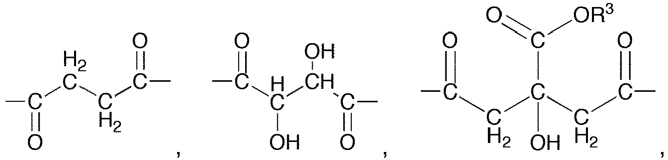 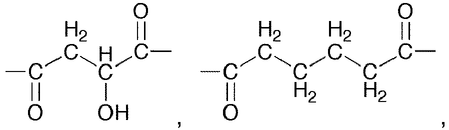 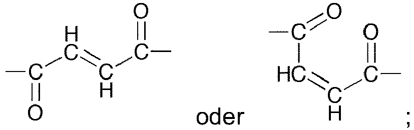 wobei in den vorstehenden Resten der Rest R³ Wasserstoff oder einen Rest - CH(CH₃) - CH₂ - C(O)OR¹ mit R¹ wie zuvor definiert darstellt;
• einen Rest H₃C - CH(OH) - CH₂ - C(O) -;
• einen Rest Y - OH mit Y wie zuvor definiert;
jedoch mit der Maßgabe, dass mindestens ein Rest R⁵, insbesondere mindestens zwei Reste R⁵, einen Rest R¹ - O - C(O) - CH₂ - CH(CH₃) - O - Y - mit R¹ und Y jeweils wie zuvor definiert darstellt; und
wobei in der allgemeinen Formel (IVb) die Variable p eine ganze Zahl von 1 bis 6, insbesondere von 1 bis 4, vorzugsweise 1 oder 2, besonders bevorzugt 1, darstellt.

13. Polyglycerinester von mit Oxobutanol veresterten Polycarbonsäuren nach einem der Ansprüche 7 bis 12,
wobei der Polyglycerinester von mit Oxobutanol veresterten Polycarbonsäuren, insbesondere Polyglycerinester von mit 4-Oxo-2-butanol veresterten Polycarbonsäuren, vorzugsweise ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, der allgemeinen Formel (IVc)
R⁴O-CH₂-CH(OR⁴)-CH₂-O-CH₂-CH(OR⁴)-CH₂-OR⁴ (IVc)
entspricht,
wobei in der allgemeinen Formel (IVc) der Rest R⁴, unabhängig voneinander, darstellt:
• Wasserstoff;
• einen Rest R¹ - O - C(O) - CH₂ - CH(CH₃) - O - (O)C - X - C(O) -, wobei der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und wobei X einen 1 bis 10, vorzugsweise 2 bis 6 Kohlenstoffatome enthaltenden und gegebenenfalls ein- oder mehrfach substituierten, insbesondere mit einem oder mehreren Hydroxyl-Resten und/oder Resten - O - C(O) - CH₂ - CH(OH) - CH₃ und/oder Carboxyl-Resten und/oder Resten - C(O) - O - CH(CH₃) - CH₂ - C(O)OR¹ mit R¹ wie zuvor definiert substituierten, gesättigten oder ungesättigten organischen Rest darstellt;
• einen Rest H₃C - CH(OH) - CH₂ - C(O) -;
• einen Rest HOOC - X - C(O) -, wobei X einen 1 bis 10, vorzugsweise 2 bis 6 Kohlenstoffatome enthaltenden und gegebenenfalls ein- oder mehrfach substituierten, insbesondere mit einem oder mehreren Hydroxyl-Resten und/oder Resten - O - C(O) - CH₂ - CH(OH) - CH₃ und/oder Carboxyl-Resten und/oder Resten - C(O) - O - CH(CH₃) - CH₂ - C(O)OR¹ mit R¹ wie zuvor definiert substituierten, gesättigten oder ungesättigten organischen Rest darstellt;
jedoch mit der Maßgabe, dass mindestens ein Rest R⁴, insbesondere mindestens zwei Reste R⁴, einen Rest R¹ - O - C(O) - CH₂ - CH(CH₃) - O - (O)C - X - C(O) -, wie zuvor definiert, darstellt.

14. Polyglycerinester von mit Oxobutanol veresterten Polycarbonsäuren nach einem der Ansprüche 7 bis 13,
wobei der Polyglycerinester von mit Oxobutanol veresterten Polycarbonsäuren, insbesondere Polyglycerinester von mit 4-Oxo-2-butanol veresterten Polycarbonsäuren, vorzugsweise ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, der allgemeinen Formel (IVd)
R⁵O-CH₂-CH(OR⁵)-CH₂-O-CH₂-CH(OR⁵)-CH₂-OR⁵ (IVd)
entspricht,
wobei in der allgemeinen Formel (IVd) der Rest R⁵, unabhängig voneinander, darstellt:
• Wasserstoff;
• einen Rest R¹ - O - C(O) - CH₂ - CH(CH₃) - O - Y -, wobei der Rest R¹ ein C₁-C₅-Alkyl oder ein Hydroxy-C₃-C₅-alkyl, insbesondere Ethyl, Butyl, Pentyl, Hydroxybutyl oder Hydroxypentyl, bevorzugt Ethyl, Hydroxybutyl oder Hydroxypentyl, besonders bevorzugt Ethyl, darstellt und wobei Y einen der folgenden Reste darstellt 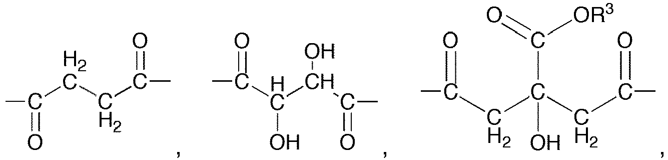 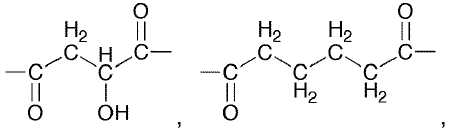 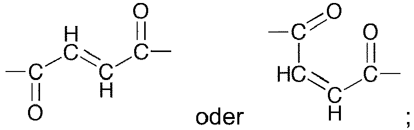 wobei in den vorstehenden Resten der Rest R³ Wasserstoff oder einen Rest - CH(CH₃) - CH₂ - C(O)OR¹ mit R¹ wie zuvor definiert darstellt;
• einen Rest H₃C - CH(OH) - CH₂ - C(O) -;
• einen Rest Y - OH mit Y wie zuvor definiert;
jedoch mit der Maßgabe, dass mindestens ein Rest R⁵, insbesondere mindestens zwei Reste R⁵, einen Rest R¹ - O - C(O) - CH₂ - CH(CH₃) - O - Y - mit R¹ und Y jeweils wie zuvor definiert darstellt.

15. Gemisch, umfassend mindestens zwei voneinander verschiedene Polyglycerinester von mit Oxobutanol veresterten Polycarbonsäuren, insbesondere Polyglycerinester von mit 4-Oxo-2-butanol veresterten Polycarbonsäuren, vorzugsweise ein- oder mehrwertige Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, insbesondere wie zuvor definiert.

16. Pharmazeutische Zusammensetzung, insbesondere Arzneimittel oder Medikament, umfassend ein Reaktionsprodukt bzw. einen Polyglycerinester von mit Oxobutanol veresterten Polycarbonsäuren, insbesondere einen Polyglycerinester von mit 4-Oxo-2-butanol veresterten Polycarbonsäuren, vorzugsweise einen ein- oder mehrwertigen Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, gemäß einem der Ansprüche 7 bis 14 und/oder ein Gemisch gemäß Anspruch 15.

17. Pharmazeutische Zusammensetzung nach Anspruch 16 zur Verwendung bei der prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

18. Reaktionsprodukt bzw. Polyglycerinester von mit Oxobutanol veresterten Polycarbonsäuren, insbesondere Polyglycerinester von mit 4-Oxo-2-butanol veresterten Polycarbonsäuren, vorzugsweise ein- oder mehrwertiger Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, gemäß einem der Ansprüche 7 bis 14 und/oder ein Gemisch gemäß Anspruch 15 zur Verwendung bei der prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glucosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

19. Nahrungsmittel- und/oder Lebensmittelerzeugnis, umfassend ein Reaktionsprodukt bzw. einen Polyglycerinester von mit Oxobutanol veresterten Polycarbonsäuren, insbesondere einen Polyglycerinester von mit 4-Oxo-2-butanol veresterten Polycarbonsäuren, vorzugsweise einen ein- oder mehrwertigen Polyglycerinester von mit 4-Oxo-4-(C₁-C₅-alkoxy)-2-butanol oder mit 4-Oxo-4-(hydroxy-C₃-C₅-alkoxy)-2-butanol veresterten Polycarbonsäuren, gemäß einem der Ansprüche 7 bis 14 und/oder ein Gemisch gemäß Anspruch 15.

## Claims

1. A method for producing polyglycerol esters of polycarboxylic acids esterified with oxobutanol, especially mono- or polyvalent polyglycerol esters of polycarboxylic acids esterified with 4-oxo-4-(C₁-C₅-alkoxy)-2-butanol or with 4-oxo-4-(hydroxy-C₃-C₅-alkoxy)-2-butanol,
wherein
(i) at least one oxobutanol, especially 3-hydroxybutanoate, preferentially 4-oxo-2-butanol, preferably 4-oxo-4-(C₁-C₅-alkoxy)-2-butanol or 4-oxo-4-(hydroxy-C₃-C₅-alkoxy)-2-butanol, of the general formula (I)
CH₃-CH(OH)-CH₂-C(O)OR¹ (I)
wherein, in the general formula (I), the radical R¹ represents C₁-C₅-alkyl or hydroxy-C₃-C₅-alkyl, especially ethyl, butyl, pentyl, hydroxybutyl or hydroxypentyl, preferably ethyl, hydroxybutyl or hydroxypentyl, more preferably ethyl,
(ii) at least one polycarboxylic acid (II), especially at least one polycarboxylic acid comprising at least two carboxyl groups, wherein the polycarboxylic acid (II) is used in the form of the free polycarboxylic acid, in the form of a salt of the polycarboxylic acid, in the form of a polycarboxylic acid ester or in the form of the polycarboxylic acid anhydride, and
(iii) at least one polyglycerol (III)
are reacted and/or caused to react with one another,
wherein the method is carried out in one step or else in several steps, and
wherein the method is carried out in the absence of solvents and/or without any solvent, and
wherein, when the polycarboxylic acid (II) is used in the form of the free acid, water is formed simultaneously during the reaction, wherein the water is continuously withdrawn from the reaction; and/or
wherein, when the polycarboxylic acid (II) is used in the form of the anhydride, the corresponding free polycarboxylic acid (II) and water are formed, wherein the resulting free polycarboxylic acid (II) is further reacted or, after the reaction has taken place, is removed and optionally recycled and wherein the water is continuously withdrawn from the reaction; and/or
wherein, when the polycarboxylic acid (II) is used in the form of the ester, the corresponding ester alcohol and water are formed, wherein the resulting ester alcohol and water are continuously withdrawn from the reaction,
so that, as a reaction product (IV), one or more polyglycerol esters of polycarboxylic acids esterified with oxobutanol, especially one or more polyglycerol esters of polycarboxylic acids esterified with 4-oxo-2-butanol, preferentially one or more mono- or polyvalent polyglycerol esters of polycarboxylic acids esterified with 4-oxo-4-(C₁-C₅-alkoxy)-2-butanol or with 4-oxo-4-(hydroxy-C₃-C₅-alkoxy)-2-butanol, are obtained.

2. The method according to claim 1,
wherein the method is carried out in several steps, especially in two steps,
wherein
(a) in a first method step (a), at least one oxobutanol, especially 3-hydroxybutanoate, preferentially 4-oxo-2-butanol, preferably 4-oxo-4-(C₁-C₅-alkoxy)-2-butanol or 4-oxo-4-(hydroxy-C₃-C₅-alkoxy)-2-butanol, of the general formula (I)
CH₃-CH(OH)-CH₂-C(O)OR¹ (I)
wherein, in the general formula (I), the radical R¹ represents C₁-C₅-alkyl or hydroxy-C₃-C₅-alkyl, especially ethyl, butyl, pentyl, hydroxybutyl or hydroxypentyl, preferably ethyl, hydroxybutyl or hydroxypentyl, more preferably ethyl,
is reacted and/or caused to react with at least one polycarboxylic acid (II), especially with at least one polycarboxylic acid comprising at least two carboxyl groups, especially in an esterification reaction and/or under esterification conditions,
especially so that, as a reaction intermediate (IV') of the method step (a), one or more polycarboxylic acid esters of oxobutanol, especially one or more polycarboxylic acid esters of 3-hydroxybutanoate, preferably one or more polycarboxylic acid esters of 4-oxo-2-butanol, preferentially one or more polycarboxylic acid esters of 4-oxo-4-(C₁-C₅-alkoxy)-2-butanol or of 4-oxo-4-(hydroxy-C₃-C₅-alkoxy)-2-butanol, are obtained; and
(b) subsequently, in a second method step (b), the reaction intermediate product (IV') obtained in method step (a) is reacted and/or caused to react with at least one polyglycerol (III), especially in an esterification reaction and/or under esterification conditions.

3. The method according to any of the preceding claims,
wherein the polycarboxylic acid (II) is selected from the group of succinic acid, tartaric acid, citric acid, malic acid, adipic acid, fumaric acid and maleic acid and their anhydrides as well as combinations or mixtures thereof, especially selected from the group of succinic acid, tartaric acid, citric acid, malic acid, adipic acid and fumaric acid and their anhydrides as well as combinations or mixtures thereof, preferably selected from the group of succinic acid and adipic acid and their anhydrides as well as combinations or mixtures thereof; and/or
wherein the polycarboxylic acid (II) is a naturally occurring carboxylic acid or its anhydride or derivative, especially reaction product, especially a carboxylic acid or its anhydride or derivative, especially reaction product, occurring in human and/or animal metabolism; and/or
wherein the polycarboxylic acid (II) is an ingredient, especially an additive, approved under food law.

4. The method according to any of the preceding claims,
wherein the polyglycerol corresponds to the general formula (Illa)
HO-CH₂-CH(OH)-CH₂-[O-CH₂-CH(OH)-CH₂]ₚ-OH (IIIa)
wherein, in general formula (Ilia), the variable p represents an integer from 1 to 6, especially from 1 to 4, preferentially 1 or 2, more preferably 1; and/or
wherein the polyglycerol (III) is a diglycerol of formula (IIIb)
HO-CH₂-CH(OH)-CH₂-O-CH₂-CH(OH)-CH₂-OH (IIIb);
and/or
wherein the polyglycerol (III) is not propane-1,2,3-triol (glycerol).

5. The method according to any of the preceding claims,
wherein hydroxyl groups and/or carboxyl groups still present in the reaction product after the reaction has been performed are at least partially, preferentially completely, functionalized, especially esterified; and/or
wherein the reaction is followed by a partial, especially complete functionalization, especially esterification, of hydroxyl groups and/or carboxyl groups still present.

6. A reaction intermediate product in the form of a polycarboxylic acid ester of oxobutanol, especially polycarboxylic acid ester of 3-hydroxybutanoate, preferably polycarboxylic acid ester of 4-oxo-2-butanol, preferentially polycarboxylic acid ester of 4-oxo-4-(C₁-C₅-alkoxy)-2-butanol or of 4-oxo-4-(hydroxy-C₃-C₅-alkoxy)-2-butanol, obtainable by a method according to any of the preceding claims, especially obtainable as reaction intermediate product (IV') of the first method step (a) of the method according to any of the preceding claims.

7. A reaction product in the form of a polyglycerol ester of polycarboxylic acids esterified with oxobutanol, especially polyglycerol ester of polycarboxylic acids esterified with 4-oxo-2-butanol, preferentially mono- or polyvalent polyglycerol ester of polycarboxylic acids esterified with 4-oxo-4-(C₁-C₅-alkoxy)-2-butanol or with 4-oxo-4-(hydroxy-C₃-C₅-alkoxy)-2-butanol, obtainable by the method according to any of the preceding claims.

8. A polyglycerol ester of polycarboxylic acids esterified with oxobutanol,
wherein the polyglycerol ester of polycarboxylic acids esterified with oxobutanol, especially polyglycerol ester of polycarboxylic acids esterified with 4-oxo-2-butanol, preferentially mono- or polyvalent polyglycerol ester of polycarboxylic acids esterified with 4-oxo-4-(C₁-C₅-alkoxy)-2-butanol or with 4-oxo-4-(hydroxy-C₃-C₅-alkoxy)-2-butanol, corresponds to the general formula (IVa)
R⁴O-CH₂-CH(OR⁴)-CH₂-[O-CH₂-CH(OR⁴)-CH₂]ₚ-OR⁴ (IVa)
wherein, in the general formula (IVa), the radical R⁴, independently of one another, represents:
• hydrogen;
• a radical R¹ - O - C(O) - CH₂ - CH(CH₃) - O - (O)C - X - C(O) -, wherein the radical R¹ represents C₁-C₅-alkyl or hydroxy-C₃-C₅-alkyl, especially ethyl, butyl, pentyl, hydroxybutyl or hydroxypentyl, preferably ethyl, hydroxybutyl or hydroxypentyl, more preferably ethyl, and wherein X represents a saturated or unsaturated and optionally mono- or polysubstituted, especially substituted with one or more hydroxyl radicals and/or radicals - O - C(O) - CH₂ - CH(OH) - CH₃ and/or carboxyl radicals and/or radicals - C(O) - O - CH(CH₃) - CH₂ - C(O)OR¹ with R¹ as defined hereinabove, organic radical comprising 1 to 10, preferentially comprising 2 to 6 carbon atoms;
• a radical H₃C - CH(OH) - CH₂ - C(O) -;
• a radical HOOC - X - C(O) -, wherein X represents a saturated or unsaturated and optionally mono- or polysubstituted, especially substituted with one or more hydroxyl radicals and/or radicals - O - C(O) - CH₂ - CH(OH) - CH₃ and/or carboxyl radicals and/or radicals - C(O) - O - CH(CH₃) - CH₂ - C(O)OR¹ substituted with R¹ as defined hereinabove, organic radical comprising 1 to 10, preferentially 2 to 6 carbon atoms;
however, with the proviso that at least one radical R⁴, especially at least two radicals R⁴, represents a radical R¹ - O - C(O) - CH₂ - CH(CH₃) - O - (O)C - X - C(O) -, as defined hereinabove; and
wherein, in the general formula (IVa), the variable p represents an integer from 1 to 6, especially from 1 to 4, preferentially 1 or 2, more preferably 1.

9. The polyglycerol ester of polycarboxylic acids esterified with oxobutanol according to claim 7 or 8,
wherein the polyglycerol ester of polycarboxylic acids esterified with oxobutanol, especially polyglycerol ester of polycarboxylic acids esterified with 4-oxo-2-butanol, preferentially mono- or polyvalent polyglycerol ester of polycarboxylic acids esterified with 4-oxo-4-(C₁-C₅-alkoxy)-2-butanol or with 4-oxo-4-(hydroxy-C₃-C₅-alkoxy)-2-butanol, corresponds to the general formula (IVa)
R⁴O-CH₂-CH(OR⁴)-CH₂-[O-CH₂-CH(OR⁴)-CH₂]ₚ-OR⁴ (IVa)
wherein, in the general formula (IVa), the radical R⁴, independently of one another, represents:
• hydrogen;
• a radical R¹ - O - C(O) - CH₂ - CH(CH₃) - O - (O)C - X - C(O) -, wherein the radical R¹ represents C₁-C₅-alkyl or hydroxy-C₃-C₅-alkyl, especially ethyl, butyl, pentyl, hydroxybutyl or hydroxypentyl, preferably ethyl, hydroxybutyl or hydroxypentyl, more preferably ethyl, and wherein X represents a saturated or unsaturated and optionally mono- or polysubstituted, especially substituted with one or more hydroxyl radicals and/or radicals - O - C(O) - CH₂ - CH(OH) - CH₃ and/or carboxyl radicals and/or radicals - C(O) - O - CH(CH₃) - CH₂ - C(O)OR¹ with R¹ as defined hereinabove, organic radical comprising 1 to 10, preferentially 2 to 6 carbon atoms;
however, with the proviso that at least one radical R⁴, especially at least two radicals R⁴, represents a radical R¹ - O - C(O) - CH₂ - CH(CH₃) - O - (O)C - X - C(O) -, as defined hereinabove; and
wherein, in the general formula (IVa), the variable p represents an integer from 1 to 6, especially from 1 to 4, preferentially 1 or 2, more preferably 1.

10. The polyglycerol ester of polycarboxylic acids esterified with oxobutanol according to any of the claims 7 to 9,
wherein the polyglycerol ester of polycarboxylic acids esterified with oxobutanol, especially polyglycerol ester of polycarboxylic acids esterified with 4-oxo-2-butanol, preferentially mono- or polyvalent polyglycerol ester of polycarboxylic acids esterified with 4-oxo-4-(C₁-C₅-alkoxy)-2-butanol or with 4-oxo-4-(hydroxy-C₃-C₅-alkoxy)-2-butanol, corresponds to the general formula (IVa)
R⁴O-CH₂-CH(OR⁴)-CH₂-[O-CH₂-CH(OR⁴)-CH₂]ₚ-OR⁴ (IVa)
wherein, in the general formula (IVa), the radical R⁴, independently of one another, represents:
• hydrogen;
• a radical R¹ - O - C(O) - CH₂ - CH(CH₃) - O - (O)C - X - C(O) -, wherein the radical R¹ represents C₁-C₅-alkyl or hydroxy-C₃-C₅-alkyl, especially ethyl, butyl, pentyl, hydroxybutyl or hydroxypentyl, preferably ethyl, hydroxybutyl or hydroxypentyl, more preferably ethyl, and wherein X represents a saturated or unsaturated and optionally mono- or polysubstituted, especially substituted with one or more hydroxyl radicals and/or carboxyl radicals, organic radical comprising 1 to 10, preferentially 2 to 6 carbon atoms;
however, with the proviso that at least one radical R⁴, especially at least two radicals R⁴, represents a radical R¹ - O - C(O) - CH₂ - CH(CH₃) - O - (O)C - X - C(O) -, as defined hereinabove; and
wherein, in the general formula (IVa), the variable p represents an integer from 1 to 6, especially from 1 to 4, preferentially 1 or 2, more preferably 1.

11. The polyglycerol ester of polycarboxylic acids esterified with oxobutanol according to any of the claims 7 to 10,
wherein the polyglycerol ester of polycarboxylic acids esterified with oxobutanol, especially polyglycerol ester of polycarboxylic acids esterified with 4-oxo-2-butanol, preferentially mono- or polyvalent polyglycerol ester of polycarboxylic acids esterified with 4-oxo-4-(C₁-C₅-alkoxy)-2-butanol or with 4-oxo-4-(hydroxy-C₃-C₅-alkoxy)-2-butanol, corresponds to the general formula (IVb)
R⁵O-CH₂-CH(OR⁵)-CH₂-[O-CH₂-CH(OR⁵)-CH₂]ₚ-OR⁵ (IVb)
wherein, in the general formula (IVb), the radical R⁵, independently of one another, represents:
• hydrogen;
• a radical R¹ - O - C(O) - CH₂ - CH(CH₃) - O - Y -, wherein the radical R¹ represents C₁-C₅-alkyl or hydroxy-C₃-C₅-alkyl, especially ethyl, butyl, pentyl, hydroxybutyl or hydroxypentyl, preferably ethyl, hydroxybutyl or hydroxypentyl, more preferably ethyl, and wherein Y is derived from a polycarboxylic acid selected from the group of succinic acid, tartaric acid, citric acid, malic acid, adipic acid, fumaric acid and maleic acid as well as combinations or mixtures thereof, especially selected from the group of succinic acid, tartaric acid, citric acid, malic acid, adipic acid and fumaric acid as well as combinations or mixtures thereof, preferably selected from the group of succinic acid and adipic acid and anhydrides thereof as well as combinations or mixtures thereof, especially wherein in the case of citric acid the further carboxyl group present is esterified with a radical - CH(CH₃) - CH₂ - C(O)OR¹ with R¹ as defined hereinabove;
• a radical H₃C - CH(OH) - CH₂ - C(O) -;
• a radical - Y - OH with Y as defined hereinabove;
however, with the proviso that at least one radical R⁵, especially at least two radicals R⁵, represents a radical R¹ - O - C(O) - CH₂ - CH(CH₃) - O - Y - with R¹ and Y each as defined hereinabove; and
wherein, in the general formula (IVb), the variable p represents an integer from 1 to 6, especially from 1 to 4, preferentially 1 or 2, more preferably 1.

12. The polyglycerol ester of polycarboxylic acids esterified with oxobutanol according to any of the claims 7 to 11,
wherein the polyglycerol ester of polycarboxylic acids esterified with oxobutanol, especially polyglycerol ester of polycarboxylic acids esterified with 4-oxo-2-butanol, preferentially mono- or polyvalent polyglycerol ester of polycarboxylic acids esterified with 4-oxo-4-(C₁-C₅-alkoxy)-2-butanol or with 4-oxo-4-(hydroxy-C₃-C₅-alkoxy)-2-butanol, corresponds to the general formula (IVb)
R⁵O-CH₂-CH(OR⁵)-CH₂-[O-CH₂-CH(OR⁵)-CH₂]ₚ-OR⁵ (IVb)
wherein, in the general formula (IVb), the radical R⁵, independently of one another, represents:
• hydrogen;
• a radical R¹ - O - C(O) - CH₂ - CH(CH₃) - O - Y -, wherein the radical R¹ represents C₁-C₅-alkyl or hydroxy-C₃-C₅-alkyl, especially ethyl, butyl, pentyl, hydroxybutyl or hydroxypentyl, preferably ethyl, hydroxybutyl or hydroxypentyl, more preferably ethyl, and
wherein Y represents one of the following radicals: 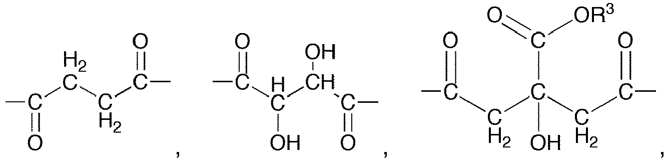 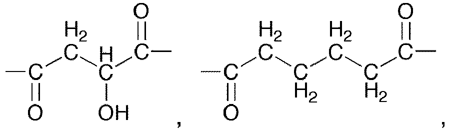 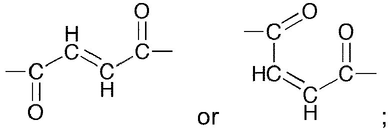 wherein, in the above radicals, the radical R³ represents hydrogen or a radical - CH(CH₃) - CH₂ - C(O)OR¹ with R¹ as defined hereinabove;
• a radical H₃C - CH(OH) - CH₂ - C(O) -;
• a radical - Y - OH with Y as defined hereinabove;
however, with the proviso that at least one radical R⁵, especially at least two radicals R⁵, represents a radical R¹ - O - C(O) - CH₂ - CH(CH₃) - O - Y - with R¹ and Y each as defined hereinabove; and
wherein, in the general formula (IVb), the variable p represents an integer from 1 to 6, especially from 1 to 4, preferentially 1 or 2, more preferably 1.

13. The polyglycerol ester of polycarboxylic acids esterified with oxobutanol according to any of the claims 7 to 12,
wherein the polyglycerol ester of polycarboxylic acids esterified with oxobutanol, especially polyglycerol ester of polycarboxylic acids esterified with 4-oxo-2-butanol, preferentially mono- or polyvalent polyglycerol ester of polycarboxylic acids esterified with 4-oxo-4-(C₁-C₅-alkoxy)-2-butanol or with 4-oxo-4-(hydroxy-C₃-C₅-alkoxy)-2-butanol, corresponds to the general formula (IVc)
R⁴O-CH₂-CH(OR⁴)-CH₂-O-CH₂-CH(OR⁴)-CH₂-OR⁴ (IVc)
wherein, in the general formula (IVc), the radical R⁴, independently of one another, represents
• hydrogen;
• a radical R¹ - O - C(O) - CH₂ - CH(CH₃) - O - (O)C - X - C(O) -, wherein the radical R¹ represents C₁-C₅-alkyl or hydroxy-C₃-C₅-alkyl, especially ethyl, butyl, pentyl, hydroxybutyl or hydroxypentyl, preferably ethyl, hydroxybutyl or hydroxypentyl, more preferably ethyl, and wherein X represents a saturated or unsaturated and optionally mono- or polysubstituted, especially substituted with one or more hydroxyl radicals and/or radicals - O - C(O) - CH₂ - CH(OH) - CH₃ and/or carboxyl radicals and/ or radicals - C(O) - O - CH(CH₃) - CH₂ - C(O)OR¹ with R¹ as defined hereinabove, organic radical comprising 1 to 10, preferentially comprising 2 to 6 carbon atoms;
• a radical H₃C - CH(OH) - CH₂ - C(O) -;
• a radical HOOC - X - C(O) -, wherein X represents a saturated or unsaturated and optionally mono- or polysubstituted, especially substituted with one or more hydroxyl radicals and/or radicals - O - C(O) - CH₂ - CH(OH) - CH₃ and/or carboxyl radicals and/or radicals - C(O) - O - CH(CH₃) - CH₂ - C(O)OR¹ substituted with R¹ as defined hereinabove, organic radical comprising 1 to 10, preferentially 2 to 6 carbon atoms;
however, with the proviso that at least one radical R⁴, especially at least two radicals R⁴, represents a radical R¹ - O - C(O) - CH₂ - CH(CH₃) - O - (O)C - X - C(O) -, as defined hereinabove.

14. The polyglycerol ester of polycarboxylic acids esterified with oxobutanol according to any of the claims 7 to 13,
wherein the polyglycerol ester of polycarboxylic acids esterified with oxobutanol, especially polyglycerol ester of polycarboxylic acids esterified with 4-oxo-2-butanol, preferentially mono- or polyvalent polyglycerol ester of polycarboxylic acids esterified with 4-oxo-4-(C₁-C₅-alkoxy)-2-butanol or with 4-oxo-4-(hydroxy-C₃-C₅-alkoxy)-2-butanol, corresponds to the general formula (IVd)
R⁵O-CH₂-CH(OR⁵)-CH₂-O-CH₂-CH(OR⁵)-CH₂-OR⁵ (IVd)
wherein, in the general formula (IVd), the radical R⁵, independently of one another, represents
• hydrogen;
• a radical R¹ - O - C(O) - CH₂ - CH(CH₃) - O - Y -, wherein the radical R¹ represents C₁-C₅-alkyl or hydroxy-C₃-C₅-alkyl, especially ethyl, butyl, pentyl, hydroxybutyl or hydroxypentyl, preferably ethyl, hydroxybutyl or hydroxypentyl, more preferably ethyl, and wherein Y represents one of the following radicals 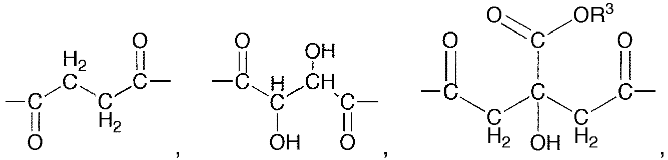 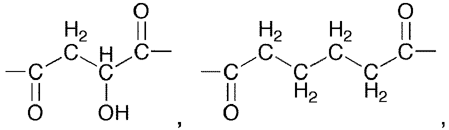 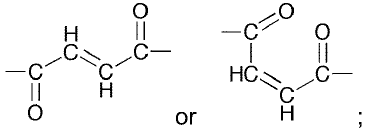 wherein, in the above radicals, the radical R³ represents hydrogen or a radical - CH(CH₃) - CH₂ - C(O)OR¹ with R¹ as defined hereinabove;
• a radical H₃C - CH(OH) - CH₂ - C(O) -;
• a radical - Y - OH with Y as defined hereinabove;
however, with the proviso that at least one radical R⁵, especially at least two radicals R⁵, represents a radical R¹ - O - C(O) - CH₂ - CH(CH₃) - O - Y - with R¹ and Y each as defined hereinabove.

15. A mixture comprising at least two different polyglycerol esters of polycarboxylic acids esterified with oxobutanol, especially polyglycerol esters of polycarboxylic acids esterified with 4-oxo-2-butanol, preferentially mono- or polyvalent polyglycerol esters of polycarboxylic acids esterified with 4-oxo-4-(C₁-C₅-alkoxy)-2-butanol or with 4-oxo-4-(hydroxy-C₃-C₅-alkoxy)-2-butanol, especially as defined hereinabove.

16. A pharmaceutical composition, especially a drug or medicament, comprising a reaction product or a polyglycerol ester of polycarboxylic acids esterified with oxobutanol, especially a polyglycerol ester of polycarboxylic acids esterified with 4-oxo-2-butanol, preferentially a mono- or polyvalent polyglycerol ester of polycarboxylic acids esterified with 4-oxo-4-(C₁-C₅-alkoxy)-2-butanol or with 4-oxo-4-(hydroxy-C₃-C₅-alkoxy)-2-butanol, according to any of the claims 7 to 14 and/or a mixture according to claim 15.

17. The pharmaceutical composition according to claim 16 for use in the prophylactic and/or therapeutic treatment of diseases of the human or animal body, especially diseases associated with a disorder of the energy metabolism, especially keto-body metabolism, such as especially craniocerebral trauma, stroke, hypoxia, cardiovascular diseases such as myocardial infarction, refeeding syndrome, anorexia, epilepsy, neurodegenerative diseases such as dementia, Alzheimer's disease, Parkinson's disease, multiple sclerosis and amyotrophic lateral sclerosis, fat metabolic diseases such as glucose transporter defect (GLUT1 defect), VL-FAOD and mitochondriopathies such as mitochondrial thiolase defect, Huntington's disease, cancers such as T-cell lymphomas, astrocytomas and glioblastomas, HIV, rheumatic diseases such as rheumatoid arthritis and arthritis urica, diseases of the gastrointestinal tract such as chronic inflammatory bowel diseases, especially ulcerative colitis and Crohn's disease, lyosomal storage diseases such as sphingolipidosis, especially Niemann-Pick disease, diabetes mellitus and effects or side-effects of chemotherapy.

18. A reaction product or a polyglycerol esters of polycarboxylic acids esterified with oxobutanol, especially polyglycerol esters of polycarboxylic acids esterified with 4-oxo-2-butanol, preferentially mono- or polyvalent polyglycerol ester of polycarboxylic acids esterified with 4-oxo-4-(C₁-C₅-alkoxy)-2-butanol or with 4-oxo-4-(hydroxy-C₃-C₅-alkoxy)-2-butanol, according to any of the claims 7 to 14 and/or mixture according to claim 15 for use in the prophylactic and/or therapeutic treatment of diseases of the human or animal body, especially diseases associated with a disorder of the energy metabolism, especially keto-body metabolism, such as especially craniocerebral trauma, stroke, hypoxia, cardiovascular diseases such as myocardial infarction, refeeding syndrome, anorexia, epilepsy, neurodegenerative diseases such as dementia, Alzheimer's disease, Parkinson's disease, multiple sclerosis and amyotrophic lateral sclerosis, fat metabolic diseases such as glucose transporter defect (GLUT1 defect), VL-FAOD and mitochondriopathies such as mitochondrial thiolase defect, Huntington's disease, cancers such as T-cell lymphomas, astrocytomas and glioblastomas, HIV, rheumatic diseases such as rheumatoid arthritis and arthritis urica, diseases of the gastrointestinal tract such as chronic inflammatory bowel diseases, especially ulcerative colitis and Crohn's disease, lyosomal storage diseases such as sphingolipidosis, especially Niemann-Pick disease, diabetes mellitus and effects or side-effects of chemotherapy.

19. A food and/or a food product comprising a reaction product or a polyglycerol ester of polycarboxylic acids esterified with oxobutanol, especially polyglycerol ester of polycarboxylic acids esterified with 4-oxo-2-butanol, preferentially mono- or polyvalent polyglycerol ester of polycarboxylic acids esterified with 4-oxo-4-(C₁-C₅-alkoxy)-2-butanol or with 4-oxo-4-(hydroxy-C₃-C₅-alkoxy)-2-butanol, according to any of the claims 7 to 14 and/or a mixture according to claim 15.

## Revendications

1. Procédé de préparation d'esters de polyglycérol d'acides polycarboxyliques estérifiés avec de l'oxobutanol, en particulier d'esters de polyglycérol mono- ou polyvalents d'acides polycarboxyliques estérifiés avec du 4-oxo-4-(C₁-C₅-alcoxy)-2-butanol ou avec du 4-oxo-4-(hydroxy-C₃-C₅ -alcoxy)-2-butanol,
où
(i) au moins un oxobutanol, en particulier un 3-hydroxybutanoate, de préférence un 4-oxo-2-butanol, de préférence un 4-oxo-4-(C₁-C₅-alcoxy)-2-butanol ou un 4-oxo-4-(hydroxy-C₃-C₅-alcoxy)-2-butanol, de formule générale (I)
CH₃-CH(OH)-CH₂-C(O)OR¹ (I)
où, dans la formule générale (I), le radical R¹ représente un C₁-C₅-alkyle ou un hydroxy-C₃-C₅-alkyle, en particulier éthyle, butyle, pentyle, hydroxybutyle ou hydroxypentyle, de préférence éthyle, hydroxybutyle ou hydroxypentyle, de manière particulièrement préférée éthyle,
(ii) au moins un acide polycarboxylique (II), en particulier au moins un acide polycarboxylique contenant au moins deux groupes carboxyle, l'acide polycarboxylique (II) étant utilisé sous forme d'acide polycarboxylique libre, sous forme d'un sel de l'acide polycarboxylique, sous forme d'un ester d'acide polycarboxylique ou sous forme de l'anhydride d'acide polycarboxylique, et
(iii) au moins un polyglycérol (III)
sont amenés à réagir et/ou sont à réagir entre eux,
le procédé étant mis en oeuvre en une seule étape ou en plusieurs étapes, et
le procédé étant mis en oeuvre en l'absence de solvants et/ou sans aucun solvant, et
où, lors de l'utilisation de l'acide polycarboxylique (II) sous forme d'acide libre, de l'eau est formée simultanément lors de la réaction, l'eau étant extraite en continu de la réaction; et/ou
où, lors de l'utilisation de l'acide polycarboxylique (II) sous forme de l'anhydride, l'acide polycarboxylique (II) libre correspondant et de l'eau sont formés, l'acide polycarboxylique (II) libre formé étant soumis à une réaction supplémentaire ou étant éliminé et éventuellement recyclé après que la réaction a eu lieu, et l'eau étant retirée en continu de la réaction; et/ou
où, lors de l'utilisation de l'acide polycarboxylique (II) sous forme d'ester, l'alcool ester correspondant et de l'eau sont formés, l'alcool ester et l'eau formés étant retirés en continu de la réaction,
de manière à obtenir, comme produit de réaction (IV), un ou plusieurs esters de polyglycérol d'acides polycarboxyliques estérifiés par l'oxobutanol, en particulier un ou plusieurs esters de polyglycérol d'acides polycarboxyliques estérifiés par le 4-oxo-2-butanol, de préférence un ou plusieurs esters de polyglycérol monovalents ou polyvalents d'acides polycarboxyliques estérifiés avec le 4-oxo-4-(C₁-C₅-alcoxy)-2-butanol ou avec le 4-oxo-4-(hydroxy-C₃-C₅-alcoxy)-2-butanol.

2. Procédé selon la revendication 1,
le procédé étant réalisé en plusieurs étapes, notamment en deux étapes,
où
(a) dans une première étape de procédé (a), au moins un oxobutanol, en particulier un 3-hydroxybutanoate, de préférence un 4-oxo-2-butanol, de préférence un 4-oxo-4-(C₁-C₅-alcoxy)-2-butanol ou un 4-oxo-4-(hydroxy-C₃-C₅-alcoxy)-2-butanol, de formule générale (I)
CH₃-CH(OH)-CH₂-C(O)OR¹ (I)
où, dans la formule générale (I), le radical R¹ représente un C₁-C₅-alkyle ou un hydroxy-C₃-C₅-alkyle, en particulier éthyle, butyle, pentyle, hydroxybutyle ou hydroxypentyle, de préférence éthyle, hydroxybutyle ou hydroxypentyle, de manière particulièrement préférée éthyle,
est mis en réaction et/ou amené à réagir avec au moins un acide polycarboxylique (II), en particulier avec au moins un acide polycarboxylique contenant au moins deux groupes carboxyle, en particulier est mis en réaction et/ou amené à réagir dans une réaction d'estérification et/ou sous des conditions d'estérification,
de sorte que, en particulier, comme produit intermédiaire de réaction (IV') de l'étape de procédé (a), un ou plusieurs esters d'acide polycarboxylique d'oxobutanol, en particulier un ou plusieurs esters d'acide polycarboxylique de 3-hydroxybutanoate, de préférence un ou plusieurs esters d'acide polycarboxylique de 4-oxo-2-butanol, de préférence un ou plusieurs esters d'acide polycarboxylique de 4-oxo-4-(C₁-C₅-alcoxy)-2-butanol ou de 4-oxo-4-(hydroxy-C₃-C₅-alcoxy)-2-butanol, sont obtenus; et
(b) ensuite, dans une deuxième étape de procédé (b), le produit intermédiaire de réaction (IV') obtenu dans l'étape de procédé (a) est mis en réaction et/ou amené à réagir avec au moins un polyglycérol (III), en particulier est mis en réaction et/ou amené à réagir dans une réaction d'estérification et/ou sous des conditions d'estérification.

3. Procédé selon l'une quelconque des revendications précédentes,
l'acide polycarboxylique (II) étant choisi parmi le groupe de l'acide succinique, de l'acide tartrique, de l'acide citrique, de l'acide malique, de l'acide adipique, de l'acide fumarique et de l'acide maléique et de leurs anhydrides ainsi que de leurs combinaisons ou mélanges, en particulier étant choisi parmi le groupe de l'acide succinique, l'acide tartrique, l'acide citrique, l'acide malique, l'acide adipique et l'acide fumarique et leurs anhydrides ainsi que leurs combinaisons ou mélanges, de préférence est choisi parmi le groupe de l'acide succinique et de l'acide adipique et leurs anhydrides ainsi que leurs combinaisons ou mélanges; et/ou
l'acide polycarboxylique (II) étant un acide carboxylique d'origine naturelle ou son anhydride ou dérivé, en particulier un produit de réaction, en particulier un acide carboxylique présent dans le métabolisme humain et/ou animal ou son anhydride ou dérivé, en particulier un produit de réaction; et/ou
l'acide polycarboxylique (II) étant un ingrédient, en particulier un additif, autorisé par la législation sur les denrées alimentaires.

4. Procédé selon l'une quelconque des revendications précédentes,
où le polyglycérol correspond à la formule générale (IIIa)
HO-CH₂-CH(OH)-CH₂-[O-CH₂-CH(OH)-CH₂]ₚ-OH (IIIa)
où, dans la formule générale (IIIa), la variable p représente un nombre entier de 1 à 6, en particulier de 1 à 4, de préférence 1 ou 2, de manière particulièrement préférée 1; et/ou
où le polyglycérol (III) est un diglycérol de formule (IIIb)
HO-CH₂-CH(OH)-CH₂-O-CH₂-CH(OH)-CH₂-OH (IIIb)
et/ou
où le polyglycérol (III) n'est pas le propane-1,2,3-triol (glycérol).

5. Procédé selon l'une quelconque des revendications précédentes,
où les groupes hydroxyle et/ou les groupes carboxyle encore présents dans le produit de réaction après la réaction sont au moins partiellement, de préférence complètement, fonctionnalisés, en particulier estérifiés; et/ou
où la réaction est suivie d'une fonctionnalisation partielle, en particulier complète, en particulier d'une estérification, de groupes hydroxyle et/ou de groupes carboxyle encore présents.

6. Produit intermédiaire de réaction sous forme d'un ester d'acide polycarboxylique d'oxobutanol, en particulier d'un ester d'acide polycarboxylique de 3-hydroxybutanoate, de préférence d'un ester d'acide polycarboxylique de 4-oxo-2-butanol, de préférence d'un ester d'acide polycarboxylique de 4-oxo-4-(C₁-C₅-alcoxy)-2-butanol ou de 4-oxo-4-(hydroxy-C₃-C₅-alcoxy)-2-butanol, pouvant être obtenu par un procédé selon l'une quelconque des revendications précédentes, en particulier pouvant être obtenu en tant qu'intermédiaire réactionnel (IV') de la première étape (a) du procédé selon l'une quelconque des revendications précédentes.

7. Produit de réaction sous forme d'un ester de polyglycérol d'acides polycarboxyliques estérifiés par l'oxobutanol, en particulier d'un ester de polyglycérol d'acides polycarboxyliques estérifiés par le 4-oxo-2-butanol, de préférence d'un ester de polyglycérol mono- ou polyvalent d'acides polycarboxyliques estérifiés par le 4-oxo-4-(C₁-C₅-alcoxy)-2-butanol ou par le 4-oxo-4-(hydroxy-C₃-C₅-alcoxy)-2-butanol, pouvant être obtenu par un procédé selon l'une des revendications précédentes.

8. Esters de polyglycérol d'acides polycarboxyliques estérifiés par l'oxobutanol,
où l'ester de polyglycérol d'acides polycarboxyliques estérifiés par l'oxobutanol, en particulier l'ester de polyglycérol d'acides polycarboxyliques estérifiés par le 4-oxo-2-butanol, de préférence l'ester de polyglycérol mono- ou polyvalents d'acides polycarboxyliques estérifiés avec le 4-oxo-4-(C₁-C₅-alcoxy)-2-butanol ou avec le 4-oxo-4-(hydroxy-C₃-C₅-alcoxy)-2-butanol, correspond à la formule générale (IVa)
R⁴O-CH₂-CH(OR⁴)-CH₂-[O-CH₂-CH(OR⁴)-CH₂]ₚ-OR⁴ (IVa)
où, dans la formule générale (IVa), le radical R⁴ représente, indépendamment les uns des autres:
• l'hydrogène;
• un radical R¹ - O - C(O) - CH₂ - CH(CH₃) - O - (O)C - X - C(O) -, le radical R¹ représentant un C₁-C₅-alkyle ou un hydroxy-C₃-C₅-alkyle, en particulier éthyle, butyle, pentyle, hydroxybutyle ou hydroxypentyle, de préférence éthyle, hydroxybutyle ou hydroxypentyle, de manière particulièrement préférée éthyle, et X représentant un radical de 1 à 10, de préférence 2 à 6 atomes de carbone, et étant éventuellement substitué une ou plusieurs fois, en particulier par un ou plusieurs radicaux hydroxyle et/ou radicaux - O - C(O) - CH₂ - CH(OH) - CH₃ et/ou radicaux carboxyle et/ou radicaux - C(O) - O - CH(CH₃) - CH₂ - C(O)OR¹ avec R¹ comme défini précédemment, représente un radical organique saturé ou insaturé;
• un radical H₃C - CH(OH) - CH₂ - C(O) -;
• un radical HOOC - X - C(O) -, où X représente un radical contenant 1 à 10, de préférence 2 à 6 atomes de carbone, et étant éventuellement mono- ou polysubstitué, en particulier avec un ou plusieurs radicaux hydroxyle et/ou radicaux - O - C(O) - CH₂ - CH(OH) - CH₃ et/ou radicaux carboxyle et/ou radicaux - C(O) - O - CH(CH₃) - CH₂ - C(O)OR¹ avec R¹ comme défini précédemment, représente un radical organique saturé ou insaturé;
à condition toutefois qu'au moins un radical R⁴, en particulier au moins deux radicaux R⁴, représente un radical R¹ - O - C(O) - CH₂ - CH(CH₃) - O - (O)C - X - C(O) - tel que défini précédemment; et
où, dans la formule générale (IVa), la variable p représente un nombre entier de 1 à 6, en particulier de 1 à 4, de préférence 1 ou 2, de manière particulièrement préférée 1.

9. Esters de polyglycérol d'acides polycarboxyliques estérifiés par l'oxobutanol selon la revendication 7 ou 8,
où l'ester de polyglycérol d'acides polycarboxyliques estérifiés par l'oxobutanol, en particulier l'ester de polyglycérol d'acides polycarboxyliques estérifiés par le 4-oxo-2-butanol, de préférence l'ester de polyglycérol mono- ou polyvalents d'acides polycarboxyliques estérifiés avec le 4-oxo-4-(C₁-C₅-alcoxy)-2-butanol ou avec le 4-oxo-4-(hydroxy-C₃-C₅-alcoxy)-2-butanol, correspond à la formule générale (IVa)
R⁴O-CH₂-CH(OR⁴)-CH₂-[O-CH₂-CH(OR⁴)-CH₂]ₚ-OR⁴ (IVa)
où, dans la formule générale (IVa), le radical R⁴ représente, indépendamment les uns des autres:
• l'hydrogène;
• un radical R¹ - O - C(O) - CH₂ - CH(CH₃) - O - (O)C - X - C(O) -, le radical R¹ représentant un C₁-C₅-alkyle ou un hydroxy-C₃-C₅-alkyle, en particulier éthyle, butyle, pentyle, hydroxybutyle ou hydroxypentyle, de préférence éthyle, hydroxybutyle ou hydroxypentyle, de manière particulièrement préférée éthyle, et X représentant un radical de 1 à 10, de préférence 2 à 6 atomes de carbone, et étant éventuellement substitué une ou plusieurs fois, en particulier par un ou plusieurs radicaux hydroxyle et/ou radicaux - O - C(O) - CH₂ - CH(OH) - CH₃ et/ou radicaux carboxyle et/ou radicaux - C(O) - O - CH(CH₃) - CH₂ - C(O)OR¹ avec R¹ comme défini précédemment, représente un radical organique saturé ou insaturé;
à condition toutefois qu'au moins un radical R⁴, en particulier au moins deux radicaux R⁴, représente un radical R¹ - O - C(O) - CH₂ - CH(CH₃) - O - (O)C - X - C(O) - tel que défini précédemment; et
où, dans la formule générale (IVa), la variable p représente un nombre entier de 1 à 6, en particulier de 1 à 4, de préférence 1 ou 2, de manière particulièrement préférée 1.

10. Esters polyglycériques d'acides polycarboxyliques estérifiés par l'oxobutanol selon l'une quelconque des revendications 7 à 9,
l'ester de polyglycérol d'acides polycarboxyliques estérifiés par l'oxobutanol, en particulier l'ester de polyglycérol d'acides polycarboxyliques estérifiés par le 4-oxo-2-butanol, de préférence l'ester de polyglycérol mono- ou polyvalents d'acides polycarboxyliques estérifiés avec le 4-oxo-4-(C₁-C₅-alcoxy)-2-butanol ou avec le 4-oxo-4-(hydroxy-C₃-C₅-alcoxy)-2-butanol, de formule générale (IVa)
R⁴O-CH₂-CH(OR⁴)-CH₂-[O-CH₂-CH(OR⁴)-CH₂]ₚ--OR⁴ (IVa)
correspond,
où, dans la formule générale (IVa), le radical R⁴ représente, indépendamment les uns des autres:
• l'hydrogène;
• un radical R¹ - O - C(O) - CH₂ - CH(CH₃) - O - (O)C - X - C(O) -, le radical R¹ étant un C₁-C₅-alkyle ou un hydroxy-C₃-C₅-alkyle, en particulier éthyle, butyle, pentyle, hydroxybutyle ou hydroxypentyle, de préférence éthyle, hydroxybutyle ou hydroxypentyle, de manière particulièrement préférée éthyle, et où X représente un radical organique saturé ou insaturé contenant 1 à 10, de préférence 2 à 6 atomes de carbone, et étant éventuellement mono- ou polysubstitué, en particulier substitué par un ou plusieurs radicaux hydroxyle et/ou radicaux carboxyle;
à condition toutefois qu'au moins un radical R⁴, en particulier au moins deux radicaux R⁴, représente un radical R¹ - O - C(O) - CH₂ - CH(CH₃) - O - (O)C - X - C(O) - tel que défini précédemment; et
où, dans la formule générale (IVa), la variable p représente un nombre entier de 1 à 6, en particulier de 1 à 4, de préférence 1 ou 2, de manière particulièrement préférée 1.

11. Esters polyglycériques d'acides polycarboxyliques estérifiés par l'oxobutanol selon l'une quelconque des revendications 7 à 10,
où l'ester de polyglycérol d'acides polycarboxyliques estérifiés par l'oxobutanol, en particulier l'ester de polyglycérol d'acides polycarboxyliques estérifiés par le 4-oxo-2-butanol, de préférence l'ester de polyglycérol mono- ou polyvalents d'acides polycarboxyliques estérifiés avec le 4-oxo-4-(C₁-C₅-alcoxy)-2- butanol ou avec le 4-oxo-4-(hydroxy-C₃-C₅-alcoxy)-2-butanol, correspond à la formule générale (IVb)
R⁵O-CH₂-CH(OR⁵)-CH₂-[O-CH₂-CH(OR⁵)-CH₂]ₚ-OR⁵ (IVb)
où, dans la formule générale (IVb), le radical R⁵ représente, indépendamment les uns des autres:
• l'hydrogène;
• un radical R¹ - O - C(O) - CH₂ - CH(CH₃) - O - Y -, où le radical R¹ est un C₁-C₅-alkyle ou un hydroxy-C₃-C₅-alkyle, en particulier éthyle, butyle, pentyle, hydroxybutyle ou hydroxypentyle, de préférence éthyle, hydroxybutyle ou hydroxypentyle, de manière particulièrement préférée éthyle, et Y est dérivé d'un acide polycarboxylique choisi parmi le groupe constitué par l'acide succinique, l'acide tartrique, l'acide citrique, l'acide malique, l'acide adipique, l'acide fumarique et l'acide maléique, ainsi que leurs combinaisons ou mélanges, en particulier choisi parmi le groupe de l'acide succinique, de l'acide tartrique, de l'acide citrique, de l'acide malique, de l'acide adipique et de l'acide fumarique ainsi que leurs combinaisons ou mélanges, de préférence choisi parmi le groupe de l'acide succinique et de l'acide adipique et de leurs anhydrides ainsi que leurs combinaisons ou mélanges, en particulier où, dans le cas de l'acide citrique, l'autre groupe carboxyle présent est estérifié avec un reste - CH(CH₃) - CH₂ - C(O)OR¹ avec R¹ comme défini précédemment;
• un radical H₃C - CH(OH) - CH₂ - C(O) -;
• un radical - Y - OH avec Y tel que défini précédemment;
à condition toutefois qu'au moins un radical R⁵, en particulier au moins deux radicaux R⁵, représente un radical R¹ - O - C(O) - CH₂ - CH(CH₃) - O - Y - avec R¹ et Y respectivement comme défini précédemment; et
où, dans la formule générale (IVb), la variable p représente un nombre entier de 1 à 6, en particulier de 1 à 4, de préférence 1 ou 2, de manière particulièrement préférée 1.

12. Esters polyglycériques d'acides polycarboxyliques estérifiés par l'oxobutanol selon l'une quelconque des revendications 7 à 11,
où l'ester de polyglycérol d'acides polycarboxyliques estérifiés par l'oxobutanol, en particulier l'ester de polyglycérol d'acides polycarboxyliques estérifiés par le 4-oxo-2-butanol, de préférence l'ester de polyglycérol mono- ou polyvalents d'acides polycarboxyliques estérifiés avec le 4-oxo-4-(C₁-C₅-alcoxy)-2- butanol ou avec le 4-oxo-4-(hydroxy-C₃-C₅-alcoxy)-2-butanol, correspond à la formule générale (IVb)
R⁵O-CH₂-CH(OR⁵)-CH₂-[O-CH₂-CH(OR⁵)-CH₂]ₚ-OR⁵ (IVb)
où, dans la formule générale (IVb), le radical R⁵ représente, indépendamment les uns des autres:
• l'hydrogène;
• un radical R¹ - O - C(O) - CH₂ - CH(CH₃) - O - Y -, le radical R¹ représentant un C₁-C₅-alkyle ou un hydroxy-C₃-C₅-alkyle, en particulier éthyle, butyle, pentyle, hydroxybutyle ou hydroxypentyle, de préférence éthyle, hydroxybutyle ou hydroxypentyle, de manière particulièrement préférée éthyle et
Y représente l'un des radicaux suivants: 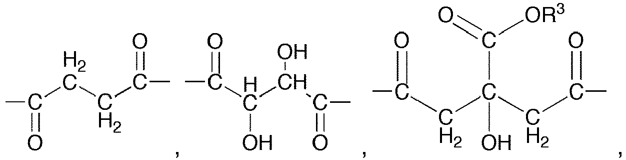 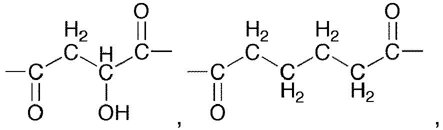 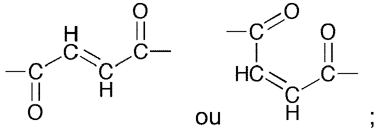 où, dans les radicaux précédents, le radical R³ représente l'hydrogène ou un radical - CH(CH₃) - CH₂ - C(O)OR¹ avec R¹ tel que défini précédemment;
• un radical H₃C - CH(OH) - CH₂ - C(O) -;
• un radical Y - OH avec Y tel que défini précédemment;
à condition toutefois qu'au moins un radical R⁵, en particulier au moins deux radicaux R⁵, représente un radical R¹ - O - C(O) - CH₂ - CH(CH₃) - O - Y - avec R¹ et Y respectivement comme défini précédemment; et
où, dans la formule générale (IVb), la variable p représente un nombre entier de 1 à 6, en particulier de 1 à 4, de préférence 1 ou 2, de manière particulièrement préférée 1.

13. Esters polyglycériques d'acides polycarboxyliques estérifiés par l'oxobutanol selon l'une quelconque des revendications 7 à 12,
où l'ester de polyglycérol d'acides polycarboxyliques estérifiés par l'oxobutanol, en particulier l'ester de polyglycérol d'acides polycarboxyliques estérifiés par le 4-oxo-2-butanol, de préférence l'ester de polyglycérol mono- ou polyvalents d'acides polycarboxyliques estérifiés avec le 4-oxo-4-(C₁-C₅-alcoxy)-2-butanol ou avec le 4-oxo-4-(hydroxy-C₃-C₅-alcoxy)-2-butanol, correspond à la formule générale (IVc)
R⁴O-CH₂-CH(OR⁴)-CH₂-O-CH₂-CH(OR⁴)-CH₂-OR⁴ (IVc)
où, dans la formule générale (IVc), le radical R⁴ représente, indépendamment les uns des autres:
• l'hydrogène;
• un radical R¹ - O - C(O) - CH₂ - CH(CH₃) - O - (O)C - X - C(O) -, le radical R¹ représentant un C₁-C₅-alkyle ou un hydroxy-C₃-C₅-alkyle, en particulier éthyle, butyle, pentyle, hydroxybutyle ou hydroxypentyle, de préférence éthyle, hydroxybutyle ou hydroxypentyle, de manière particulièrement préférée éthyle, et X représentant un radical de 1 à 10, de préférence 2 à 6 atomes de carbone, et étant éventuellement substitué une ou plusieurs fois, en particulier par un ou plusieurs radicaux hydroxyle et/ou radicaux - O - C(O) - CH₂ - CH(OH) - CH₃ et/ou radicaux carboxyle et/ou radicaux - C(O) - O - CH(CH₃) - CH₂ - C(O)OR¹ avec R¹ comme défini précédemment, représente un radical organique saturé ou insaturé;
• un radical H₃C - CH(OH) - CH₂ - C(O) -;
• un radical HOOC - X - C(O) -, où X représente un radical contenant 1 à 10, de préférence 2 à 6 atomes de carbone, et étant éventuellement mono- ou polysubstitué, en particulier avec un ou plusieurs radicaux hydroxyle et/ou radicaux - O - C(O) - CH₂ - CH(OH) - CH₃ et/ou radicaux carboxyle et/ou radicaux - C(O) - O - CH(CH₃) - CH₂ - C(O)OR¹ avec R¹ représente un radical organique saturé ou insaturé et substitué comme défini précédemment;
à condition toutefois qu'au moins un radical R⁴, en particulier au moins deux radicaux R⁴, représente un radical R¹ - O - C(O) - CH₂ - CH(CH₃) - O - (O)C - X - C(O) -, tel que défini précédemment.

14. Esters polyglycériques d'acides polycarboxyliques estérifiés par l'oxobutanol selon l'une quelconque des revendications 7 à 13,
où l'ester de polyglycérol d'acides polycarboxyliques estérifiés par l'oxobutanol, en particulier l'ester de polyglycérol d'acides polycarboxyliques estérifiés par le 4-oxo-2-butanol, de préférence l'ester de polyglycérol mono- ou polyvalents d'acides polycarboxyliques estérifiés avec le 4-oxo-4-(C₁-C₅-alcoxy)-2-butanol ou avec le 4-oxo-4-(hydroxy-C₃-C₅-alcoxy)-2-butanol, correspond à la formule générale (IVd)
R⁵O-CH₂-CH(OR⁵)-CH₂-O-CH₂-CH(OR⁵)-CH₂-OR⁵ (IVd)
où, dans la formule générale (IVd), le radical R⁵ représente, indépendamment les uns des autres:
• l'hydrogène;
• un radical R¹ - O - C(O) - CH₂ - CH(CH₃) - O - Y -, le radical R¹ représentant un C₁-C₅-alkyle ou un hydroxy-C₃-C₅-alkyle, en particulier éthyle, butyle, pentyle, hydroxybutyle ou hydroxypentyle, de préférence éthyle, hydroxybutyle ou hydroxypentyle, de manière particulièrement préférée éthyle, et Y représentant un des radicaux suivants 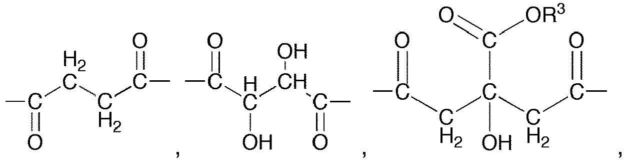 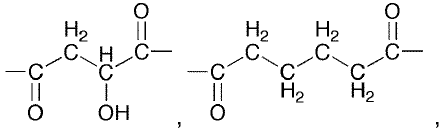 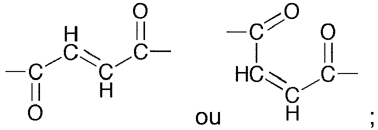 où, dans les radicaux précédents, le radical R³ représente l'hydrogène ou un radical - CH(CH₃) - CH₂ - C(O)OR¹ avec R¹ tel que défini précédemment;
• un radical H₃C - CH(OH) - CH₂ - C(O) -;
• un radical Y - OH avec Y tel que défini précédemment;
à condition toutefois qu'au moins un radical R⁵, en particulier au moins deux radicaux R⁵, représente un radical R¹ - O - C(O) - CH₂ - CH(CH₃) - O - Y - avec R¹ et Y respectivement comme défini précédemment.

15. Mélange comprenant au moins deux esters de polyglycérol différents l'un de l'autre d'acides polycarboxyliques estérifiés avec de l'oxobutanol, en particulier deux esters de polyglycérol d'acides polycarboxyliques estérifiés avec du 4-oxo-2-butanol, de préférence deux esters de polyglycérol -mono- ou polyvalents d'acides polycarboxyliques estérifiés par le 4-oxo-4-(C₁-C₅-alcoxy)-2-butanol ou par le 4-oxo-4-(hydroxy-C₃-C₅-alcoxy)-2-butanol, en particulier tels que définis précédemment.

16. Composition pharmaceutique, en particulier un médicament ou une médication, comprenant un produit de réaction ou un ester de polyglycérol d'acides polycarboxyliques estérifiés par l'oxobutanol, en particulier un ester de polyglycérol d'acides polycarboxyliques estérifiés par le 4-oxo-2-butanol, de préférence un ester de polyglycérol mono- ou polyvalent d'acides polycarboxyliques estérifiés par le 4-oxo-4-(C₁ -C₅-alcoxy)-2-butanol ou par le 4-oxo-4-(hydroxy-C₃-C₅-alcoxy)-2-butanol, selon l'une des revendications 7 à 14 et/ou un mélange selon la revendication 15.

17. Composition pharmaceutique selon la revendication 16 pour son utilisation dans le traitement prophylactique et/ou thérapeutique de maladies du corps humain ou animal, en particulier de maladies liées à un trouble du métabolisme énergétique, en particulier du métabolisme des corps cétoniques, comme notamment les traumatismes crâniens, les accidents vasculaires cérébraux, les hypoxies, les maladies cardiovasculaires comme l'infarctus du myocarde, le syndrome de réalimentation, les anorexies, l'épilepsie, les maladies neurodégénératives comme la démence, la maladie d'Alzheimer, la maladie de Parkinson, la sclérose en plaques et la sclérose latérale amyotrophique, les maladies du métabolisme lipidique telles que le déficit en transporteur de glucose (déficit GLUT1), la VL-FAOD et les mitochondriopathies telles que le déficit en thiolase mitochondriale, la chorée de Huntington, les cancers tels que les lymphomes à cellules T, les astrocytomes et les glioblastomes, le VIH, les maladies rhumatismales telles que la polyarthrite rhumatoïde et l'arthrite urique, les maladies du tractus gastro-intestinal telles que les maladies inflammatoires chroniques de l'intestin, notamment la colite ulcéreuse et la maladie de Crohn, les maladies du stockage lyosomique telles que les sphingolipidoses, notamment la maladie de Niemann-Pick, le diabète sucré et les effets ou effets secondaires des chimiothérapies.

18. Produit de réaction ou ester de polyglycérol d'acides polycarboxyliques estérifiés par l'oxobutanol, en particulier ester de polyglycérol d'acides polycarboxyliques estérifiés par le 4-oxo-2-butanol, de préférence ester de polyglycérol mono- ou polyvalent d'acides polycarboxyliques estérifiés par le 4-oxo-4-(C₁-C₅-alkoxy)-2-butanol ou par le 4-oxo-4-(hydroxy-C₃-C₅-alkoxy)-2-butanol, selon l'une quelconque des revendications 7 à 14 et/ou un mélange selon la revendication 15 pour son utilisation dans le traitement prophylactique et/ou thérapeutique de maladies du corps humain ou animal, notamment de maladies liées à un trouble du métabolisme énergétique, en particulier du métabolisme des corps cétoniques, telles que notamment les traumatismes crâniens, les accidents vasculaires cérébraux, les hypoxies, les maladies cardiovasculaires telles que l'infarctus du myocarde, le syndrome de réalimentation, les anorexies, l'épilepsie, les maladies neurodégénératives telles que la démence, la maladie d'Alzheimer, la maladie de Parkinson, la sclérose en plaques et la sclérose latérale amyotrophique, les maladies du métabolisme lipidique telles que le déficit en transporteur de glucose (déficit GLUT1), la VL-FAOD et les mitochondriopathies telles que le déficit en thiolase mitochondriale, la chorée de Huntington, les cancers tels que les lymphomes à cellules T, les astrocytomes et les glioblastomes, le VIH, les maladies rhumatismales telles que l'arthrite rhumatoïde et l'arthrite urique, les maladies du tractus gastro-intestinal telles que les maladies inflammatoires chroniques de l'intestin, notamment la colite ulcéreuse et la maladie de Crohn, les maladies de stockage lyosomal telles que les sphingolipidoses, notamment la maladie de Niemann-Pick, le diabète sucré et les effets ou effets secondaires des chimiothérapies.

19. Produit alimentaire et/ou nutritionnel comprenant un produit de réaction, respectivement un ester de polyglycérol d'acides polycarboxyliques estérifiés par l'oxobutanol, en particulier un ester de polyglycérol d'acides polycarboxyliques estérifiés par le 4-oxo-2-butanol, de préférence un ester de polyglycérol mono- ou polyvalent d'acides polycarboxyliques estérifiés par le 4-oxo-4-(C₁-C₅-alcoxy)-2-butanol ou par le 4-oxo-4-(hydroxy-C₃-C₅-alcoxy)-2-butanol, selon l'une des revendications 7 à 14 et/ou un mélange selon la revendication 15.
